# EUROPEAN PATENT APPLICATION

(11) **EP 4 703 356 A1**
(43) Date of publication of application: **04.03.2026**
(21) Application number: 24796226.9
(22) Date of filing: 26.04.2024
(51) Int. Cl.: C07D 401/06, C07D 471/00, A61K 31/438, A61P 27/02, A61P 9/10, A61P 13/12

(54) **SALT OF PIPERIDYLINDOLE COMPOUND AND PREPARATION METHOD THEREFOR**

(30) Priority: 27.04.2023 CN 202310477153
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: YANG, Tong, Shanghai 201203 (CN); LIU, Xueliang, Lianyungang, Jiangsu 222047 (CN); HE, Lei, Lianyungang, Jiangsu 222047 (CN)
(74) Representative: EP&C
(86) International application number: PCT/CN2024/090012
(87) International publication number: WO 2024/222852

(57) **Abstract**

The present invention relates to a salt of a piperidylindole compound, and a preparation method therefor and a medical use thereof. The present invention specifically relates to a salt of a compound as shown in general formula (I) and a crystal form, a preparation method, a pharmaceutical composition containing a therapeutically effective amount of the crystal form, and a pharmaceutical use thereof for treating diseases or conditions.

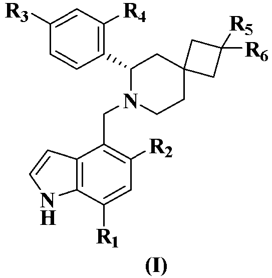

## Description

### Technical Field

The present invention belongs to the field of biomedicine, and specifically relates to a crystal form of a piperidylindole compound, and a preparation method therefor, and the use thereof.

### Background Art

The complement system is part of an innate immune surveillance system and plays a key role in pathogen elimination and tissue homeostasis. The complement cascade can be activated by three different pathways, including the classical pathway (CP), the lectin pathway (LP), and the alternative pathway (AP). CP and LP are initiated on target surfaces through the binding of immune complexes, mannan-binding lectins or ficolins to specific microbial carbohydrate moiety patterns. In contrast, the AP does not require specific initiation. The AP cascade is triggered by the spontaneous hydrolysis (tick-over) of C3 and the subsequent deposition of C3b on activated surfaces. The three complement activation pathways converge on two major events, C3 cleavage and C5 cleavage. The C3 convertase cleaves C3 into C3a and C3b. C3b forms additional AP C3 convertases (amplification) and C5 convertases. The C5 convertase cleaves C5 into C5a and C5b. The resulting C5b initiates the formation of the C5b-9 membrane attack complex (MAC) with C6-C9, which lyses bacteria and cells by inserting into their membranes. The cleavage products C3a and C5a act as anaphylatoxins, promoting proinflammatory responses through leukocyte activation and chemotaxis. C3b also facilitates phagocytosis via opsonization and plays a critical role in the clearance of bacteria and cellular waste products such as immune complexes and apoptotic cells. (Front Immunol. 2015 Jun 2; 6:262. doi: 10.3389/fimmu.2015.00262. eCollection 2015. Complement System Part I - Molecular Mechanisms of Activation and Regulation. Nicolas S Merle, Sarah Elizabeth Church , Veronique Fremeaux-Bacchi, Lubka T Roumenina). AP maintains the basal complement activity by "tick over". Furthermore, even when initiation occurs via other CPs or LPs, the AP contributes more than 80% of the activation of the terminal cleavage pathway (MAC formation) through the amplification loop. (Harboe, M., Garred, P., KarlstroM, E., Lindstad, J.K., Stahl, G.L., and Mollnes, T.E., 2009). The downstream effects of mannan-induced lectin complement pathway activation are quantitatively dependent on amplification by the alternative pathway (Mol. Immunol. 47, 373-380. https://doi.org/10.1016/j.molimm.2009.09.005). Spontaneously activated C3 forms a C3 convertase by binding to factor B (FB). After factor D cleaves FB into Bb, C3b and Bb generate the AP C3 convertase (C3bBb). The newly formed C3bBb cleaves additional C3 to produce more AP C3 convertase, leading to amplification of the complement cascade. Since AP is capable of reaching full complement activity within seconds, it may cause damage to normal tissues if not properly controlled. (J Clin Invest. 2020 May 1;130(5):2152-2163. doi: 10.1172/JCI136094. Complementopathies and precision medicine. Eleni Gavriilaki, Robert A Brodsky). Dysregulated complement activation has been implicated in diseases affecting various organs, including paroxysmal nocturnal haemoglobinuria, age-related macular degeneration, rheumatoid arthritis, haemolytic uremic syndrome, myasthenia gravis, and C3 glomerulonephritis. (J Clin Invest. 2020 May 1; 130(5):2152-2163, doi: 10.1172/JCI136094). Therefore, controlling APs through FB inhibition may represent a potent strategy to limit excessive activation of the complement pathway.

Currently, no small molecules have been approved for modulating the complement pathway. Examples of factor B inhibitors are described in: Advanced Vision Therapies Inc., WO 2008/106644, entitled "TREATMENT OF DISEASES CHARACTERISED BY INFLAMMATION"; Wellstat Immunotherapeutics LLC., WO 2012/151468, entitled "COMPLEMENT FACTOR B ANALOGUES AND THEIR USES"; William Marsh Rice University, WO 2014/035876, entitled "HEAT-INACTIVATED COMPLEMENT FACTOR B COMPOSITIONS AND METHODS"; Muse Research and Development Foundation, PCT/US 1999/023485, entitled "BLOCKING FACTOR B TO TREAT COMPLEMENT-MEDIATED IMMUNE DISEASES"; and Novartis AG., WO 2013/192345 and US 2015/126492, entitled "COMPLEMENT PATHWAY MODULATORS AND USES THEREOF". Other complement factor B inhibitors are described in Novartis patents WO 2015/06241, US 2016/311779, WO 215/009166, US 2016/152605, WO 2014/14638 and US 2016/024079. Another example of a complement factor B inhibitor is WO 2015/038939 by IONIS Pharmaceuticals, entitled "MODULATORS OF COMPLEMENT FACTOR B". Granted patents covering complement factor B inhibitors include US 9452990, US 9676728, US 9682968, and US 9475806.

Given a wide spectrum of diseases caused by overactivation of complement pathways, there is a significant unmet need for patients with complement disorders. The present invention aims to provide compounds that modulate factor B and are used to treat diseases associated with dysregulation of complement pathways.

PCT/CN2022/127975 discloses the structures of a series of piperidylindole compounds. In subsequent research and development, in order to make the product easy to handle, filter and dry, and in order to find a suitable crystal that is easy to store and has long-term stability, the present invention conducts a comprehensive study on the crystal form of the above-described compounds.

### Summary of the Invention

All contents involved in patent application PCT/CN2022/127975 are incorporated herein by reference.

In one aspect, provided is an acid salt of a compound as shown in general formula (I), wherein the compound has a structure as shown below: 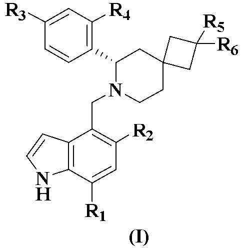 wherein
R₁ is selected from C₁-C₃ alkyl or C₃-C₆ cycloalkyl;
R₂ is selected from C₁-C₃ alkyl, C₁-C₃ alkoxy or C₃-C₆ cycloalkyl;
R₃ is selected from -COOH, -C(O)NHSO₂CH₃ or -S(O)NHCH₃;
R₄ is selected from hydrogen, halogen, C₁-C₃ alkyl or C₁-C₃ haloalkyl;
R₅ and R₆ are each independently selected from halogen;
provided that the compound as shown in general formula (I) is not 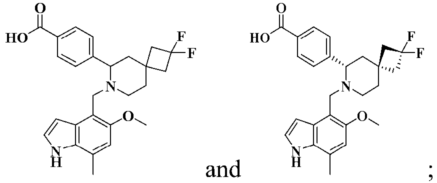
the acid is an inorganic acid or an organic acid; the inorganic acid is selected from hydrochloric acid, sulphuric acid, nitric acid, hydrobromic acid, hydrofluoric acid, hydroiodic acid or phosphoric acid; the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, embonic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, or L-malic acid.

In certain embodiments of the present invention,
R₁ is C₁-C₃ alkyl;
R₂ is selected from C₁-C₃ alkoxy or cyclopropyl;
R₃ is -COOH;
R₄ is selected from hydrogen, C₁-C₃ alkyl or C₁-C₃ haloalkyl;
R₅ and R₆ are each independently selected from hydrogen or halogen.

In certain embodiments of the present invention, the general formula (I) is selected from the following compounds: 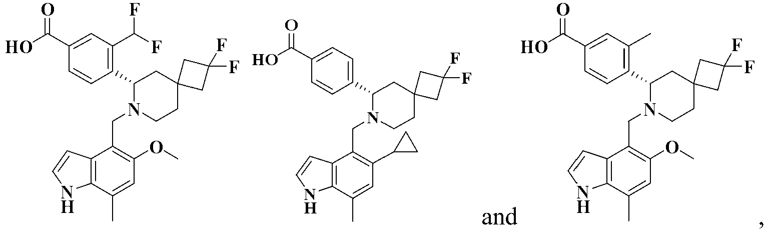 wherein the acid is an inorganic acid or an organic acid; the inorganic acid is selected from hydrochloric acid, sulphuric acid, phosphoric acid or hydrobromic acid, and the organic acid is selected from 1,5-naphthalenedisulphonic acid, methanesulphonic acid, ethanesulphonic acid, p-toluenesulphonic acid, hydroxyethylsulphonic acid, acetic acid, maleic acid, fumaric acid, oxalic acid, malonic acid, tartaric acid, malic acid, adipic acid, hippuric acid, succinic acid or camphoric acid; preferably, the acid is selected from p-toluenesulphonic acid, methanesulphonic acid or hydrochloric acid; further preferably, the acid is hydrochloric acid.

In certain embodiments of the present invention, the compound is (S)-4-(2,2-difluoro-7-((5-methoxy-7-methyl-1H-indol-4-yl)methyl)-7-azaspiro[3.5]nonan-6-yl)-3-methylbenzoic acid, wherein the acid is an inorganic acid or an organic acid; the inorganic acid is selected from hydrochloric acid, sulphuric acid, phosphoric acid or hydrobromic acid, and the organic acid is selected from 1,5-naphthalenedisulphonic acid, methanesulphonic acid, ethanesulphonic acid, p-toluenesulphonic acid, hydroxyethylsulphonic acid, acetic acid, maleic acid, fumaric acid, oxalic acid, malonic acid, tartaric acid, malic acid, adipic acid, hippuric acid, succinic acid or camphoric acid; preferably, the acid is selected from p-toluenesulphonic acid, methanesulphonic acid or hydrochloric acid; further preferably, the acid is hydrochloric acid.

In certain embodiments of the present invention, the number of acid molecules is 0.2-3, preferably 0.2, 0.5, 1, 1.5, 1.2, 2, 2.5 or 3, further preferably 0.5, 1, 2 or 3, and even further preferably 1.

In certain embodiments of the present invention, the acid salt is a hydrate or an anhydrate, preferably an anhydrate; and when the acid salt is a hydrate, the number of water molecules is 0.2 to 3, preferably 0.2, 0.5, 1, 1.5, 2, 2.5 or 3, and more preferably 0.5, 1, 2 or 3.

In a preferred embodiment of the present invention, provided is a compound, i.e., (S)-4-(2,2-difluoro-7-((5-methoxy-7-methyl-1H-indol-4-yl)methyl)-7-azaspiro[3.5]nonan-6-yl)-3-methylbenzoic acid (compound 3), or a stereoisomer and salt thereof, wherein the compound has a structure as shown below:

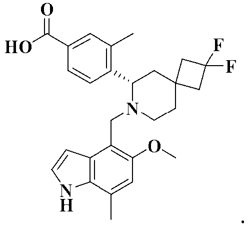

In certain embodiments of the present invention, the acid salt of (S)-4-(2,2-difluoro-7-((5-methoxy-7-methyl-1H-indol-4-yl)methyl)-7-azaspiro[3.5]nonan-6-yl)-3-methylbenzoic acid is in a crystal form.

In a preferred embodiment of the present invention, the crystal form of the salt of (S)-4-(2,2-difluoro-7-((5-methoxy-7-methyl-1H-indol-4-yl)methyl)-7-azaspiro[3.5]nonan-6-yl)-3-methylbenzoic acid is selected from crystal form A of a hydrobromide, crystal forms A to I of a hydrochloride, crystal form A of a methanesulfonate, crystal form A of a hippurate, crystal form A of a malonate, crystal form A of an oxalate, crystal form A of an adipate, crystal form A of a succinate, crystal form A of a phosphate, or crystal form A of a fumarate.

In a further preferred embodiment of the present invention, the crystal form A of the hydrobromide has an X-ray powder diffraction pattern comprising a diffraction peak at one or more of 2θ (±0.2°) of 8.8, 17.5, 15.3, 22.8, 26.4, 22.0, 25.4, 31.0 and 35.5, preferably comprising a characteristic peak at any 2, 4, 6 and 8 of the 2θ.

In a further preferred embodiment of the present invention, the crystal form A of the hydrobromide has an X-ray powder diffraction pattern comprising a characteristic peak at one or more of 2θ (±0.2°) of 8.8, 15.3, 17.5 and 22.8, preferably 2-4 of the 2θ, more preferably 3-4 of the 2θ, and most preferably 4 of the 2θ; preferably, further comprising a characteristic peak at one or more of 2θ (±0.2°) of 22.0 and 26.4; further preferably, further comprising a characteristic peak at one or more of 2θ (±0.2°) of 25.4 and 31.0; more preferably, further comprising a characteristic peak at 2θ (±0.2°) of 35.5.

For example, the characteristic peaks are at 2θ (±0.2°) of
8.8, 17.5, 15.3 and 22.8;
8.8, 17.5, 15.3, 22.8, 26.4, 22.0 and 25.4;
8.8, 17.5, 15.3, 22.8, 26.4, 22.0, 25.4, 31.0 and 35.5.

By using Cu-Kα radiation, the X-ray characteristic diffraction peaks represented by 2θ angles and interplanar spacing d values are shown in Table 1.

**Table 1**

| No. | XRPD data of form A of hydrobromide | | | | | |
|---|---|---|---|---|---|---|
| | 2θ (±0.2°) | d value | Peak height | Proportion (I%) | Area | Proportion (I%) |
| 1 | 4.373 | 20.1875 | 203 | 2.6 | 380 | 1 |
| 2 | 8.75 | 10.0971 | 7937 | 100 | 36966 | 100 |
| 3 | 9.797 | 9.0207 | 588 | 7.4 | 3174 | 8.6 |
| 4 | 10.164 | 8.696 | 172 | 2.2 | 770 | 2.1 |
| 5 | 11.265 | 7.8483 | 143 | 1.8 | 724 | 2 |
| 6 | 12.239 | 7.2259 | 63 | 0.8 | 452 | 1.2 |
| 7 | 13.145 | 6.7295 | 248 | 3.1 | 1358 | 3.7 |
| 8 | 14.048 | 6.299 | 318 | 4 | 1802 | 4.9 |
| 9 | 15.291 | 5.7897 | 1582 | 19.9 | 8496 | 23 |
| 10 | 15.617 | 5.6694 | 66 | 0.8 | 479 | 1.3 |
| 11 | 17.546 | 5.0504 | 3040 | 38.3 | 17067 | 46.2 |
| 12 | 18.917 | 4.6873 | 162 | 2 | 1215 | 3.3 |
| 13 | 20.1 | 4.4139 | 96 | 1.2 | 786 | 2.1 |
| 14 | 20.292 | 4.3727 | 51 | 0.6 | 652 | 1.8 |
| 15 | 20.728 | 4.2817 | 321 | 4 | 1737 | 4.7 |
| 16 | 21.316 | 4.1649 | 150 | 1.9 | 792 | 2.1 |
| 17 | 21.985 | 4.0396 | 3313 | 41.7 | 18276 | 49.4 |
| 18 | 22.649 | 3.9226 | 160 | 2 | 1836 | 5 |
| 19 | 22.835 | 3.8912 | 1186 | 14.9 | 8584 | 23.2 |
| 20 | 24.54 | 3.6245 | 211 | 2.7 | 1455 | 3.9 |
| 21 | 25.208 | 3.53 | 403 | 5.1 | 3764 | 10.2 |
| 22 | 25.365 | 3.5084 | 712 | 9 | 6957 | 18.8 |
| 23 | 26.446 | 3.3674 | 1055 | 13.3 | 6221 | 16.8 |
| 24 | 26.932 | 3.3079 | 169 | 2.1 | 1193 | 3.2 |
| 25 | 27.155 | 3.2811 | 51 | 0.6 | 392 | 1.1 |
| 26 | 27.785 | 3.2081 | 46 | 0.6 | 215 | 0.6 |
| 27 | 29.562 | 3.0192 | 68 | 0.9 | 381 | 1 |
| 28 | 29.824 | 2.9933 | 63 | 0.8 | 569 | 1.5 |
| 29 | 30.951 | 2.8868 | 827 | 10.4 | 5789 | 15.7 |
| 30 | 31.92 | 2.8014 | 83 | 1 | 1114 | 3 |
| 31 | 32.213 | 2.7765 | 66 | 0.8 | 504 | 1.4 |
| 32 | 33.879 | 2.6437 | 139 | 1.8 | 1062 | 2.9 |
| 33 | 35.509 | 2.526 | 1094 | 13.8 | 8189 | 22.2 |
| 34 | 38.286 | 2.3489 | 56 | 0.7 | 468 | 1.3 |
| 35 | 39.276 | 2.292 | 70 | 0.9 | 496 | 1.3 |

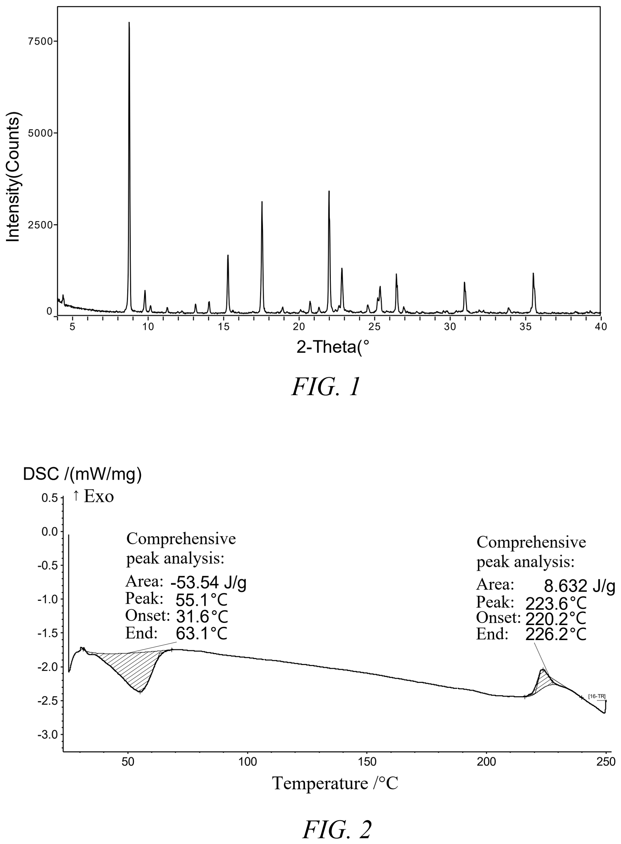

The crystal form A of the hydrobromide of compound 3 of the present invention has an X-ray powder diffraction pattern substantially as shown in FIG. 1, and a DSC pattern substantially as shown in FIG. 2.

In a further preferred embodiment of the present invention, the crystal form A of the hydrochloride has an X-ray powder diffraction pattern comprising a diffraction peak at one or more of 2θ (±0.2°) of 9.2, 15.7, 11.4, 16.8, 20.0, 13.7, 19.6, 20.3 and 21.7, preferably comprising a characteristic peak at any 2, 4, 6 and 8 of the 2θ;

In a further preferred embodiment of the present invention, the crystal form A of the hydrochloride has an X-ray powder diffraction pattern comprising a characteristic peak at one or more of 2θ (±0.2°) of 9.2, 11.4, 15.7 and 20.0, preferably 2-4 of the 2θ, more preferably 3-4 of the 2θ, and most preferably 4 of the 2θ; preferably, further comprising a characteristic peak at one or more of 2θ (±0.2°) of 13.7 and 16.8; further preferably, further comprising a characteristic peak at one or more of 2θ (±0.2°) of 19.6 and 20.3; more preferably, further comprising a characteristic peak at 2θ (±0.2°) of 16.5 and 21.7.

For example, the characteristic peaks are at 2θ (±0.2°) of
15.7 and 20.0;
9.2, 15.7 and 20.0;
9.2, 15.7, 11.4 and 16.8;
9.2, 15.7, 11.4 and 20.0;
9.2, 15.7, 16.8 and 20.0;
9.2, 15.7, 11.4, 16.8, 20.0 and 19.6;
9.2, 15.7, 11.4, 16.8, 19.6 and 13.7;
9.2, 21.7, 11.4, 16.8, 20.0 and 13.7;
9.2, 15.7, 11.4, 21.7, 20.0 and 13.7;
19.6, 15.7, 16.5, 16.8, 21.7 and 13.7;
9.2, 15.7, 16.5, 16.8, 19.6 and 13.7;
9.2, 15.7, 11.4, 16.8, 20.0, 13.7, 16.5 and 20.3;
9.2, 15.7, 11.4, 16.8, 21.7, 13.7, 19.6 and 20.3;
9.2, 15.7, 11.4, 16.8, 20.0, 16.5, 19.6 and 20.3;
21.7, 15.7, 11.4, 16.5, 20.0, 13.7, 19.6 and 20.3;
9.2, 21.7, 11.4, 16.8, 20.0, 13.7, 19.6 and 16.5;
9.2, 15.7, 21.7, 16.8, 20.0, 13.7, 19.6 and 20.3;
9.2, 15.7, 11.4, 16.8, 20.0, 13.7, 19.6, 20.3 and 21.7;

By using Cu-Kα radiation, the X-ray characteristic diffraction peaks represented by 2θ angles and interplanar spacing d values are shown in Table 2.

**Table 2**

| No. | XRPD data of form A of hydrochloride | | | | | |
|---|---|---|---|---|---|---|
| | 2θ (±0.2°) | d value | Peak height | Proportion (I%) | Area | Proportion (I%) |
| 1 | 8.463 | 10.4388 | 166 | 9.5 | 1060 | 4.7 |
| 2 | 9.15 | 9.6571 | 1034 | 59 | 9001 | 40.1 |
| 3 | 9.981 | 8.8546 | 411 | 23.4 | 4850 | 21.6 |
| 4 | 10.286 | 8.5925 | 86 | 4.9 | 1196 | 5.3 |
| 5 | 11.404 | 7.7532 | 921 | 52.5 | 8259 | 36.8 |
| 6 | 12.108 | 7.3036 | 133 | 7.6 | 1109 | 4.9 |
| 7 | 13.69 | 6.4631 | 1100 | 62.7 | 9272 | 41.3 |
| 8 | 14.339 | 6.1719 | 330 | 18.8 | 3576 | 15.9 |
| 9 | 14.543 | 6.0857 | 98 | 5.6 | 1301 | 5.8 |
| 10 | 15.696 | 5.6411 | 1728 | 98.6 | 13671 | 60.9 |
| 11 | 16.124 | 5.4924 | 164 | 9.4 | 1910 | 8.5 |
| 12 | 16.47 | 5.3779 | 499 | 28.5 | 5365 | 23.9 |
| 13 | 16.814 | 5.2686 | 610 | 34.8 | 6052 | 27 |
| 14 | 17.538 | 5.0527 | 102 | 5.8 | 606 | 2.7 |
| 15 | 18.192 | 4.8723 | 377 | 21.5 | 4736 | 21.1 |
| 16 | 18.618 | 4.762 | 281 | 16 | 2982 | 13.3 |
| 17 | 19.568 | 4.5328 | 901 | 51.4 | 8940 | 39.8 |
| 18 | 20.016 | 4.4324 | 1753 | 100 | 22444 | 100 |
| 19 | 20.321 | 4.3666 | 794 | 45.3 | 13178 | 58.7 |
| 20 | 21.192 | 4.1889 | 475 | 27.1 | 6749 | 30.1 |
| 21 | 21.436 | 4.1418 | 352 | 20.1 | 7012 | 31.2 |
| 22 | 21.739 | 4.0847 | 1000 | 57 | 13282 | 59.2 |
| 23 | 22.534 | 3.9425 | 83 | 4.7 | 796 | 3.5 |
| 24 | 22.933 | 3.8748 | 204 | 11.6 | 1891 | 8.4 |
| 25 | 24.173 | 3.6787 | 210 | 12 | 2615 | 11.7 |
| 26 | 25.512 | 3.4886 | 239 | 13.6 | 2658 | 11.8 |
| 27 | 26.037 | 3.4194 | 272 | 15.5 | 4887 | 21.8 |
| 28 | 26.721 | 3.3334 | 265 | 15.1 | 2578 | 11.5 |
| 29 | 27.357 | 3.2574 | 94 | 5.4 | 2873 | 12.8 |
| 30 | 27.641 | 3.2245 | 280 | 16 | 5012 | 22.3 |
| 31 | 28.038 | 3.1798 | 175 | 10 | 1599 | 7.1 |
| 32 | 28.648 | 3.1134 | 91 | 5.2 | 1775 | 7.9 |
| 33 | 28.854 | 3.0917 | 97 | 5.5 | 1784 | 7.9 |
| 34 | 29.889 | 2.9869 | 174 | 9.9 | 3986 | 17.8 |
| 35 | 30.316 | 2.9459 | 243 | 13.9 | 4833 | 21.5 |
| 36 | 31.471 | 2.8403 | 260 | 14.8 | 3469 | 15.5 |
| 37 | 31.977 | 2.7965 | 342 | 19.5 | 4923 | 21.9 |
| 38 | 32.507 | 2.7521 | 70 | 4 | 1162 | 5.2 |
| 39 | 37.614 | 2.3894 | 96 | 5.5 | 1761 | 7.8 |

The crystal form A of the hydrochloride of compound 3 of the present invention has an X-ray powder diffraction pattern substantially as shown in FIG. 3, a DSC pattern substantially as shown in FIG. 4, and a TGA pattern substantially as shown in FIG. 5.

In certain embodiments of the present invention, the crystal form B of the hydrochloride has an X-ray powder diffraction pattern comprising a diffraction peak at one or more of 2θ (±0.2°) of 7.2, 15.9, 7.8, 14.0, 20.0, 10.5, 17.7 and 21.5, preferably comprising a characteristic peak at any 2, 4, 6 and 8 of the 2θ;

In certain embodiments of the present invention, the crystal form B of the hydrochloride has an X-ray powder diffraction pattern comprising a characteristic peak at one or more of 2θ (±0.2°) of 7.2, 7.8, 14.0 and 15.9, preferably 2-4 of the 2θ, more preferably 3-4 of the 2θ, and most preferably 4 of the 2θ; preferably, further comprising a characteristic peak at one or more of 2θ (±0.2°) of 10.5 and 20.0; further preferably, further comprising a characteristic peak at one or more of 2θ (±0.2°) of 17.7 and 21.5.

For example, the characteristic peaks are at 2θ (±0.2°) of
7.2, 15.9, 7.8 and 14.0;
7.2, 15.9, 7.8, 14.0, 20.0 and 10.5;
7.2, 15.9, 7.8, 14.0, 20.0, 10.5, 17.7 and 21.5.

By using Cu-Kα radiation, the X-ray characteristic diffraction peaks represented by 2θ angles and interplanar spacing d values are shown in Table 3.

**Table 3**

| No. | XRPD data of form B of hydrochloride | | | | | |
|---|---|---|---|---|---|---|
| | 2θ (±0.2°) | d value | Peak height | Proportion (I%) | Area | Proportion (I%) |
| 1 | 7.18 | 12.3018 | 345 | 85.4 | 3428 | 87.6 |
| 2 | 7.831 | 11.2801 | 156 | 38.6 | 1547 | 39.5 |
| 3 | 10.526 | 8.3974 | 113 | 28 | 968 | 24.7 |
| 4 | 10.776 | 8.203 | 70 | 17.3 | 1678 | 42.9 |
| 5 | 10.959 | 8.0665 | 102 | 25.2 | 910 | 23.2 |
| 6 | 12.6 | 7.0193 | 102 | 25.2 | 1204 | 30.8 |
| 7 | 13.954 | 6.3413 | 329 | 81.4 | 2727 | 69.7 |
| 8 | 14.91 | 5.9367 | 90 | 22.3 | 979 | 25 |
| 9 | 15.945 | 5.5536 | 404 | 100 | 2712 | 69.3 |
| 10 | 17.721 | 5.0007 | 247 | 61.1 | 3915 | 100 |
| 11 | 18.715 | 4.7374 | 63 | 15.6 | 495 | 12.6 |
| 12 | 19.166 | 4.627 | 103 | 25.5 | 801 | 20.5 |
| 13 | 19.957 | 4.4452 | 194 | 48 | 3042 | 77.7 |
| 14 | 20.257 | 4.3801 | 58 | 14.4 | 987 | 25.2 |
| 15 | 21.492 | 4.1311 | 156 | 38.6 | 1486 | 38 |
| 16 | 22.018 | 4.0336 | 51 | 12.6 | 163 | 4.2 |
| 17 | 22.302 | 3.9829 | 67 | 16.6 | 523 | 13.4 |
| 18 | 22.712 | 3.912 | 133 | 32.9 | 1819 | 46.5 |
| 19 | 24.01 | 3.7033 | 54 | 13.4 | 305 | 7.8 |
| 20 | 24.315 | 3.6575 | 74 | 18.3 | 1749 | 44.7 |
| 21 | 25.591 | 3.478 | 130 | 32.2 | 1909 | 48.8 |
| 22 | 26.561 | 3.3531 | 70 | 17.3 | 716 | 18.3 |

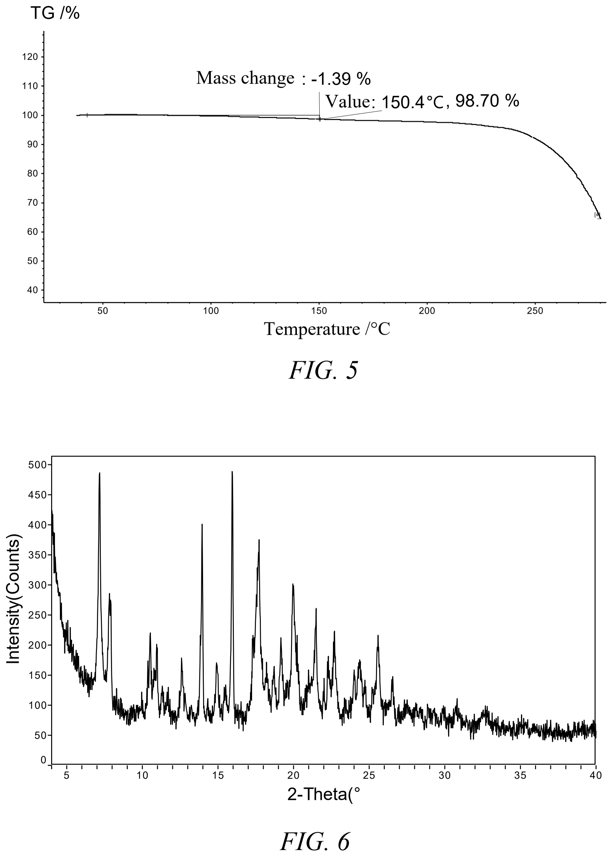

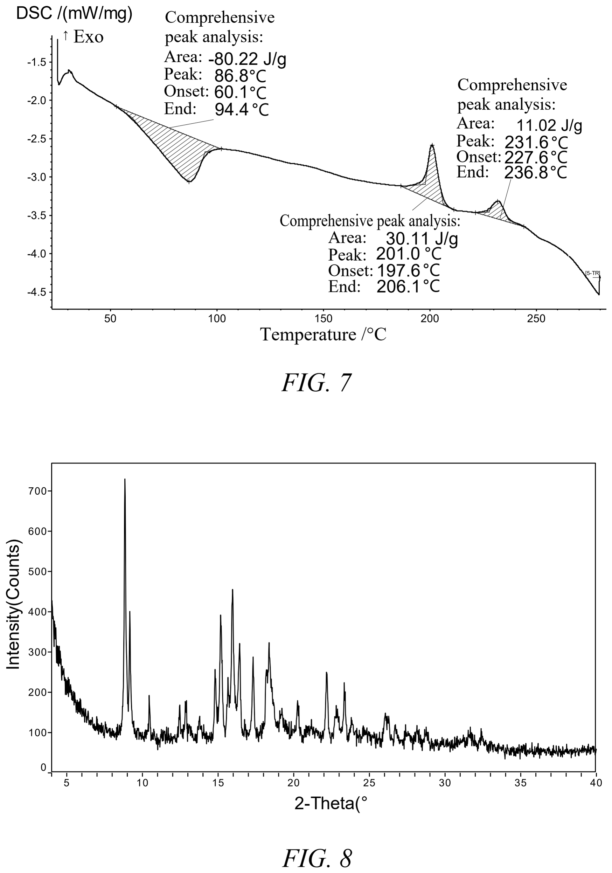

The crystal form B of the hydrochloride of compound 3 of the present invention has an X-ray powder diffraction pattern substantially as shown in FIG. 6, and a DSC pattern substantially as shown in FIG. 7.

In certain embodiments of the present invention, the crystal form C of the hydrochloride has an X-ray powder diffraction pattern comprising a diffraction peak at one or more of 2θ (±0.2°) of 9.2, 16.0, 14.8, 15.2, 8.8 and 18.4, preferably comprising a characteristic peak at any 2, 4 and 6 of the 2θ;

In certain embodiments of the present invention, the crystal form C of the hydrochloride has an X-ray powder diffraction pattern comprising a characteristic peak at one or more of 2θ (±0.2°) of 9.2, 14.8, 15.2 and 16.0, preferably 2-4 of the 20, more preferably 3-4 of the 20, and most preferably 4 of the 2θ; preferably, further comprising a characteristic peak at one or more of 2θ (±0.2°) of 8.8 and 18.4.

For example, the characteristic peaks are at 2θ (±0.2°) of
9.2, 16.0, 14.8 and 15.2;
9.2, 16.0, 14.8, 15.2, 8.8 and 18.4.

By using Cu-Kα radiation, the X-ray characteristic diffraction peaks represented by 2θ angles and interplanar spacing d values are shown in Table 4.

**Table 4**

| No. | XRPD data of form C of hydrochloride | | | | | |
|---|---|---|---|---|---|---|
| | 2θ (±0.2°) | d value | Peak height | Proportion (I%) | Area | Proportion (I%) |
| 1 | 8.847 | 9.9875 | 620 | 100 | 4684 | 100 |
| 2 | 9.17 | 9.6364 | 291 | 46.9 | 1998 | 42.7 |
| 3 | 10.456 | 8.4537 | 108 | 17.4 | 739 | 15.8 |
| 4 | 12.47 | 7.0922 | 75 | 12.1 | 450 | 9.6 |
| 5 | 12.847 | 6.8853 | 73 | 11.8 | 350 | 7.5 |
| 6 | 13.769 | 6.426 | 49 | 7.9 | 479 | 10.2 |
| 7 | 14.807 | 5.9778 | 137 | 22.1 | 686 | 14.6 |
| 8 | 15.174 | 5.8342 | 296 | 47.7 | 3154 | 67.3 |
| 9 | 15.658 | 5.6546 | 120 | 19.4 | 1016 | 21.7 |
| 10 | 15.96 | 5.5484 | 316 | 51 | 3540 | 75.6 |
| 11 | 16.428 | 5.3916 | 178 | 28.7 | 962 | 20.5 |
| 12 | 17.32 | 5.1158 | 197 | 31.8 | 1498 | 32 |
| 13 | 18.375 | 4.8244 | 216 | 34.8 | 3898 | 83.2 |
| 14 | 19.168 | 4.6265 | 32 | 5.2 | 441 | 9.4 |
| 15 | 20.258 | 4.38 | 91 | 14.7 | 963 | 20.6 |
| 16 | 22.169 | 4.0066 | 166 | 26.8 | 1480 | 31.6 |
| 17 | 22.814 | 3.8948 | 74 | 11.9 | 836 | 17.8 |
| 18 | 23.344 | 3.8075 | 129 | 20.8 | 841 | 18 |
| 19 | 23.81 | 3.7339 | 48 | 7.7 | 401 | 8.6 |
| 20 | 26.056 | 3.417 | 73 | 11.8 | 1269 | 27.1 |
| 21 | 26.721 | 3.3334 | 45 | 7.3 | 409 | 8.7 |

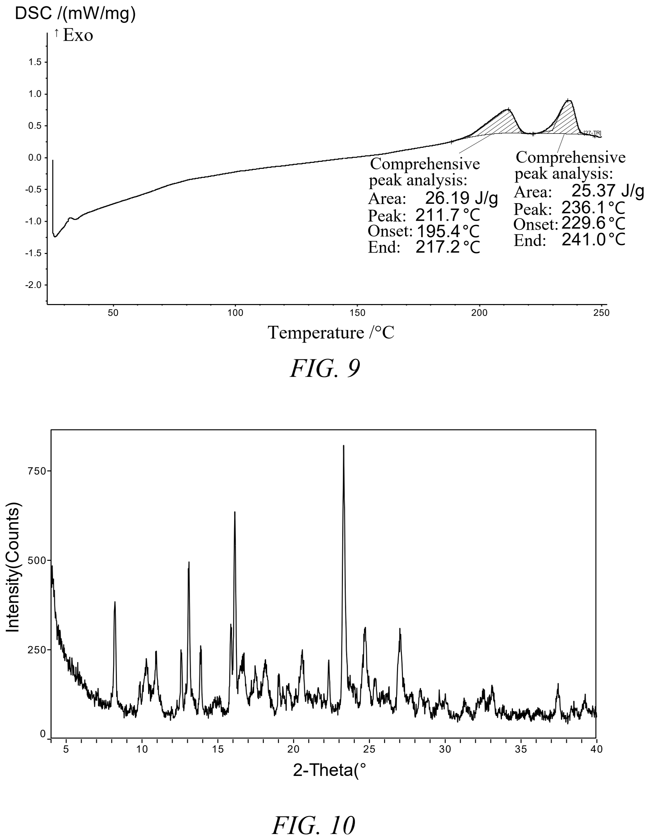

The crystal form C of the hydrochloride of compound 3 of the present invention has an X-ray powder diffraction pattern substantially as shown in FIG. 8, and a DSC pattern substantially as shown in FIG. 9.

In certain embodiments of the present invention, the crystal form D of the hydrochloride has an X-ray powder diffraction pattern comprising a diffraction peak at one or more of 2θ (±0.2°) of 23.3, 16.1, 13.1, 8.2, 15.9, 24.7 and 27.1, preferably comprising a characteristic peak at any 2, 4, 6 and 8 of the 2θ;

In certain embodiments of the present invention, the crystal form D of the hydrochloride has an X-ray powder diffraction pattern comprising a characteristic peak at one or more of 2θ (±0.2°) of 8.2, 13.1, 23.3 and 16.1, preferably 2-4 of the 20, more preferably 3-4 of the 20, and most preferably 4 of the 2θ; preferably, further comprising a characteristic peak at one or more of 2θ (±0.2°) of 15.9 and 24.7; further preferably, further comprising a characteristic peak at 2θ (±0.2°) of 27.1.

For example, the characteristic peaks are at 2θ (±0.2°) of
23.3, 16.1, 13.1 and 8.2;
23.3, 16.1, 13.1, 8.2, 15.9 and 24.7;
23.3, 16.1, 13.1, 8.2, 15.9, 24.7 and 27.1;

By using Cu-Kα radiation, the X-ray characteristic diffraction peaks represented by 2θ angles and interplanar spacing d values are shown in Table 5.

**Table 5**

| No. | XRPD data of form D of hydrochloride | | | | | |
|---|---|---|---|---|---|---|
| | 2θ (±0.2°) | d value | Peak height | Proportion (I%) | Area | Proportion (I%) |
| 1 | 8.216 | 10.7523 | 299 | 42.4 | 2790 | 42.6 |
| 2 | 9.87 | 8.9541 | 64 | 9.1 | 367 | 5.6 |
| 3 | 10.287 | 8.5917 | 108 | 15.3 | 1333 | 20.4 |
| 4 | 10.95 | 8.0736 | 145 | 20.5 | 1606 | 24.5 |
| 5 | 12.582 | 7.0293 | 172 | 24.4 | 1313 | 20.1 |
| 6 | 13.1 | 6.7529 | 417 | 59.1 | 4011 | 61.3 |
| 7 | 13.874 | 6.3775 | 189 | 26.8 | 1464 | 22.4 |
| 8 | 15.862 | 5.5825 | 209 | 29.6 | 1063 | 16.2 |
| 9 | 16.124 | 5.4924 | 511 | 72.4 | 4094 | 62.5 |
| 10 | 16.67 | 5.3136 | 107 | 15.2 | 1497 | 22.9 |
| 11 | 17.482 | 5.0688 | 78 | 11 | 925 | 14.1 |
| 12 | 18.149 | 4.8839 | 118 | 16.7 | 2030 | 31 |
| 13 | 19.024 | 4.6613 | 98 | 13.9 | 933 | 14.3 |
| 14 | 19.29 | 4.5976 | 50 | 7.1 | 392 | 6 |
| 15 | 19.671 | 4.5093 | 54 | 7.6 | 544 | 8.3 |
| 16 | 20.582 | 4.3118 | 140 | 19.8 | 1802 | 27.5 |
| 17 | 22.325 | 3.9789 | 141 | 20 | 1163 | 17.8 |
| 18 | 23.301 | 3.8144 | 706 | 100 | 6547 | 100 |
| 19 | 24.738 | 3.596 | 215 | 30.5 | 3543 | 54.1 |
| 20 | 25.369 | 3.5079 | 68 | 9.6 | 698 | 10.7 |
| 21 | 27.068 | 3.2915 | 204 | 28.9 | 3478 | 53.1 |
| 22 | 28.365 | 3.1439 | 64 | 9.1 | 787 | 12 |
| 23 | 29.61 | 3.0144 | 55 | 7.8 | 423 | 6.5 |
| 24 | 30.009 | 2.9752 | 54 | 7.6 | 797 | 12.2 |
| 25 | 31.247 | 2.8601 | 53 | 7.5 | 750 | 11.5 |
| 26 | 32.505 | 2.7523 | 51 | 7.2 | 638 | 9.7 |
| 27 | 33.076 | 2.706 | 72 | 10.2 | 869 | 13.3 |
| 28 | 37.448 | 2.3995 | 95 | 13.5 | 1120 | 17.1 |
| 29 | 39.232 | 2.2945 | 58 | 8.2 | 647 | 9.9 |

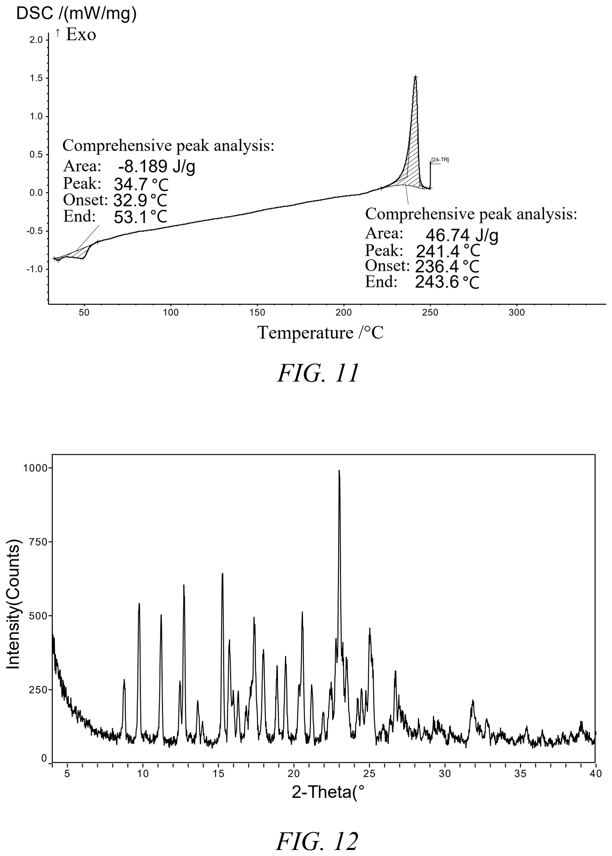

The crystal form D of the hydrochloride of compound 3 of the present invention has an X-ray powder diffraction pattern substantially as shown in FIG. 10, and a DSC pattern substantially as shown in FIG. 11.

In certain embodiments of the present invention, the crystal form E of the hydrochloride has an X-ray powder diffraction pattern comprising a diffraction peak at one or more of 2θ (±0.2°) of 23.0, 15.3, 9.8, 11.2, 12.7, 17.4, 20.6 and 25.0, preferably comprising a characteristic peak at any 2, 4, 6 and 8 of the 2θ;

In certain embodiments of the present invention, the crystal form E of the hydrochloride has an X-ray powder diffraction pattern comprising a characteristic peak at one or more of 2θ (±0.2°) of 9.8, 11.2, 23.0 and 15.3, preferably 2-4 of the 20, more preferably 3-4 of the 20, and most preferably 4 of the 2θ; preferably, further comprising a characteristic peak at one or more of 2θ (±0.2°) of 12.7 and 17.4; further preferably, further comprising a characteristic peak at one or more of 2θ (±0.2°) of 20.6 and 25.0.

For example, the characteristic peaks are at 2θ (±0.2°) of
23.0, 15.3, 9.8 and 11.2;
23.0, 15.3, 9.8, 11.2, 12.7 and 17.4;
23.0, 15.3, 9.8, 11.2, 12.7, 17.4, 20.6 and 25.0;

By using Cu-Kα radiation, the X-ray characteristic diffraction peaks represented by 2θ angles and interplanar spacing d values are shown in Table 6.

**Table 6**

| No. | XRPD data of form E of hydrochloride | | | | | |
|---|---|---|---|---|---|---|
| | 2θ (±0.2°) | d value | Peak height | Proportion (I%) | Area | Proportion (I%) |
| 1 | 8.764 | 10.0815 | 213 | 26.9 | 2443 | 27.9 |
| 2 | 9.757 | 9.0578 | 466 | 58.9 | 4000 | 45.7 |
| 3 | 11.216 | 7.8822 | 434 | 54.9 | 3742 | 42.7 |
| 4 | 12.455 | 7.1007 | 207 | 26.2 | 1999 | 22.8 |
| 5 | 12.72 | 6.9534 | 531 | 67.1 | 4279 | 48.9 |
| 6 | 13.631 | 6.4909 | 130 | 16.4 | 980 | 11.2 |
| 7 | 13.953 | 6.3419 | 71 | 9 | 622 | 7.1 |
| 8 | 15.288 | 5.7907 | 557 | 70.4 | 4463 | 51 |
| 9 | 15.737 | 5.6266 | 323 | 40.8 | 3980 | 45.5 |
| 10 | 15.983 | 5.5407 | 156 | 19.7 | 2507 | 28.6 |
| 11 | 16.323 | 5.426 | 155 | 19.6 | 1032 | 11.8 |
| 12 | 16.85 | 5.2573 | 67 | 8.5 | 209 | 2.4 |
| 13 | 17.362 | 5.1035 | 380 | 48 | 4970 | 56.8 |
| 14 | 17.988 | 4.9273 | 301 | 38.1 | 3053 | 34.9 |
| 15 | 18.88 | 4.6963 | 245 | 31 | 1963 | 22.4 |
| 16 | 19.45 | 4.5601 | 273 | 34.5 | 2243 | 25.6 |
| 17 | 20.323 | 4.366 | 187 | 23.6 | 2392 | 27.3 |
| 18 | 20.563 | 4.3156 | 423 | 53.5 | 4995 | 57.1 |
| 19 | 21.174 | 4.1925 | 191 | 24.1 | 1570 | 17.9 |
| 20 | 21.942 | 4.0475 | 88 | 11.1 | 705 | 8.1 |
| 21 | 22.447 | 3.9575 | 101 | 12.8 | 1066 | 12.2 |
| 22 | 23.0 | 3.8635 | 791 | 100 | 8755 | 100 |
| 23 | 23.465 | 3.788 | 265 | 33.5 | 2718 | 31 |
| 24 | 24.2 | 3.6747 | 99 | 12.5 | 575 | 6.6 |
| 25 | 24.476 | 3.6338 | 127 | 16.1 | 1083 | 12.4 |
| 26 | 25.024 | 3.5555 | 352 | 44.5 | 5946 | 67.9 |
| 27 | 26.741 | 3.3309 | 210 | 26.5 | 3171 | 36.2 |
| 28 | 28.254 | 3.156 | 66 | 8.3 | 436 | 5 |
| 29 | 31.835 | 2.8086 | 127 | 16.1 | 2066 | 23.6 |
| 30 | 32.767 | 2.7308 | 71 | 9 | 834 | 9.5 |

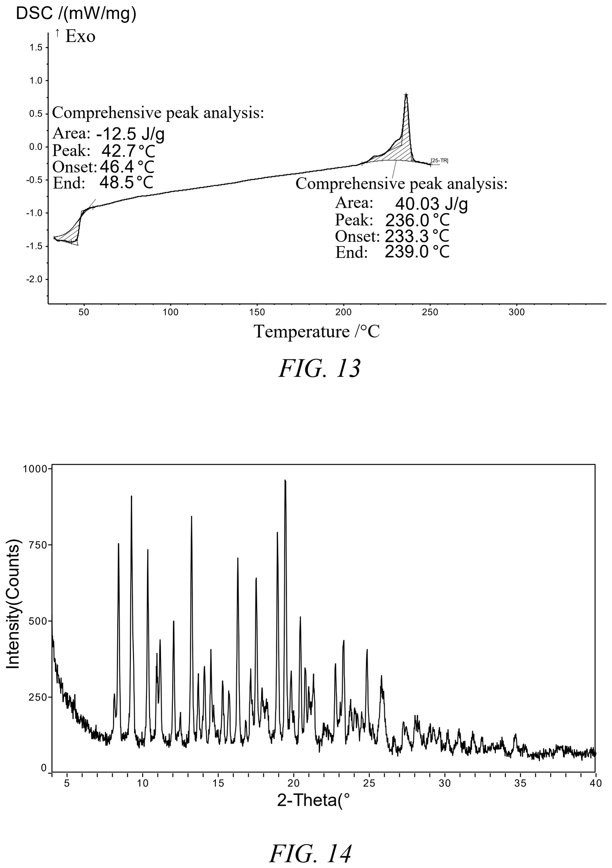

The crystal form E of the hydrochloride of compound 3 of the present invention has an X-ray powder diffraction pattern substantially as shown in FIG. 12, and a DSC pattern substantially as shown in FIG. 13.

In certain embodiments of the present invention, the crystal form F of the hydrochloride has an X-ray powder diffraction pattern comprising a diffraction peak at one or more of 2θ (±0.2°) of 9.3, 19.4, 8.4, 10.3, 13.2, 17.5 and 18.9, preferably comprising a characteristic peak at any 2, 4, 6 and 8 of the 2θ;

In certain embodiments of the present invention, the crystal form F of the hydrochloride has an X-ray powder diffraction pattern comprising a characteristic peak at one or more of 2θ (±0.2°) of 8.4, 9.3, 10.3 and 19.4, preferably 2-4 of the 2θ, more preferably 3-4 of the 2θ, and most preferably 4 of the 2θ; preferably, further comprising a characteristic peak at one or more of 2θ (±0.2°) of 13.2 and 17.5; further preferably, further comprising a characteristic peak at one or more of 2θ (±0.2°) of 18.9.

For example, the characteristic peaks are at 2θ (±0.2°) of
9.3, 19.4, 8.4 and 10.3;
9.3, 19.4, 8.4, 10.3, 13.2 and 17.5;
9.3, 19.4, 8.4, 10.3, 13.2, 17.5 and 18.9;

By using Cu-Kα radiation, the X-ray characteristic diffraction peaks represented by 2θ angles and interplanar spacing d values are shown in Table 7.

**Table 7**

| No. | XRPD data of form F of hydrochloride | | | | | |
|---|---|---|---|---|---|---|
| | 2θ (±0.2°) | d value | Peak height | Proportion (I%) | Area | Proportion (I%) |
| 1 | 8.135 | 10.8601 | 151 | 18.6 | 1539 | 22.7 |
| 2 | 8.403 | 10.5135 | 642 | 79 | 5248 | 77.6 |
| 3 | 9.27 | 9.532 | 805 | 99 | 6765 | 100 |
| 4 | 10.346 | 8.5433 | 617 | 75.9 | 3684 | 54.5 |
| 5 | 10.952 | 8.0717 | 275 | 33.8 | 2309 | 34.1 |
| 6 | 11.158 | 7.9235 | 332 | 40.8 | 3560 | 52.6 |
| 7 | 12.064 | 7.3303 | 387 | 47.6 | 2277 | 33.7 |
| 8 | 12.506 | 7.0723 | 87 | 10.7 | 335 | 5 |
| 9 | 13.244 | 6.6798 | 735 | 90.4 | 4939 | 73 |
| 10 | 13.687 | 6.4646 | 201 | 24.7 | 1013 | 15 |
| 11 | 14.095 | 6.278 | 234 | 28.8 | 1569 | 23.2 |
| 12 | 14.523 | 6.0941 | 298 | 36.7 | 2476 | 36.6 |
| 13 | 14.704 | 6.0195 | 115 | 14.1 | 1145 | 16.9 |
| 14 | 15.295 | 5.788 | 193 | 23.7 | 1253 | 18.5 |
| 15 | 15.703 | 5.6388 | 163 | 20 | 1068 | 15.8 |
| 16 | 16.305 | 5.4317 | 604 | 74.3 | 4130 | 61 |
| 17 | 16.821 | 5.2664 | 66 | 8.1 | 306 | 4.5 |
| 18 | 17.154 | 5.165 | 187 | 23 | 960 | 14.2 |
| 19 | 17.522 | 5.0573 | 492 | 60.5 | 3472 | 51.3 |
| 20 | 17.907 | 4.9494 | 124 | 15.3 | 1975 | 29.2 |
| 21 | 18.232 | 4.8618 | 99 | 12.2 | 1502 | 22.2 |
| 22 | 18.924 | 4.6856 | 676 | 83.1 | 4771 | 70.5 |
| 23 | 19.433 | 4.5639 | 813 | 100 | 5653 | 83.6 |
| 24 | 19.832 | 4.473 | 177 | 21.8 | 1076 | 15.9 |
| 25 | 19.999 | 4.4361 | 66 | 8.1 | 246 | 3.6 |
| 26 | 20.439 | 4.3415 | 377 | 46.4 | 2293 | 33.9 |
| 27 | 20.753 | 4.2766 | 167 | 20.5 | 746 | 11 |
| 28 | 20.993 | 4.2282 | 151 | 18.6 | 3244 | 48 |
| 29 | 21.314 | 4.1653 | 179 | 22 | 1535 | 22.7 |
| 30 | 21.963 | 4.0436 | 49 | 6 | 271 | 4 |
| 31 | 22.768 | 3.9024 | 210 | 25.8 | 1131 | 16.7 |
| 32 | 23.315 | 3.8122 | 290 | 35.7 | 2299 | 34 |
| 33 | 23.764 | 3.7412 | 90 | 11.1 | 219 | 3.2 |
| 34 | 24.032 | 3.7 | 86 | 10.6 | 1201 | 17.8 |
| 35 | 24.227 | 3.6706 | 51 | 6.3 | 589 | 8.7 |
| 36 | 24.502 | 3.63 | 63 | 7.7 | 372 | 5.5 |
| 37 | 24.858 | 3.5789 | 285 | 35.1 | 2855 | 42.2 |

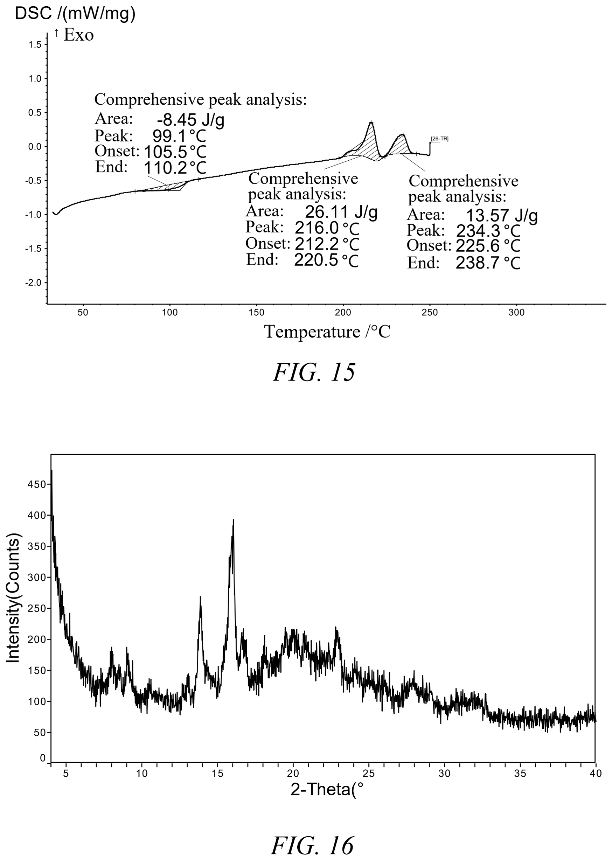

The crystal form F of the hydrochloride of compound 3 of the present invention has an X-ray powder diffraction pattern substantially as shown in FIG. 14, and a DSC pattern substantially as shown in FIG. 15.

In certain embodiments of the present invention, the crystal form G of the hydrochloride has an X-ray powder diffraction pattern comprising a diffraction peak at one or more of 2θ (±0.2°) of 8.0, 9.9, 13.0, 13.9, 16.0, 16.6 and 22.9, preferably comprising a characteristic peak at any 2, 4, 6 and 8 of the 2θ;

In certain embodiments of the present invention, the crystal form G of the hydrochloride has an X-ray powder diffraction pattern comprising a characteristic peak at one or more of 2θ (±0.2°) of 8.0 and 9.9, preferably, further comprising a characteristic peak at one or more of 2θ (±0.2°) of 13.0 and 13.9; further preferably, further comprising a characteristic peak at one or more of 2θ (±0.2°) of 16.0 and 16.6; more preferably, further comprising a characteristic peak at 2θ (±0.2°) of 22.9.

For example, the characteristic peaks are at 2θ (±0.2°) of
8.0, 9.9, 13.0 and 13.9;
8.0, 9.9, 13.0, 13.9, 16.0 and 16.6;
8.0, 9.9, 13.0, 13.9, 16.0, 16.6 and 22.9;

By using Cu-Kα radiation, the X-ray characteristic diffraction peaks represented by 2θ angles and interplanar spacing d values are shown in Table 8.

**Table 8**

| No. | XRPD data of form G of hydrochloride | | | | | |
|---|---|---|---|---|---|---|
| | 2θ (±0.2°) | d value | Peak height | Proportion (I%) | Area | Proportion (I%) |
| 1 | 8.021 | 11.0135 | 57 | 25.4 | 539 | 14.4 |
| 2 | 9.086 | 9.7247 | 58 | 25.9 | 702 | 18.8 |
| 3 | 13.082 | 6.7619 | 45 | 20.1 | 572 | 15.3 |
| 4 | 13.912 | 6.3605 | 124 | 55.4 | 1979 | 53 |
| 5 | 16.058 | 5.5147 | 224 | 100 | 3736 | 100 |
| 6 | 16.606 | 5.3339 | 65 | 29 | 1229 | 32.9 |
| 7 | 18.109 | 4.8947 | 64 | 28.6 | 447 | 12 |
| 8 | 22.918 | 3.8772 | 65 | 29 | 1125 | 30.1 |

The crystal form G of the hydrochloride of compound 3 of the present invention has an X-ray powder diffraction pattern substantially as shown in FIG. 16.

In certain embodiments of the present invention, the crystal form H of the hydrochloride has an X-ray powder diffraction pattern comprising a diffraction peak at one or more of 2θ (±0.2°) of 6.8, 10.0, 11.4, 13.6, 20.0, 21.5, 26.0 and 31.9, preferably comprising a characteristic peak at any 2, 4, 6 and 8 of the 2θ;

In certain embodiments of the present invention, the crystal form H of the hydrochloride has an X-ray powder diffraction pattern comprising a characteristic peak at one or more of 2θ (±0.2°) of 6.8, 10.0, 11.4 and 13.6, preferably 2-4 of the 20, more preferably 3-4 of the 20, and most preferably 4 of the 2θ; preferably, further comprising a characteristic peak at one or more of 2θ (±0.2°) of 20.0 and 21.5; further preferably, further comprising a characteristic peak at one or more of 2θ (±0.2°) of 26.0 and 31.9.

For example, the characteristic peaks are at 2θ (±0.2°) of
6.8, 10.0, 11.4 and 13.6;
6.8, 10.0, 11.4, 13.6, 20.0 and 21.5;
6.8, 10.0, 11.4, 13.6, 20.0, 21.5, 26.0 and 31.9;

By using Cu-Kα radiation, the X-ray characteristic diffraction peaks represented by 2θ angles and interplanar spacing d values are shown in Table 9.

**Table 9**

| No. | XRPD data of form H of hydrochloride | | | | | |
|---|---|---|---|---|---|---|
| | 2θ (±0.2°) | d value | Peak height | Proportion (I%) | Area | Proportion (I%) |
| 1 | 6.837 | 12.9188 | 558 | 11.2 | 6455 | 13.1 |
| 2 | 9.163 | 9.6433 | 212 | 4.3 | 1421 | 2.9 |
| 3 | 9.981 | 8.8544 | 713 | 14.3 | 6558 | 13.3 |
| 4 | 11.403 | 7.7537 | 127 | 2.6 | 1084 | 2.2 |
| 5 | 12.073 | 7.3247 | 55 | 1.1 | 682 | 1.4 |
| 6 | 13.629 | 6.4916 | 131 | 2.6 | 976 | 2 |
| 7 | 14.32 | 6.18 | 132 | 2.7 | 1438 | 2.9 |
| 8 | 15.654 | 5.6561 | 139 | 2.8 | 937 | 1.9 |
| 9 | 16.467 | 5.3787 | 403 | 8.1 | 2916 | 5.9 |
| 10 | 16.835 | 5.2619 | 295 | 5.9 | 2400 | 4.9 |
| 11 | 18.189 | 4.8731 | 229 | 4.6 | 2000 | 4 |
| 12 | 19.589 | 4.5281 | 232 | 4.7 | 2550 | 5.2 |
| 13 | 20.016 | 4.4323 | 4972 | 100 | 49437 | 100 |
| 14 | 21.458 | 4.1376 | 646 | 13 | 10217 | 20.7 |
| 15 | 24.234 | 3.6696 | 74 | 1.5 | 723 | 1.5 |
| 16 | 26.016 | 3.4221 | 451 | 9.1 | 5113 | 10.3 |
| 17 | 27.134 | 3.2836 | 121 | 2.4 | 1339 | 2.7 |
| 18 | 30.211 | 2.9559 | 405 | 8.1 | 4208 | 8.5 |
| 19 | 31.939 | 2.7997 | 498 | 10 | 5489 | 11.1 |
| 20 | 36.072 | 2.4879 | 62 | 1.2 | 1019 | 2.1 |

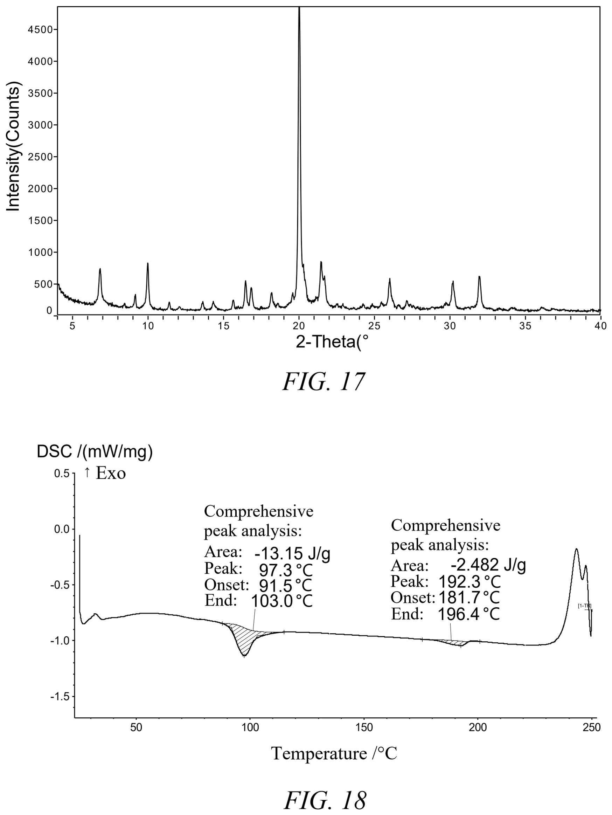

The crystal form H of the hydrochloride of compound 3 of the present invention has an X-ray powder diffraction pattern substantially as shown in FIG. 17, and a DSC pattern substantially as shown in FIG. 18.

In certain embodiments of the present invention, the crystal form I of the hydrochloride has an X-ray powder diffraction pattern comprising a diffraction peak at one or more of 2θ (±0.2°) of 9.5, 19.6, 13.8, 23.0, 24.2, 16.8, 18.0 and 21.2, preferably comprising a characteristic peak at any 2, 4, 6 and 8 of the 2θ;

In certain embodiments of the present invention, the crystal form I of the hydrochloride has an X-ray powder diffraction pattern comprising a characteristic peak at one or more of 2θ (±0.2°) of 9.5, 13.8, 19.6 and 23.0, preferably 2-4 of the 20, more preferably 3-4 of the 20, and most preferably 4 of the 2θ; preferably, further comprising a characteristic peak at one or more of 2θ (±0.2°) of 10.4 and 24.2; further preferably, further comprising a characteristic peak at one or more of 2θ (±0.2°) of 16.8 and 18.0; more preferably, further comprising a characteristic peak at 2θ (±0.2°) of 8.8 and 21.2;

For example, the characteristic peaks are at 2θ (±0.2°) of
9.5 and 19.6;
9.5, 19.6 and 13.8;
9.5, 19.6, 13.8 and 23.0;
9.5, 19.6, 10.4 and 23.0;
9.5, 10.4, 13.8 and 23.0;
10.4, 19.6, 13.8 and 24.2;
9.5, 10.4, 19.6, 13.8, 23.0 and 24.2;
9.5, 10.4, 19.6, 13.8, 23.0 and 16.8;
9.5, 10.4, 19.6, 13.8, 16.8 and 24.2;
9.5, 10.4, 19.6, 18.0, 23.0 and 24.2;
16.8, 10.4, 18.0, 13.8, 23.0 and 24.2;
9.5, 16.8, 18.0, 13.8, 23.0 and 24.2;
9.5, 19.6, 13.8, 23.0, 24.2, 10.4, 16.8 and 18.0;
8.8, 19.6, 13.8, 23.0, 24.2, 10.4, 16.8 and 21.2;
9.5, 19.6, 13.8, 23.0, 24.2, 10.4, 8.8 and 18.0;
9.5, 19.6, 13.8, 23.0, 24.2, 8.8, 21.2 and 18.0;
9.5, 8.8, 13.8, 23.0, 21.2, 10.4, 16.8 and 18.0;
9.5, 19.6, 8.8, 21.2, 24.2, 10.4, 16.8 and 18.0;

By using Cu-Kα radiation, the X-ray characteristic diffraction peaks represented by 2θ angles and interplanar spacing d values are shown in Table 10.

**Table 10**

| No. . | XRPD data of form I of hydrochloride | | | | | |
|---|---|---|---|---|---|---|
| | 2θ (±0.2°) | d value | Peak height | Proportion (I%) | Area | Proportion (I%) |
| 1 | 8.823 | 10.0145 | 402 | 28.5 | 2516 | 29.6 |
| 2 | 9.494 | 9.3078 | 1413 | 100 | 8502 | 100 |
| 3 | 9.757 | 9.0578 | 316 | 22.4 | 1820 | 21.4 |
| 4 | 10.381 | 8.5141 | 424 | 30 | 2551 | 30 |
| 5 | 11.294 | 7.8279 | 220 | 15.6 | 1415 | 16.6 |
| 6 | 12.043 | 7.3428 | 194 | 13.7 | 1196 | 14.1 |
| 7 | 13.773 | 6.4244 | 782 | 55.3 | 4737 | 55.7 |
| 8 | 14.097 | 6.2775 | 120 | 8.5 | 704 | 8.3 |
| 9 | 14.385 | 6.1522 | 157 | 11.1 | 970 | 11.4 |
| 10 | 14.705 | 6.019 | 109 | 7.7 | 742 | 8.7 |
| 11 | 15.113 | 5.8576 | 130 | 9.2 | 912 | 10.7 |
| 12 | 16.347 | 5.4179 | 124 | 8.8 | 1007 | 11.8 |
| 13 | 16.787 | 5.2768 | 630 | 44.6 | 4830 | 56.8 |
| 14 | 17.87 | 4.9594 | 311 | 22 | 2918 | 34.3 |
| 15 | 18.029 | 4.9161 | 609 | 43.1 | 7547 | 88.8 |
| 16 | 19.042 | 4.6569 | 477 | 33.8 | 3214 | 37.8 |
| 17 | 19.567 | 4.533 | 1173 | 83 | 8475 | 99.7 |
| 18 | 19.753 | 4.4908 | 413 | 29.2 | 6046 | 71.1 |
| 19 | 20.214 | 4.3894 | 77 | 5.4 | 342 | 4 |
| 20 | 20.625 | 4.3028 | 350 | 24.8 | 2388 | 28.1 |
| 21 | 21.212 | 4.1851 | 632 | 44.7 | 3924 | 46.2 |
| 22 | 21.682 | 4.0955 | 513 | 36.3 | 3298 | 38.8 |
| 23 | 22.979 | 3.8671 | 752 | 53.2 | 6246 | 73.5 |
| 24 | 24.191 | 3.676 | 597 | 42.3 | 4171 | 49.1 |
| 25 | 24.805 | 3.5864 | 419 | 29.7 | 3371 | 39.6 |
| 26 | 25.626 | 3.4733 | 210 | 14.9 | 2100 | 24.7 |
| 27 | 25.894 | 3.438 | 322 | 22.8 | 2707 | 31.8 |
| 28 | 26.446 | 3.3674 | 415 | 29.4 | 3317 | 39 |
| 29 | 26.809 | 3.3226 | 252 | 17.8 | 2238 | 26.3 |
| 30 | 27.577 | 3.2319 | 239 | 16.9 | 2363 | 27.8 |
| 31 | 28.427 | 3.1371 | 107 | 7.6 | 827 | 9.7 |
| 32 | 28.999 | 3.0765 | 198 | 14 | 1491 | 17.5 |
| 33 | 29.362 | 3.0394 | 247 | 17.5 | 1934 | 22.7 |
| 34 | 30.412 | 2.9368 | 158 | 11.2 | 2031 | 23.9 |
| 35 | 31.265 | 2.8585 | 94 | 6.7 | 1103 | 13 |
| 36 | 32.117 | 2.7846 | 186 | 13.2 | 1653 | 19.4 |
| 37 | 33.258 | 2.6916 | 138 | 9.8 | 2410 | 28.3 |
| 38 | 36.479 | 2.461 | 109 | 7.7 | 1356 | 15.9 |
| 39 | 37.816 | 2.3771 | 116 | 8.2 | 1248 | 14.7 |

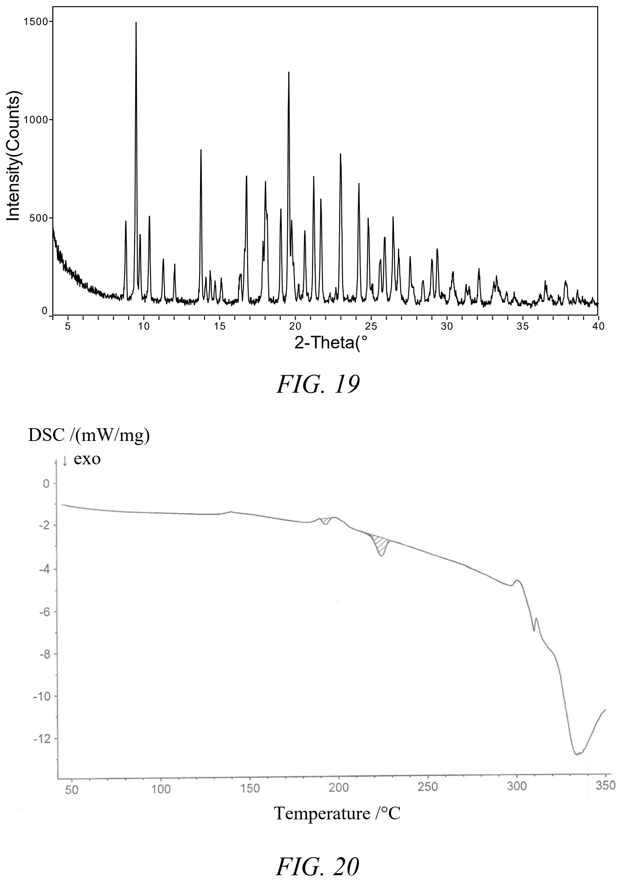

The crystal form I of the hydrochloride of compound 3 of the present invention has an X-ray powder diffraction pattern substantially as shown in FIG. 19, and a DSC pattern substantially as shown in FIG. 20; and the crystal form I of the hydrochloride is a hydrate crystal form. The crystal form I of the hydrochloride of the present invention has good reproducibility and stability and is suitable for industrial production.

In certain embodiments of the present invention, the crystal form J of the hydrochloride has an X-ray powder diffraction pattern comprising a diffraction peak at one or more of 2θ (±0.2°) of 9.5, 19.6, 13.8, 23.0, 21.2, 21.7, 26.5, 7.5, 8.8, 9.5, 18.0 and 18.1, preferably comprising a characteristic peak at any 2, 4, 6 and 8 of the 2θ;

In certain embodiments of the present invention, the crystal form J of the hydrochloride has an X-ray powder diffraction pattern comprising a characteristic peak at one or more of 2θ (±0.2°) of 9.5, 13.8, 19.6 and 23.0, preferably 2-4 of the 20, more preferably 3-4 of the 20, and most preferably 4 of the 2θ; preferably, further comprising a characteristic peak at one or more of 2θ (±0.2°) of 21.2 and 21.7; further preferably, further comprising a characteristic peak at one or more of 2θ (±0.2°) of 7.5 and 26.5; more preferably, further comprising a characteristic peak at one or more of 2θ (±0.2°) of 8.8, 9.5, 18.0 and 18.1.

For example, the characteristic peaks are at 2θ (±0.2°) of
9.5, 19.6, 13.8 and 23.0;
9.5, 19.6, 13.8, 23.0, 21.2, 21.7 and 26.5;
9.5, 19.6, 13.8, 23.0, 21.2, 21.7, 26.5, 7.5, 8.8, 9.5 and 18.1.

By using Cu-Kα radiation, the X-ray characteristic diffraction peaks represented by 2θ angles and interplanar spacing d values are shown in Table 11.

**Table 11**

| No. | XRPD data of form J of hydrochloride | | | | | |
|---|---|---|---|---|---|---|
| | 2θ (±0.2°) | d value | Peak height | Proportion (I%) | Area | Proportion (I%) |
| 1 | 7.487 | 11.7984 | 181 | 30.3 | 1402 | 24.2 |
| 2 | 7.896 | 11.1871 | 76 | 12.7 | 1146 | 19.7 |
| 3 | 8.846 | 9.9886 | 204 | 34.2 | 1311 | 22.6 |
| 4 | 9.514 | 9.2885 | 597 | 100 | 5602 | 96.5 |
| 5 | 10.066 | 8.78 | 89 | 14.9 | 567 | 9.8 |
| 6 | 10.407 | 8.4936 | 171 | 28.6 | 1202 | 20.7 |
| 7 | 10.679 | 8.2771 | 60 | 10.1 | 360 | 6.2 |
| 8 | 11.319 | 7.8105 | 107 | 17.9 | 1063 | 18.3 |
| 9 | 12.066 | 7.3287 | 94 | 15.7 | 444 | 7.6 |
| 10 | 13.793 | 6.4149 | 359 | 60.1 | 2533 | 43.6 |
| 11 | 14.418 | 6.1384 | 107 | 17.9 | 1227 | 21.1 |
| 12 | 15.168 | 5.8364 | 83 | 13.9 | 918 | 15.8 |
| 13 | 15.737 | 5.6265 | 86 | 14.4 | 895 | 15.4 |
| 14 | 16.469 | 5.3783 | 76 | 12.7 | 862 | 14.8 |
| 15 | 16.793 | 5.275 | 268 | 44.9 | 3054 | 52.6 |
| 16 | 18.112 | 4.8937 | 282 | 47.2 | 3707 | 63.9 |
| 17 | 18.597 | 4.7671 | 69 | 11.6 | 658 | 11.3 |
| 18 | 19.083 | 4.647 | 119 | 19.9 | 1047 | 18 |
| 19 | 19.591 | 4.5276 | 422 | 70.7 | 5805 | 100 |
| 20 | 20.627 | 4.3025 | 116 | 19.4 | 1885 | 32.5 |
| 21 | 21.199 | 4.1877 | 247 | 41.4 | 2154 | 37.1 |
| 22 | 21.721 | 4.0881 | 221 | 37 | 2276 | 39.2 |
| 23 | 23.038 | 3.8574 | 311 | 52.1 | 3465 | 59.7 |
| 24 | 24.213 | 3.6727 | 255 | 42.7 | 2385 | 41.1 |
| 25 | 24.824 | 3.5837 | 226 | 37.9 | 2333 | 40.2 |
| 26 | 25.63 | 3.4728 | 109 | 18.3 | 1309 | 22.5 |
| 27 | 25.915 | 3.4352 | 180 | 30.2 | 1784 | 30.7 |
| 28 | 26.485 | 3.3626 | 268 | 44.9 | 2643 | 45.5 |
| 29 | 26.868 | 3.3155 | 82 | 13.7 | 1312 | 22.6 |
| 30 | 27.601 | 3.2292 | 137 | 22.9 | 1733 | 29.9 |
| 31 | 28.391 | 3.1411 | 51 | 8.5 | 322 | 5.5 |
| 32 | 29.016 | 3.0748 | 63 | 10.6 | 670 | 11.5 |
| 33 | 29.402 | 3.0353 | 121 | 20.3 | 772 | 13.3 |
| 34 | 30.436 | 2.9345 | 96 | 16.1 | 1688 | 29.1 |
| 35 | 31.288 | 2.8565 | 60 | 10.1 | 725 | 12.5 |
| 36 | 32.101 | 2.786 | 76 | 12.7 | 945 | 16.3 |
| 37 | 33.256 | 2.6918 | 64 | 10.7 | 1156 | 19.9 |

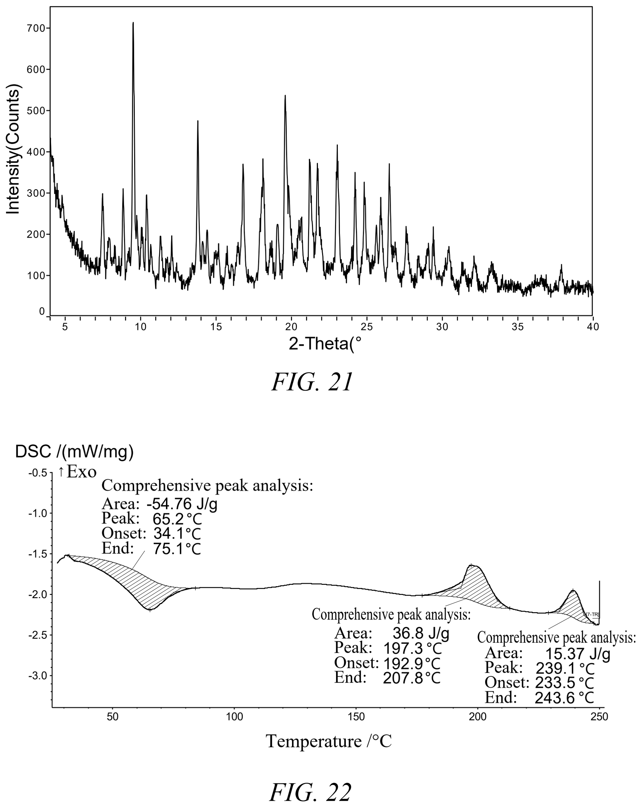

The crystal form J of the hydrochloride of compound 3 of the present invention has an X-ray powder diffraction pattern substantially as shown in FIG. 21, and a DSC pattern substantially as shown in FIG. 22.

In certain embodiments of the present invention, the crystal form A of the methanesulfonate has an X-ray powder diffraction pattern comprising a diffraction peak at one or more of 2θ (±0.2°) of 8.4, 20.0, 13.7, 14.5, 23.6, 20.5 and 23.8, preferably comprising a characteristic peak at any 2, 4 and 6 of the 2θ;

In certain embodiments of the present invention, the crystal form A of the methanesulfonate has an X-ray powder diffraction pattern comprising a characteristic peak at one or more of 2θ (±0.2°) of 8.4 and 20.0, preferably, further comprising a characteristic peak at one or more of 2θ (±0.2°) of 13.7 and 14.5; further preferably, further comprising a characteristic peak at one or more of 2θ (±0.2°) of 20.5 and 23.6; more preferably, further comprising a characteristic peak at 2θ (±0.2°) of 23.8.

For example, the characteristic peaks are at 2θ (±0.2°) of
8.4, 20.0, 13.7 and 14.5;
8.4, 20.0, 13.7, 14.5 and 23.6;
8.4, 20.0, 13.7, 14.5, 23.6, 20.5 and 23.8;

By using Cu-Kα radiation, the X-ray characteristic diffraction peaks represented by 2θ angles and interplanar spacing d values are shown in Table 12.

**Table 12**

| No. | XRPD data of crystal form A of methanesulfonate | | | | | |
|---|---|---|---|---|---|---|
| | 2θ (±0.2°) | d value | Peak height | Proportion (I%) | Area | Proportion (I%) |
| 1 | 8.359 | 10.5684 | 436 | 69.4 | 3014 | 57.1 |
| 2 | 9.274 | 9.5286 | 102 | 16.2 | 811 | 15.4 |
| 3 | 9.533 | 9.2699 | 167 | 26.6 | 1241 | 23.5 |
| 4 | 10.425 | 8.4786 | 208 | 33.1 | 1439 | 27.3 |
| 5 | 13.345 | 6.6292 | 54 | 8.6 | 318 | 6 |
| 6 | 13.732 | 6.4435 | 357 | 56.8 | 2375 | 45 |
| 7 | 14.543 | 6.0858 | 252 | 40.1 | 1895 | 35.9 |
| 8 | 15.658 | 5.6548 | 111 | 17.7 | 1577 | 29.9 |
| 9 | 17.085 | 5.1856 | 37 | 5.9 | 289 | 5.5 |
| 10 | 17.851 | 4.9647 | 106 | 16.9 | 909 | 17.2 |
| 11 | 19.171 | 4.6258 | 123 | 19.6 | 871 | 16.5 |
| 12 | 19.971 | 4.4423 | 628 | 100 | 5279 | 100 |
| 13 | 20.522 | 4.3242 | 305 | 48.6 | 2048 | 38.8 |
| 14 | 21.043 | 4.2182 | 74 | 11.8 | 781 | 14.8 |
| 15 | 22.147 | 4.0104 | 77 | 12.3 | 541 | 10.2 |
| 16 | 23.642 | 3.7601 | 206 | 32.8 | 3410 | 64.6 |
| 17 | 23.885 | 3.7224 | 138 | 22 | 2743 | 52 |
| 18 | 24.673 | 3.6053 | 57 | 9.1 | 796 | 15.1 |
| 19 | 26.427 | 3.3699 | 69 | 11 | 581 | 11 |
| 20 | 28.447 | 3.135 | 38 | 85 | 13.5 | 867 |

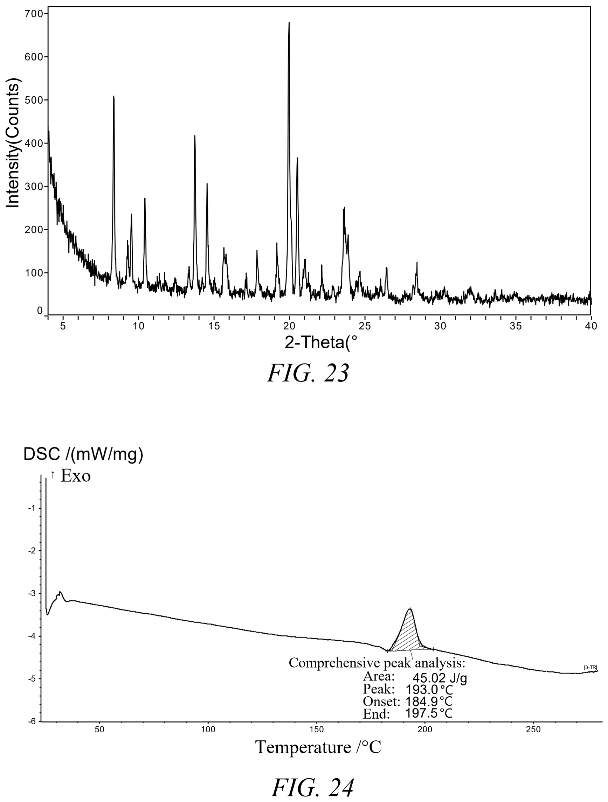

The crystal form A of the methanesulfonate of compound 3 of the present invention has an X-ray powder diffraction pattern substantially as shown in FIG. 23, and a DSC pattern substantially as shown in FIG. 24.

In certain embodiments of the present invention, the crystal form A of the p-toluenesulfonate has an X-ray powder diffraction pattern comprising a diffraction peak at one or more of 2θ (±0.2°) of 7.2, 12.8, 8.6, 10.9, 16.4, 17.7, 10.6, 13.4, 14.0, 18.2, 19.5 and 23.3, preferably comprising a characteristic peak at any 2, 4, 6 and 8 of the 2θ;

In certain embodiments of the present invention, the crystal form A of the p-toluenesulfonate has an X-ray powder diffraction pattern comprising a characteristic peak at one or more of 2θ (±0.2°) of 7.2, 8.6, 10.9 and 12.8, preferably 2-4 of the 2θ, more preferably 3-4 of the 2θ, and most preferably 4 of the 2θ; preferably, further comprising a characteristic peak at one or more of 2θ (±0.2°) of 16.4 and 17.7; further preferably, further comprising a characteristic peak at one or more of 2θ (±0.2°) of 10.6 and 13.4; more preferably, further comprising a characteristic peak at one or more of 2θ (±0.2°) of 14.0, 18.2, 19.5 and 23.3.

For example, the characteristic peaks are at 2θ (±0.2°) of
7.2, 12.8, 8.6 and 10.9;
7.2, 12.8, 8.6, 10.9, 16.4 and 17.7;
7.2, 12.8, 8.6, 10.9, 16.4, 17.7, 10.6, 13.4, 14.0 and 18.2;
7.2, 12.8, 8.6, 10.9, 16.4, 17.7, 10.6, 13.4, 14.0, 18.2, 19.5 and 23.3;

By using Cu-Kα radiation, the X-ray characteristic diffraction peaks represented by 2θ angles and interplanar spacing d values are shown in Table 13.

**Table 13**

| No. | XRPD data of crystal form A of p-toluenesulfonate | | | | | |
|---|---|---|---|---|---|---|
| | 2θ (±0.2°) | d value | Peak height | Proportion (I%) | Area | Proportion (I%) |
| 1 | 7.242 | 12.1972 | 2387 | 100 | 16880 | 100 |
| 2 | 8.642 | 10.2233 | 957 | 40.1 | 6412 | 38 |
| 3 | 10.591 | 8.3462 | 548 | 23 | 4101 | 24.3 |
| 4 | 10.893 | 8.1151 | 1047 | 43.9 | 6762 | 40.1 |
| 5 | 11.607 | 7.6175 | 219 | 9.2 | 1426 | 8.4 |
| 6 | 12.555 | 7.0445 | 97 | 4.1 | 988 | 5.9 |
| 7 | 12.798 | 6.9113 | 418 | 17.5 | 2820 | 16.7 |
| 8 | 13.407 | 6.5986 | 488 | 20.4 | 3126 | 18.5 |
| 9 | 13.952 | 6.342 | 404 | 16.9 | 2711 | 16.1 |
| 10 | 15.191 | 5.8276 | 162 | 6.8 | 1076 | 6.4 |
| 11 | 15.703 | 5.6388 | 157 | 6.6 | 1019 | 6 |
| 12 | 16.426 | 5.3921 | 545 | 22.8 | 5006 | 29.7 |
| 13 | 17.119 | 5.1755 | 182 | 7.6 | 1367 | 8.1 |
| 14 | 17.724 | 4.9999 | 1304 | 54.6 | 9114 | 54 |
| 15 | 18.212 | 4.8672 | 1362 | 57.1 | 12480 | 73.9 |
| 16 | 19.024 | 4.6611 | 62 | 2.6 | 478 | 2.8 |
| 17 | 19.468 | 4.5559 | 497 | 20.8 | 6215 | 36.8 |
| 18 | 19.976 | 4.4412 | 135 | 5.7 | 1926 | 11.4 |
| 19 | 21.252 | 4.1772 | 487 | 20.4 | 4369 | 25.9 |
| 20 | 21.86 | 4.0625 | 163 | 6.8 | 1288 | 7.6 |
| 21 | 22.31 | 3.9814 | 258 | 10.8 | 2230 | 13.2 |
| 22 | 22.874 | 3.8846 | 347 | 14.5 | 2825 | 16.7 |
| 23 | 23.342 | 3.8077 | 822 | 34.4 | 6598 | 39.1 |
| 24 | 23.823 | 3.7319 | 135 | 5.7 | 990 | 5.9 |
| 25 | 25.169 | 3.5354 | 199 | 8.3 | 1683 | 10 |
| 26 | 26.097 | 3.4117 | 100 | 4.2 | 486 | 2.9 |
| 27 | 26.565 | 3.3527 | 92 | 3.9 | 768 | 4.5 |
| 28 | 27.456 | 3.2458 | 96 | 4 | 883 | 5.2 |

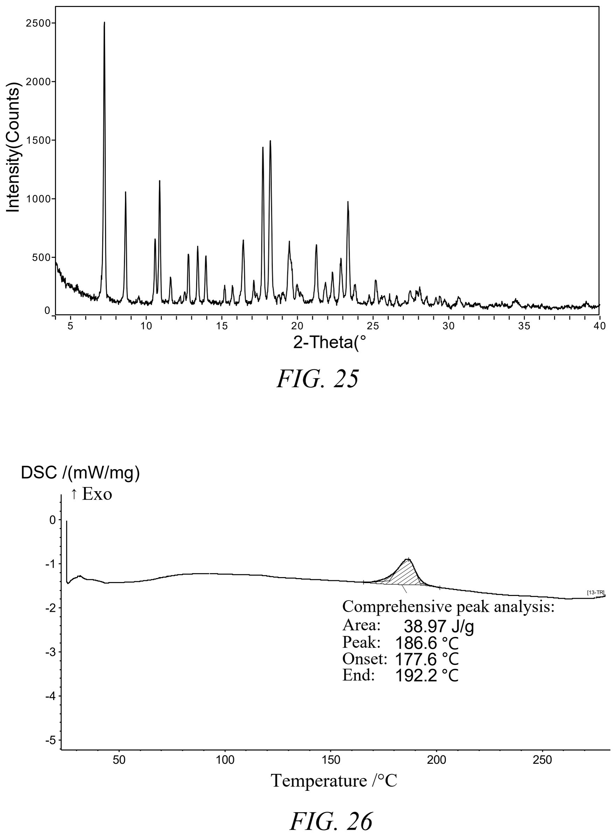

The crystal form A of the p-toluenesulfonate of compound 3 of the present invention has an X-ray powder diffraction pattern substantially as shown in FIG. 25, and a DSC pattern substantially as shown in FIG. 26.

In certain embodiments of the present invention, the crystal form A of the hippurate has an X-ray powder diffraction pattern comprising a diffraction peak at one or more of 2θ (±0.2°) of 7.4, 10.0, 8.1, 14.2, 21.6, 10.5, 20.5 and 25.8, preferably comprising a characteristic peak at any 2, 4, 6 and 8 of the 2θ;

In certain embodiments of the present invention, the crystal form A of the hippurate has an X-ray powder diffraction pattern comprising a characteristic peak at one or more of 2θ (±0.2°) of 7.4, 8.1, 10.0 and 14.2, preferably 2-4 of the 2θ, more preferably 3-4 of the 20, and most preferably 4 of the 2θ; preferably, further comprising a characteristic peak at one or more of 2θ (±0.2°) of 10.5 and 21.6; further preferably, further comprising a characteristic peak at one or more of 2θ (±0.2°) of 20.5 and 25.8.

For example, the characteristic peaks are at 2θ (±0.2°) of
7.4, 10.0, 8.1 and 14.2;
7.4, 10.0, 8.1, 14.2, 21.6, 10.5 and 20.5;
7.4, 10.0, 8.1, 14.2, 21.6, 10.5, 20.5 and 25.8.

By using Cu-Kα radiation, the X-ray characteristic diffraction peaks represented by 2θ angles and interplanar spacing d values are shown in Table 14.

**Table 14**

| No. | XRPD data of crystal form A of hippurate | | | | | |
|---|---|---|---|---|---|---|
| | 2θ (±0.2°) | d value | Peak height | Proportion (I%) | Area | Proportion (I%) |
| 1 | 7.44 | 11.873 | 109 | 100 | 1103 | 86.2 |
| 2 | 7.768 | 11.3715 | 52 | 47.7 | 933 | 72.9 |
| 3 | 8.108 | 10.8957 | 51 | 46.8 | 524 | 41 |
| 4 | 9.514 | 9.2884 | 55 | 50.5 | 285 | 22.3 |
| 5 | 10.039 | 8.8039 | 106 | 97.2 | 573 | 44.8 |
| 6 | 10.532 | 8.3927 | 54 | 49.5 | 298 | 23.3 |
| 7 | 11.829 | 7.4753 | 46 | 42.2 | 278 | 21.7 |
| 8 | 12.296 | 7.1926 | 41 | 37.6 | 331 | 25.9 |
| 9 | 14.232 | 6.2179 | 45 | 41.3 | 523 | 40.9 |
| 10 | 14.945 | 5.9228 | 73 | 67 | 797 | 62.3 |
| 11 | 20.502 | 4.3284 | 62 | 56.9 | 836 | 65.4 |
| 12 | 21.601 | 4.1106 | 55 | 50.5 | 1279 | 100 |
| 13 | 25.837 | 3.4455 | 47 | 43.1 | 428 | 33.5 |

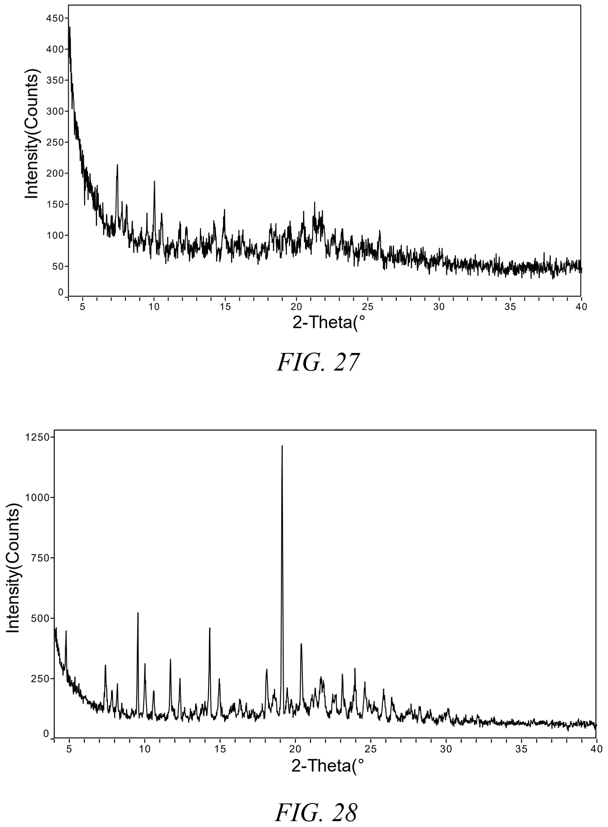

The crystal form A of the hippurate of compound 3 of the present invention has an X-ray powder diffraction pattern substantially as shown in FIG. 27.

In certain embodiments of the present invention, the crystal form A of the malonate has an X-ray powder diffraction pattern comprising a diffraction peak at one or more of 2θ (±0.2°) of 4.8, 19.1, 7.4, 9.5, 12.3, 23.1, 11.7, 14.3, 18.1 and 20.4, preferably comprising a characteristic peak at any 2, 4, 6 and 8 of the 2θ;

In certain embodiments of the present invention, the crystal form A of the malonate has an X-ray powder diffraction pattern comprising a characteristic peak at one or more of 2θ (±0.2°) of 4.8, 7.4, 9.5 and 19.1, preferably 2-4 of the 2θ, more preferably 3-4 of the 20, and most preferably 4 of the 2θ; preferably, further comprising a characteristic peak at one or more of 2θ (±0.2°) of 12.3 and 23.1; further preferably, further comprising a characteristic peak at one or more of 2θ (±0.2°) of 11.7 and 14.3; more preferably, further comprising a characteristic peak at one or more of 2θ (±0.2°) of 18.1 and 20.4.

For example, the characteristic peaks are at 2θ (±0.2°) of
4.8, 19.1, 7.4 and 9.5;
4.8, 19.1, 7.4, 9.5, 12.3 and 23.1;
4.8, 19.1, 7.4, 9.5, 12.3, 23.1, 11.7 and 14.3;
4.8, 19.1, 7.4, 9.5, 12.3, 23.1, 11.7, 14.3, 18.1 and 20.4.

By using Cu-Kα radiation, the X-ray characteristic diffraction peaks represented by 2θ angles and interplanar spacing d values are shown in Table 15.

**Table 15**

| No. | XRPD data of crystal form A of malonate | | | | | |
|---|---|---|---|---|---|---|
| | 2θ (±0.2°) | d value | Peak height | Proportion (I%) | Area | Proportion (I%) |
| 1 | 4.784 | 18.4573 | 188 | 16.9 | 750 | 14.4 |
| 2 | 7.404 | 11.9302 | 188 | 16.9 | 1170 | 22.4 |
| 3 | 7.834 | 11.2753 | 91 | 8.2 | 712 | 13.6 |
| 4 | 8.191 | 10.7848 | 128 | 11.5 | 603 | 11.6 |
| 5 | 9.547 | 9.2559 | 429 | 38.5 | 1802 | 34.5 |
| 6 | 10.022 | 8.8189 | 218 | 19.6 | 1225 | 23.5 |
| 7 | 10.606 | 8.3344 | 115 | 10.3 | 770 | 14.8 |
| 8 | 11.72 | 7.5447 | 249 | 22.4 | 1650 | 31.6 |
| 9 | 12.349 | 7.1618 | 163 | 14.6 | 843 | 16.2 |
| 10 | 13.39 | 6.6069 | 67 | 6 | 724 | 13.9 |
| 11 | 13.998 | 6.3214 | 73 | 6.6 | 1188 | 22.8 |
| 12 | 14.318 | 6.1809 | 367 | 32.9 | 2011 | 38.5 |
| 13 | 14.951 | 5.9206 | 163 | 14.6 | 1342 | 25.7 |
| 14 | 16.308 | 5.4308 | 66 | 5.9 | 457 | 8.8 |
| 15 | 18.108 | 4.895 | 184 | 16.5 | 1363 | 26.1 |
| 16 | 18.617 | 4.7621 | 99 | 8.9 | 1013 | 19.4 |
| 17 | 19.124 | 4.6371 | 1114 | 100 | 5219 | 100 |
| 18 | 19.466 | 4.5563 | 104 | 9.3 | 1165 | 22.3 |
| 19 | 20.4 | 4.3497 | 287 | 25.8 | 2506 | 48 |
| 20 | 21.313 | 4.1655 | 83 | 7.5 | 1000 | 19.2 |
| 21 | 21.699 | 4.0922 | 130 | 11.7 | 1886 | 36.1 |
| 22 | 22.488 | 3.9504 | 84 | 7.5 | 1103 | 21.1 |
| 23 | 22.633 | 3.9253 | 79 | 7.1 | 1103 | 21.1 |
| 24 | 23.121 | 3.8436 | 174 | 15.6 | 1384 | 26.5 |
| 25 | 23.648 | 3.7593 | 63 | 5.7 | 559 | 10.7 |
| 26 | 23.95 | 3.7125 | 204 | 18.3 | 2142 | 41 |
| 27 | 24.615 | 3.6137 | 140 | 12.6 | 1493 | 28.6 |
| 28 | 25.853 | 3.4433 | 125 | 11.2 | 1078 | 20.7 |
| 29 | 26.387 | 3.3749 | 93 | 8.3 | 1028 | 19.7 |
| 30 | 27.68 | 3.2201 | 65 | 5.8 | 932 | 17.9 |

The crystal form A of the malonate of compound 3 of the present invention has an X-ray powder diffraction pattern substantially as shown in FIG. 28.

In certain embodiments of the present invention, the crystal form A of the oxalate has an X-ray powder diffraction pattern comprising a diffraction peak at one or more of 2θ (±0.2°) of 4.8, 9.6, 10.1, 14.4, 19.2, 18.6, 22.1, 22.8 and 24.1, preferably comprising a characteristic peak at any 2, 4, 6 and 8 of the 2θ;

In certain embodiments of the present invention, the crystal form A of the oxalate has an X-ray powder diffraction pattern comprising a characteristic peak at one or more of 2θ (±0.2°) of 4.8, 9.6, 10.1 and 14.4, preferably 2-4 of the 2θ, more preferably 3-4 of the 20, and most preferably 4 of the 2θ; preferably, further comprising a characteristic peak at one or more of 2θ (±0.2°) of 18.6 and 19.2; further preferably, further comprising a characteristic peak at one or more of 2θ (±0.2°) of 22.1 and 22.8; more preferably, further comprising a characteristic peak at 2θ (±0.2°) of 24.1.

For example, the characteristic peaks are at 2θ (±0.2°) of
4.8, 9.6, 10.1 and 14.4;
4.8, 9.6, 10.1, 14.4, 19.2 and 18.6;
4.8, 9.6, 10.1, 14.4, 19.2, 18.6, 22.1, 22.8 and 24.1.

By using Cu-Kα radiation, the X-ray characteristic diffraction peaks represented by 2θ angles and interplanar spacing d values are shown in Table 16.

**Table 16**

| No. | XRPD data of crystal form A of oxalate | | | | | |
|---|---|---|---|---|---|---|
| | 2θ (±0.2°) | d value | Peak height | Proportion (I%) | Area | Proportion (I%) |
| 1 | 4.789 | 18.4371 | 795 | 33.9 | 2997 | 28 |
| 2 | 7.47 | 11.8253 | 248 | 10.6 | 1656 | 15.5 |
| 3 | 7.987 | 11.0605 | 112 | 4.8 | 709 | 6.6 |
| 4 | 8.336 | 10.5975 | 92 | 3.9 | 869 | 8.1 |
| 5 | 9.574 | 9.23 | 2346 | 100 | 10268 | 96.1 |
| 6 | 10.102 | 8.749 | 427 | 18.2 | 2069 | 19.4 |
| 7 | 10.747 | 8.2254 | 251 | 10.7 | 1471 | 13.8 |
| 8 | 11.685 | 7.5673 | 95 | 4 | 839 | 7.9 |
| 9 | 11.939 | 7.4066 | 57 | 2.4 | 269 | 2.5 |
| 10 | 12.492 | 7.0797 | 158 | 6.7 | 1189 | 11.1 |
| 11 | 13.509 | 6.5492 | 61 | 2.6 | 363 | 3.4 |
| 12 | 13.934 | 6.3503 | 87 | 3.7 | 454 | 4.2 |
| 13 | 14.181 | 6.2404 | 113 | 4.8 | 1525 | 14.3 |
| 14 | 14.37 | 6.1587 | 1065 | 45.4 | 6186 | 57.9 |
| 15 | 14.905 | 5.9388 | 113 | 4.8 | 936 | 8.8 |
| 16 | 15.136 | 5.8486 | 106 | 4.5 | 1064 | 10 |
| 17 | 16.041 | 5.5206 | 134 | 5.7 | 985 | 9.2 |
| 18 | 16.483 | 5.3736 | 95 | 4 | 1228 | 11.5 |
| 19 | 17.966 | 4.9331 | 106 | 4.5 | 737 | 6.9 |
| 20 | 18.251 | 4.8568 | 237 | 10.1 | 1565 | 14.6 |
| 21 | 18.619 | 4.7616 | 194 | 8.3 | 1437 | 13.4 |
| 22 | 19.195 | 4.6201 | 2050 | 87.4 | 10686 | 100 |
| 23 | 19.684 | 4.5063 | 44 | 1.9 | 318 | 3 |
| 24 | 20.015 | 4.4327 | 91 | 3.9 | 630 | 5.9 |
| 25 | 20.468 | 4.3354 | 372 | 15.9 | 2599 | 24.3 |
| 26 | 20.881 | 4.2506 | 101 | 4.3 | 542 | 5.1 |
| 27 | 21.46 | 4.1373 | 155 | 6.6 | 1530 | 14.3 |
| 28 | 22.063 | 4.0256 | 216 | 9.2 | 1315 | 12.3 |
| 29 | 22.795 | 3.8978 | 277 | 11.8 | 1433 | 13.4 |
| 30 | 23.093 | 3.8483 | 92 | 3.9 | 580 | 5.4 |
| 31 | 23.438 | 3.7924 | 164 | 7 | 965 | 9 |
| 32 | 24.052 | 3.697 | 281 | 12 | 2554 | 23.9 |
| 33 | 24.71 | 3.6 | 145 | 6.2 | 1282 | 12 |
| 34 | 25.394 | 3.5045 | 186 | 7.9 | 1770 | 16.6 |
| 35 | 25.918 | 3.4349 | 188 | 8 | 1894 | 17.7 |
| 36 | 27.559 | 3.234 | 105 | 4.5 | 1116 | 10.4 |
| 37 | 28.009 | 3.183 | 65 | 2.8 | 423 | 4 |
| 38 | 29.767 | 2.9989 | 94 | 4 | 635 | 5.9 |
| 39 | 30.138 | 2.9628 | 121 | 5.2 | 952 | 8.9 |

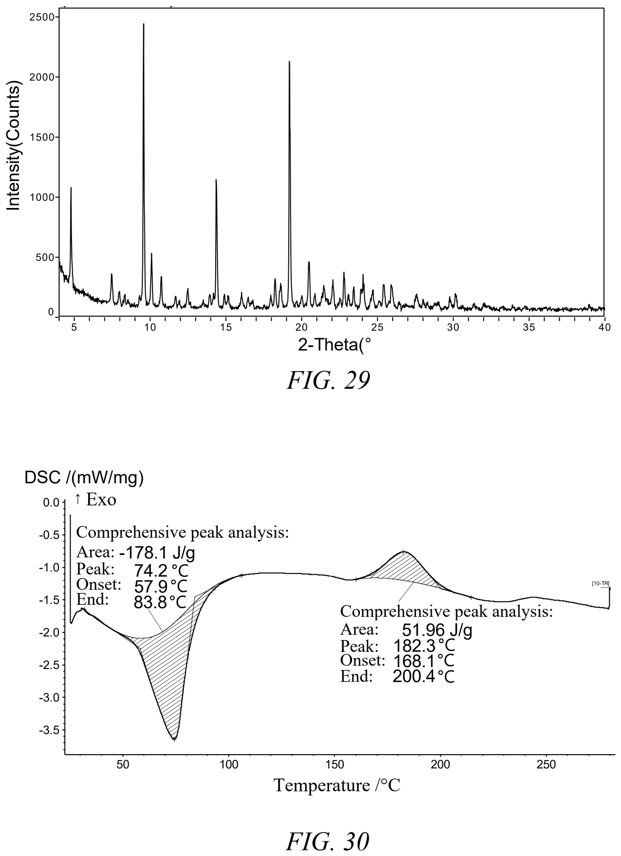

The crystal form A of the oxalate of compound 3 of the present invention has an X-ray powder diffraction pattern substantially as shown in FIG. 29, and a DSC pattern substantially as shown in FIG. 30.

In certain embodiments of the present invention, the crystal form A of the adipate has an X-ray powder diffraction pattern comprising a diffraction peak at one or more of 2θ (±0.2°) of 7.4, 10.0, 7.7, 12.3, 19.5, 21.7, 25.8, 1.8, 14.2, 14.9, 20.5, 21.2, 21.5 and 23.2, preferably comprising a characteristic peak at any 2, 4, 6 and 8 of the 2θ;

In certain embodiments of the present invention, the crystal form A of the adipate has an X-ray powder diffraction pattern comprising a characteristic peak at one or more of 2θ (±0.2°) of 7.4, 7.7, 10.0 and 12.3, preferably 2-4 of the 2θ, more preferably 3-4 of the 20, and most preferably 4 of the 2θ; preferably, further comprising a characteristic peak at one or more of 2θ (±0.2°) of 19.5 and 21.7; further preferably, further comprising a characteristic peak at one or more of 2θ (±0.2°) of 1.8 and 25.8; more preferably, further comprising a characteristic peak at one or more of 2θ (±0.2°) of 14.2, 14.9, 20.5, 21.2, 21.5 and 23.2.

For example, the characteristic peaks are at 2θ (±0.2°) of
7.4, 10.0, 7.7 and 12.3;
7.4, 10.0, 7.7, 12.3, 19.5 and 21.7;
7.4, 10.0, 7.7, 12.3, 19.5, 21.7, 25.8 and 1.8;
7.4, 10.0, 7.7, 12.3, 19.5, 21.7, 25.8, 1.8, 14.2, 14.9, 20.5, 21.2, 21.5 and 23.2;

By using Cu-Kα radiation, the X-ray characteristic diffraction peaks represented by 2θ angles and interplanar spacing d values are shown in Table 17.

**Table 17**

| No. | XRPD data of crystal form A of adipate | | | | | |
|---|---|---|---|---|---|---|
| | 2θ (±0.2°) | d value | Peak height | Proportion (I%) | Area | Proportion (I%) |
| 1 | 7.423 | 11.8989 | 323 | 100 | 1814 | 35.7 |
| 2 | 7.716 | 11.4478 | 123 | 38.1 | 789 | 15.5 |
| 3 | 8.083 | 10.9293 | 133 | 41.2 | 776 | 15.3 |
| 4 | 8.485 | 10.4122 | 75 | 23.2 | 368 | 7.2 |
| 5 | 9.052 | 9.7617 | 45 | 13.9 | 377 | 7.4 |
| 6 | 9.51 | 9.2925 | 98 | 30.3 | 530 | 10.4 |
| 7 | 10.02 | 8.8205 | 308 | 95.4 | 1526 | 30 |
| 8 | 10.511 | 8.4098 | 109 | 33.7 | 697 | 13.7 |
| 9 | 11.75 | 7.5254 | 142 | 44 | 844 | 16.6 |
| 10 | 12.254 | 7.2171 | 143 | 44.3 | 609 | 12 |
| 11 | 13.264 | 6.6693 | 79 | 24.5 | 783 | 15.4 |
| 12 | 13.805 | 6.4093 | 62 | 19.2 | 320 | 6.3 |
| 13 | 14.215 | 6.2253 | 166 | 51.4 | 1798 | 35.4 |
| 14 | 14.903 | 5.9396 | 249 | 77.1 | 2310 | 45.5 |
| 15 | 15.149 | 5.8435 | 64 | 19.8 | 298 | 5.9 |
| 16 | 15.474 | 5.7215 | 60 | 18.6 | 526 | 10.4 |
| 17 | 16.241 | 5.4532 | 90 | 27.9 | 620 | 12.2 |
| 18 | 16.746 | 5.2897 | 66 | 20.4 | 426 | 8.4 |
| 19 | 17.088 | 5.1847 | 81 | 25.1 | 817 | 16.1 |
| 20 | 18.129 | 4.8893 | 174 | 53.9 | 1823 | 35.9 |
| 21 | 18.457 | 4.8032 | 102 | 31.6 | 951 | 18.7 |
| 22 | 18.704 | 4.7401 | 68 | 21.1 | 332 | 6.5 |
| 23 | 19.06 | 4.6525 | 139 | 43 | 744 | 14.6 |
| 24 | 19.491 | 4.5506 | 162 | 50.2 | 1843 | 36.3 |
| 25 | 20.459 | 4.3373 | 207 | 64.1 | 2185 | 43 |
| 26 | 21.192 | 4.189 | 216 | 66.9 | 3835 | 75.5 |
| 27 | 21.517 | 4.1265 | 178 | 55.1 | 5080 | 100 |
| 28 | 21.701 | 4.0919 | 211 | 65.3 | 2210 | 43.5 |
| 29 | 22.429 | 3.9607 | 153 | 47.4 | 1225 | 24.1 |
| 30 | 22.594 | 3.9321 | 116 | 35.9 | 990 | 19.5 |
| 31 | 23.156 | 3.8379 | 152 | 47.1 | 1019 | 20.1 |
| 32 | 23.77 | 3.7402 | 138 | 42.7 | 1067 | 21 |
| 33 | 24.535 | 3.6253 | 75 | 23.2 | 1324 | 26.1 |
| 34 | 24.908 | 3.5718 | 76 | 23.5 | 1254 | 24.7 |
| 35 | 25.755 | 3.4562 | 137 | 42.4 | 1375 | 27.1 |
| 36 | 26.37 | 3.377 | 69 | 21.4 | 764 | 15 |

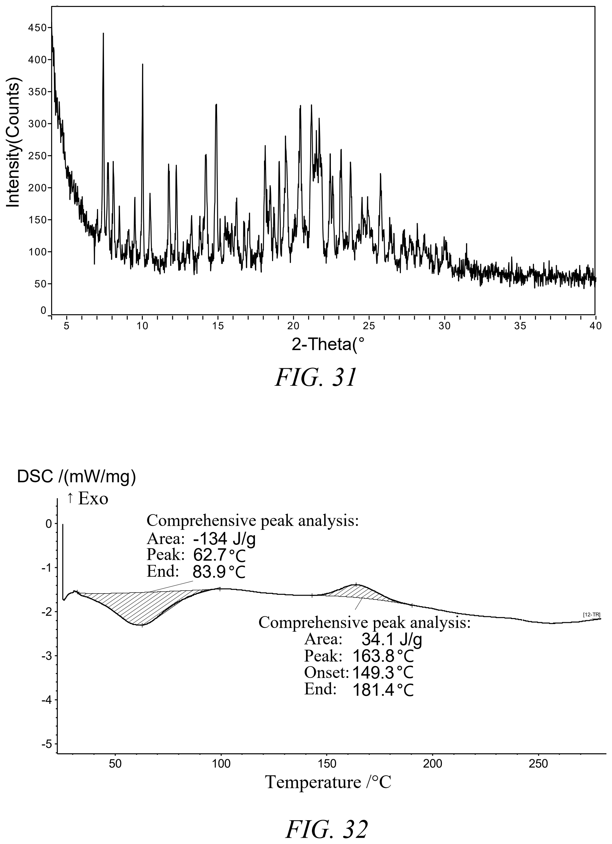

The crystal form A of the adipate of compound 3 of the present invention has an X-ray powder diffraction pattern substantially as shown in FIG. 31, and a DSC pattern substantially as shown in FIG. 32.

In certain embodiments of the present invention, the crystal form A of the succinate has an X-ray powder diffraction pattern comprising a diffraction peak at one or more of 2θ (±0.2°) of 7.4, 10.0, 7.8, 9.5, 19.1, 20.5, 8.1, 11.8, 18.2 and 25.9, preferably comprising a characteristic peak at any 2, 4, 6 and 8 of the 2θ;

In certain embodiments of the present invention, the crystal form A of the succinate has an X-ray powder diffraction pattern comprising a characteristic peak at one or more of 2θ (±0.2°) of 7.4, 7.8, 9.5 and 10.0, preferably 2-4 of the 2θ, more preferably 3-4 of the 20, and most preferably 4 of the 2θ; preferably, further comprising a characteristic peak at one or more of 2θ (±0.2°) of 19.1 and 20.5; further preferably, further comprising a characteristic peak at one or more of 2θ (±0.2°) of 8.1 and 11.8; more preferably, further comprising a characteristic peak at 2θ (±0.2°) of 18.2 and 25.9.

For example, the characteristic peaks are at 2θ (±0.2°) of
7.4, 10.0, 7.8 and 9.5;
7.4, 10.0, 7.8, 9.5, 19.1 and 20.5;
7.4, 10.0, 7.8, 9.5, 19.1, 20.5, 8.1 and 11.8;
7.4, 10.0, 7.8, 9.5, 19.1, 20.5, 8.1, 11.8, 18.2 and 25.9;

By using Cu-Kα radiation, the X-ray characteristic diffraction peaks represented by 2θ angles and interplanar spacing d values are shown in Table 18.

**Table 18**

| No. | XRPD data of crystal form A of succinate | | | | | |
|---|---|---|---|---|---|---|
| | 2θ (±0.2°) | d value | Peak height | Proportion (I%) | Area | Proportion (I%) |
| 1 | 7.445 | 11.8646 | 165 | 70.5 | 1234 | 53 |
| 2 | 7.752 | 11.395 | 71 | 30.3 | 935 | 40.2 |
| 3 | 8.119 | 10.8812 | 73 | 31.2 | 357 | 15.3 |
| 4 | 9.536 | 9.2666 | 158 | 67.5 | 801 | 34.4 |
| 5 | 10.04 | 8.8026 | 234 | 100 | 1427 | 61.3 |
| 6 | 10.549 | 8.3795 | 91 | 38.9 | 548 | 23.5 |
| 7 | 11.802 | 7.4925 | 195 | 83.3 | 1480 | 63.6 |
| 8 | 12.322 | 7.1771 | 92 | 39.3 | 490 | 21.1 |
| 9 | 13.229 | 6.6869 | 36 | 15.4 | 426 | 18.3 |
| 10 | 13.838 | 6.3943 | 51 | 21.8 | 448 | 19.3 |
| 11 | 14.299 | 6.189 | 110 | 47 | 1180 | 50.7 |
| 12 | 14.967 | 5.9144 | 141 | 60.3 | 1327 | 57 |
| 13 | 15.179 | 5.8321 | 70 | 29.9 | 445 | 19.1 |
| 14 | 16.301 | 5.4331 | 63 | 26.9 | 458 | 19.7 |
| 15 | 16.798 | 5.2737 | 97 | 41.5 | 819 | 35.2 |
| 16 | 18.173 | 4.8776 | 162 | 69.2 | 1570 | 67.5 |
| 17 | 18.781 | 4.7209 | 84 | 35.9 | 323 | 13.9 |
| 18 | 19.139 | 4.6333 | 196 | 83.8 | 1194 | 51.3 |
| 19 | 19.551 | 4.5366 | 92 | 39.3 | 808 | 34.7 |
| 20 | 20.521 | 4.3244 | 232 | 99.1 | 2327 | 100 |
| 21 | 21.326 | 4.163 | 85 | 36.3 | 977 | 42 |
| 22 | 21.822 | 4.0694 | 123 | 52.6 | 1252 | 53.8 |
| 23 | 22.69 | 3.9156 | 115 | 49.1 | 1405 | 60.4 |
| 24 | 23.204 | 3.8301 | 114 | 48.7 | 940 | 40.4 |
| 25 | 23.903 | 3.7197 | 110 | 47 | 1152 | 49.5 |
| 26 | 24.777 | 3.5903 | 69 | 29.5 | 982 | 42.2 |
| 27 | 25.878 | 3.4401 | 101 | 43.2 | 1179 | 50.7 |
| 28 | 26.483 | 3.3629 | 61 | 26.1 | 967 | 41.6 |
| 29 | 30.154 | 2.9612 | 56 | 23.9 | 751 | 32.3 |

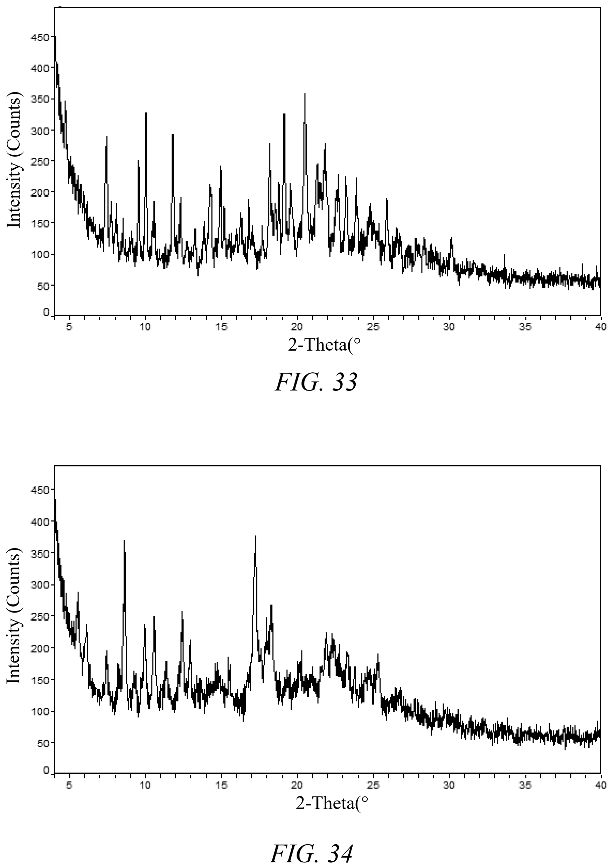

The crystal form A of the succinate of compound 3 of the present invention has an X-ray powder diffraction pattern substantially as shown in FIG. 33.

In certain embodiments of the present invention, the crystal form A of the phosphate has an X-ray powder diffraction pattern comprising a diffraction peak at one or more of 2θ (±0.2°) of 5.5, 8.6, 7.5, 12.9, 18.3, 10.6, 12.4, 17.3 and 25.3, preferably comprising a characteristic peak at any 2, 4, 6 and 8 of the 2θ;

In certain embodiments of the present invention, the crystal form A of the phosphate has an X-ray powder diffraction pattern comprising a characteristic peak at one or more of 2θ (±0.2°) of 5.5, 8.6, 7.5 and 12.9, preferably 2-4 of the 2θ, more preferably 3-4 of the 20, and most preferably 4 of the 2θ; preferably, further comprising a characteristic peak at one or more of 2θ (±0.2°) of 10.6 and 18.3; further preferably, further comprising a characteristic peak at one or more of 2θ (±0.2°) of 12.4 and 17.3; more preferably, further comprising a characteristic peak at 2θ (±0.2°) of 25.3.

For example, the characteristic peaks are at 2θ (±0.2°) of
5.5, 8.6, 7.5 and 12.9;
5.5, 8.6, 7.5, 12.9, 18.3 and 10.6;
5.5, 8.6, 7.5, 12.9, 18.3, 10.6, 12.4, 17.3 and 25.3;

By using Cu-Kα radiation, the X-ray characteristic diffraction peaks represented by 2θ angles and interplanar spacing d values are shown in Table 19.

**Table 19**

| No. | XRPD data of crystal form A of phosphate | | | | | |
|---|---|---|---|---|---|---|
| | 2θ (±0.2°) | d value | Peak height | Proportion (I%) | Area | Proportion (I%) |
| 1 | 5.546 | 15.9215 | 87 | 34.3 | 680 | 21.3 |
| 2 | 6.142 | 14.377 | 84 | 33.1 | 844 | 26.5 |
| 3 | 7.464 | 11.8344 | 80 | 31.5 | 826 | 25.9 |
| 4 | 8.624 | 10.2453 | 254 | 100 | 2471 | 77.5 |
| 5 | 9.958 | 8.8749 | 132 | 52 | 1616 | 50.7 |
| 6 | 10.572 | 8.361 | 143 | 56.3 | 1397 | 43.8 |
| 7 | 11.38 | 7.7693 | 70 | 27.6 | 984 | 30.9 |
| 8 | 12.431 | 7.1147 | 134 | 52.8 | 1490 | 46.7 |
| 9 | 12.937 | 6.8374 | 88 | 34.6 | 651 | 20.4 |
| 10 | 17.258 | 5.1341 | 225 | 88.6 | 3189 | 100 |
| 11 | 17.985 | 4.9281 | 92 | 36.2 | 1929 | 60.5 |
| 12 | 18.292 | 4.846 | 121 | 47.6 | 2069 | 64.9 |
| 13 | 21.919 | 4.0517 | 69 | 27.2 | 1054 | 33.1 |
| 14 | 22.308 | 3.9818 | 54 | 21.3 | 453 | 14.2 |
| 15 | 23.285 | 3.8169 | 47 | 18.5 | 408 | 12.8 |
| 16 | 25.305 | 3.5166 | 77 | 30.3 | 996 | 31.2 |

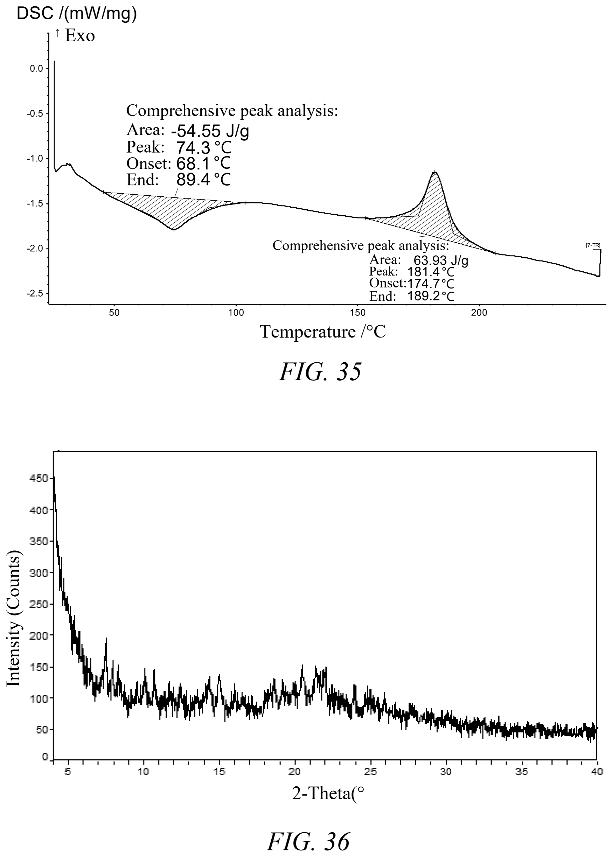

The crystal form A of the phosphate of compound 3 of the present invention has an X-ray powder diffraction pattern substantially as shown in FIG. 34, and a DSC pattern substantially as shown in FIG. 35.

In certain embodiments of the present invention, the crystal form A of the fumarate has an X-ray powder diffraction pattern comprising a diffraction peak at one or more of 2θ (±0.2°) of 7.5, 10.7, 7.9, 14.4, 15.0, 8.3, 10.0 and 20.5, preferably comprising a characteristic peak at any 2, 4, 6 and 8 of the 2θ;

In certain embodiments of the present invention, the crystal form A of the fumarate has an X-ray powder diffraction pattern comprising a characteristic peak at one or more of 2θ (±0.2°) of 7.5, 10.7, 7.9 and 14.4, preferably 2-4 of the 2θ, more preferably 3-4 of the 20, and most preferably 4 of the 2θ; preferably, further comprising a characteristic peak at one or more of 2θ (±0.2°) of 8.3 and 15.0; further preferably, further comprising a characteristic peak at one or more of 2θ (±0.2°) of 10.0 and 20.5.

For example, the characteristic peaks are at 2θ (±0.2°) of
7.5, 10.7, 7.9 and 14.4;
7.5, 10.7, 7.9, 14.4, 15.0 and 8.3;
7.5, 10.7, 7.9, 14.4, 15.0, 8.3, 10.0 and 20.5.

By using Cu-Kα radiation, the X-ray characteristic diffraction peaks represented by 2θ angles and interplanar spacing d values are shown in Table 20.

**Table 20**

| No. | XRPD data of crystal form A of fumarate | | | | | |
|---|---|---|---|---|---|---|
| | 2θ (±0.2°) | d value | Peak height | Proportion (I%) | Area | Proportional (I%) |
| 1 | 7.5 | 11.777 | 85 | 100 | 904 | 87.3 |
| 2 | 7.946 | 11.117 | 51 | 60 | 238 | 23 |
| 3 | 8.254 | 10.7036 | 39 | 45.9 | 617 | 59.6 |
| 4 | 10.065 | 8.7809 | 50 | 58.8 | 303 | 29.3 |
| 5 | 10.707 | 8.2557 | 54 | 63.5 | 306 | 29.6 |
| 6 | 14.358 | 6.1636 | 54 | 63.5 | 874 | 84.4 |
| 7 | 15.008 | 5.8984 | 62 | 72.9 | 1035 | 100 |
| 8 | 20.519 | 4.3249 | 53 | 62.4 | 546 | 52.8 |

The crystal form A of the fumarate of compound 3 of the present invention has an X-ray powder diffraction pattern substantially as shown in FIG. 36.

In a further preferred embodiment of the present invention, the above-described crystal forms are solvent-containing crystal forms, wherein the solvent is selected from water, methanol, acetone, ethyl acetate, acetonitrile, ethanol, 88% acetone, 2-methyl-tetrahydrofuran, dichloromethane, 1,4-dioxane, benzene, toluene, isopropanol, n-butanol, isobutanol, N,N-dimethylformamide, N,N-dimethylacetamide, dimethyl sulfoxide, n-propanol, tert-butanol, 2-butanone, 3-pentanone, n-heptane, ethyl formate, isopropyl acetate, cyclohexane, methyl tert-butyl ether or isopropyl ether.

In a further preferred embodiment of the present invention, the number of the solvents is 0.2-3, preferably 0.2, 0.5, 1, 1.5, 2, 2.5 or 3, more preferably 0.5, 1, 2 or 3.

The present invention further provides a method for preparing the compound as shown in general formula (I) or the stereoisomer and crystalline salt form thereof, wherein the method specifically comprises the following steps:
1) weighing an appropriate amount of a free base and dissolving the free base in a benign solvent;
2) weighing an appropriate amount of a counterion acid and dissolving the counterion acid in an organic solvent, wherein the amount of the counterion acid is preferably 1 equivalent;
3) combining the above-described two solutions, and stirring to allow precipitation, or dropwise adding a poor solvent followed by stirring to allow precipitation; and
4) rapidly centrifuging the resultant or leaving the resultant to stand for blow drying to obtain a target product,
wherein
the benign solvent is selected from methanol, dichloromethane, 1,4-dioxane, acetone, tetrahydrofuran, N,N-dimethylformamide, ethyl acetate, N,N-dimethylacetamide or N-methylpyrrolidone, preferably methanol or ethyl acetate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, acetone or ethyl acetate; the above-described benign solvent and organic solution must be miscible when used;
the poor solvent is selected from heptane, water and cyclohexane, preferably water and n-heptane.

The present invention further provides a method for preparing the compound as shown in general formula (I) or the stereoisomer and crystalline salt form thereof, wherein the method specifically comprises the following steps:
1) weighing an appropriate amount of a free base, and suspending the free base in a poor solvent;
2) weighing an appropriate amount of a counterion acid and dissolving the counterion acid in an organic solvent, wherein the amount of the counterion acid is preferably 1 equivalent;
3) combining the above-described two solutions with stirring until dissolved, and further stirring to allow precipitation, or dropwise adding a poor solvent followed by further stirring to allow precipitation; and
4) rapidly centrifuging the resultant or leaving the resultant to stand for blow drying to obtain a target product,

wherein the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol;
the poor solvent is selected from heptane, acetonitrile, ethanol, methyl tert-butyl ether, 2-methyltetrahydrofuran, ethyl acetate, isopropyl acetate or toluene, preferably methyl tert-butyl ether; the above-described poor solvent and organic solution must be miscible when used;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, embonic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, or L-malic acid, preferably methanesulphonic acid, p-toluenesulphonic acid or hydrochloric acid, and most preferably hydrochloric acid.

Another objective of the present invention is to provide a pharmaceutical composition comprising a therapeutically effective amount of the above-described acid salt of the compound, and one or more pharmaceutically acceptable carriers, diluents or excipients.

The present invention further relates to a method for treating a disease or condition associated with activation of a complement alternative pathway in a subject in need thereof by modulating complement factor B, wherein the method comprises administering to the subject an effective amount of the above-described acid salt of the compound or the above-described pharmaceutical composition.

Further, the disease or condition is selected from age-related macular degeneration, geographic atrophy, diabetic retinopathy, uveitis, retinitis pigmentosa, macular oedema, Behçet's uveitis, multifocal choroiditis, Vogt-Koyangi-Harada syndrome, intermediate uveitis, birdshot retinochoroidopathy, sympathetic ophthalmia, ocular pemphigoid, ocular pemphigus, retinal vein occlusion, neurological diseases, multiple sclerosis, stroke, Guillain-Barré syndrome, traumatic brain injury, Parkinson's disease, disorders of inappropriate or undesirable complement activation, haemodialysis complications, hyperacute allograft rejection, xenograft rejection, interleukin-2-induced toxicity during IL-2 therapy, inflammatory diseases, inflammation in autoimmune diseases, Crohn's disease, adult respiratory distress syndrome, myocarditis, postischemic reperfusion conditions, myocardial infarction, balloon angioplasty, post-pump syndrome associated with cardiopulmonary bypass or renal bypass, atherosclerosis, haemodialysis, renal ischemia, mesenteric artery reperfusion after aortic reconstruction, infectious diseases or sepsis, immune complex diseases and autoimmune diseases, rheumatoid arthritis, systemic lupus erythematosus, SLE nephritis, proliferative nephritis, liver fibrosis, haemolytic anaemia, myasthenia gravis, tissue regeneration, nerve regeneration, dyspnoea, haemoptysis, ARDS, asthma, chronic obstructive pulmonary disease, emphysema, pulmonary embolism and infarction, pneumonia, fibrogenic dust disease, pulmonary fibrosis, asthma, allergy, bronchoconstriction, hypersensitivity pneumonitis, parasitic diseases, Goodpasture syndrome, pulmonary vasculitis, pauci-immune vasculitis, immune complex-related inflammation, antiphospholipid syndrome, primary glomerulonephritis (IgAN), obesity, C3 glomerulonephritis (C3G), lupus nephritis (LN), immunoglobulin A (IgA) nephropathy, paroxysmal nocturnal haemoglobinuria (PNH), atypical haemolytic uremic syndrome (aHUS), early to intermediate age-related macular degeneration (e/i AMD), idiopathic membranous nephropathy, and immune complex membranoproliferative glomerulonephritis (IC-MPGN).

### Brief Description of the Drawings

FIG. 1 shows an XRPD diagram of crystal form A of hydrobromide.
FIG. 2 shows a DSC diagram of crystal form A of hydrobromide.

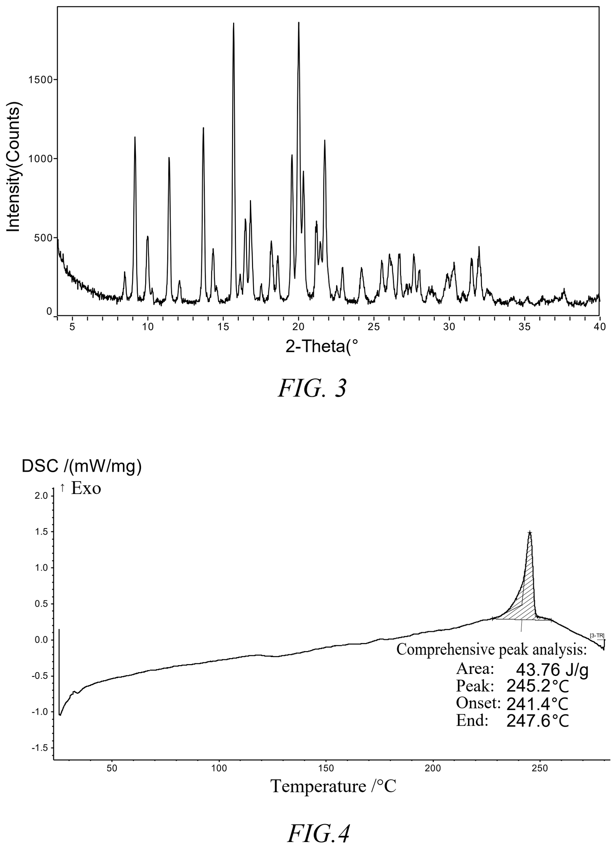

FIG. 3 shows an XRPD diagram of crystal form A of hydrochloride.
FIG. 4 shows a DSC diagram of crystal form A of hydrochloride.
FIG. 5 shows a TGA diagram of crystal form A of hydrochloride.
FIG. 6 shows an XRPD diagram of crystal form B of hydrochloride.
FIG. 7 shows a DSC diagram of crystal form B of hydrochloride.
FIG. 8 shows an XRPD diagram of crystal form C of hydrochloride.
FIG. 9 shows a DSC diagram of crystal form C of hydrochloride.
FIG. 10 shows an XRPD diagram of crystal form D of hydrochloride.
FIG. 11 shows a DSC diagram of crystal form D of hydrochloride.
FIG. 12 shows an XRPD diagram of crystal form E of hydrochloride.
FIG. 13 shows a DSC diagram of crystal form E of hydrochloride.
FIG. 14 shows an XRPD diagram of crystal form F of hydrochloride.
FIG. 15 shows a DSC diagram of crystal form F of hydrochloride.
FIG. 16 shows an XRPD diagram of crystal form G of hydrochloride.
FIG. 17 shows an XRPD diagram of crystal form H of hydrochloride.
FIG. 18 shows a DSC diagram of crystal form H of hydrochloride.
FIG. 19 shows an XRPD diagram of crystal form I of hydrochloride.
FIG. 20 shows a DSC diagram of crystal form I of hydrochloride.
FIG. 21 shows an XRPD diagram of crystal form J of hydrochloride.
FIG. 22 shows a DSC diagram of crystal form J of hydrochloride.
FIG. 23 shows an XRPD diagram of crystal form A of methanesulfonate.
FIG. 24 shows a DSC diagram of crystal form A of methanesulfonate.
FIG. 25 shows an XRPD diagram of crystal form A of p-toluenesulfonate.
FIG. 26 shows a DSC diagram of crystal form A of p-toluenesulfonate.
FIG. 27 shows an XRPD diagram of crystal form A of hippurate.
FIG. 28 shows an XRPD diagram of crystal form A of malonate.
FIG. 29 shows an XRPD diagram of crystal form A of oxalate.
FIG. 30 shows a DSC diagram of crystal form A of oxalate.
FIG. 31 shows an XRPD diagram of crystal form A of adipate.
FIG. 32 shows a DSC diagram of crystal form A of adipate.
FIG. 33 shows an XRPD diagram of crystal form A of succinate.
FIG. 34 shows an XRPD diagram of crystal form A of phosphate.
FIG. 35 shows a DSC diagram of crystal form A of phosphate.
FIG. 36 shows an XRPD diagram of crystal form A of fumarate.

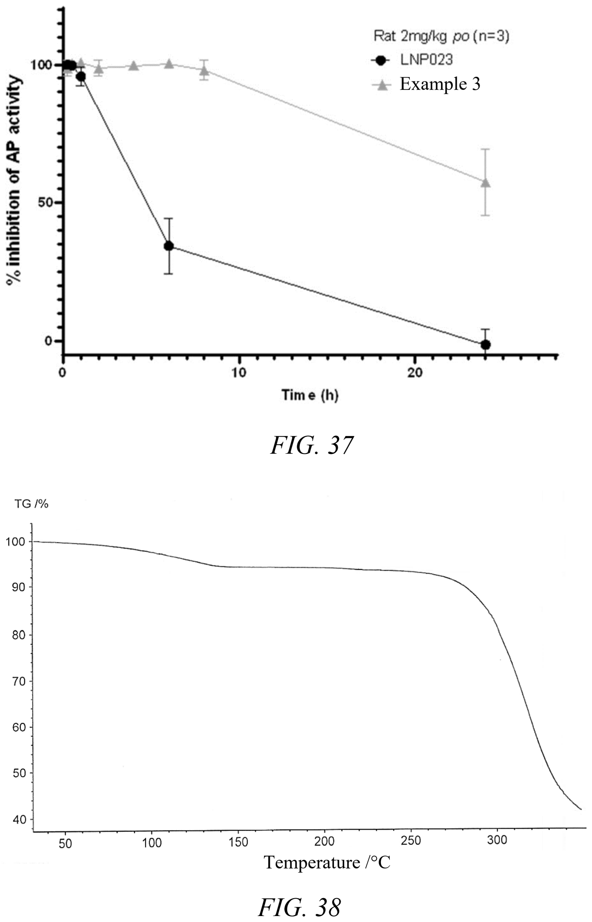

FIG. 37 shows the in vitro assessment of PD inhibition in mouse plasma.
FIG. 38 shows a TGA diagram of crystal form I of hydrochloride.

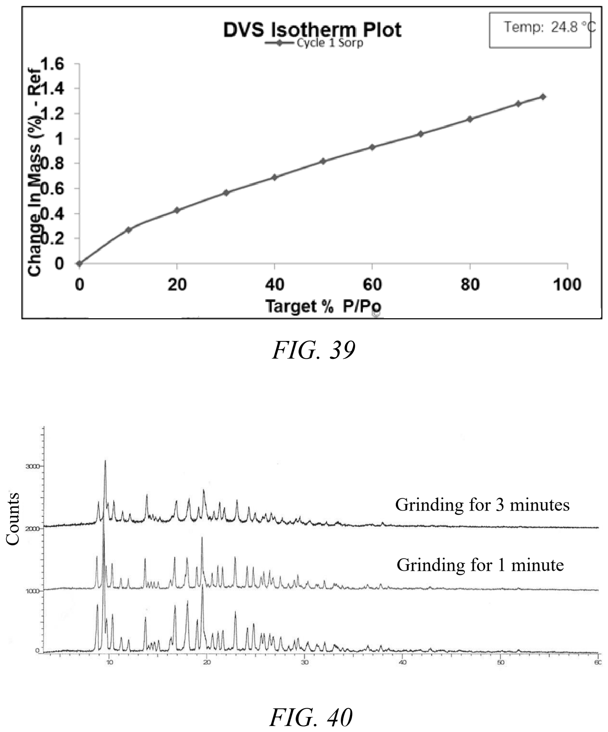

FIG. 39 shows a DVS diagram of crystal form I of hydrochloride.
FIG. 40 shows XRPD diagrams of crystal form I of hydrochloride after grinding for 1 and 3 minutes.

### Detailed Description of Embodiments

Unless stated to the contrary, the terms used in the description and claims have the following meanings.

In the present invention, alkyl refers to a saturated aliphatic hydrocarbon group, which is a straight or branched group containing 1 to 20 carbon atoms, preferably alkyl containing 1 to 8 carbon atoms, more preferably alkyl containing 1 to 6 carbon atoms, and most preferably alkyl containing 1 to 3 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc.

Alkyl may be substituted or unsubstituted, and when the alkyl is substituted, the substituents may be at any available point of attachment. The substituents are preferably one or more groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, oxo, carboxyl or a carboxylate group, and methyl, ethyl, isopropyl, tert-butyl, haloalkyl, deuteroalkyl, alkoxy-substituted alkyl and hydroxyl-substituted alkyl are preferred in the present invention, wherein the hydroxyl-substituted alkyl can be 2-hydroxyisopropyl or 1-hydroxyethyl.

In the present invention, alkoxy refers to -O-(alkyl) and -O-(unsubstituted cycloalkyl), wherein alkyl is as defined above, preferably alkyl containing 1 to 8 carbon atoms, more preferably alkyl containing 1 to 6 carbon atoms, and most preferably alkyl containing 1 to 3 carbon atoms. Non-limiting examples of alkoxy include: methoxy, ethoxy, propoxy, butoxy, cyclopropoxy, cyclobutoxy, cyclopentoxy, and cyclohexoxy. Alkoxy can be optionally substituted or unsubstituted. When the alkoxy is substituted, the substituent is preferably one or more of groups independently selected from alkyl, alkenyl, alkynyl, alkoxy, alkylthio, alkylamino, halogen, mercapto, hydroxyl, nitro, cyano, cycloalkyl, heterocycloalkyl, aryl, heteroaryl, cycloalkoxy, heterocycloalkoxy, cycloalkylthio, heterocycloalkylthio, carboxyl or a carboxylate group.

Non-limiting examples of alkoxy also include: prop-2-oxy, etc.

In the present invention, "haloalkyl" refers to alkyl substituted with one or more halogen, wherein alkyl is defined as above. Non-limiting examples of haloalkyl include: trifluoromethyl and trifluoroethyl.

Non-limiting examples of haloalkyl also include: difluoromethyl, 1,1,2,2-tetrafluoroethyl, perfluoroethyl, etc.

In the present invention, haloalkoxy refers to alkoxy substituted with one or more halogen, wherein alkoxy is defined as above.

The haloalkoxy can be fully halogenated or partially halogenated, and the number of halo can be 1, 2, 3, 4, 5, 6, 7, 8, 9, 10, etc. Halogen is preferably F, Cl, Br, or I. For example, haloalkoxy can be trifluoromethoxy, difluoromethoxy, 1,1,2,2-tetrafluoroethoxy, perfluoroethoxy, etc.

In the present invention, cycloalkyl refers to a saturated or partially unsaturated aliphatic hydrocarbon monocyclic, polycyclic (two or more rings) cyclic group, which may be optionally substituted with one or more substituents. In particular embodiments, the cycloalkyl ring contains 3 to 20 (C₃₋₂₀), 3 to 12 (C₃₋₁₂), 3 to 8 (C₃₋₈), or 3 to 6 (C₃₋₆) carbon atoms. In one embodiment, the cycloalkyl ring contains 6 to 14 (C₆₋₁₄) or 7 to 10 (C₇₋₁₀) carbon atoms; it may contain one or more double bonds, but does not have a completely conjugated π electron system. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and the polycyclic cycloalkyl includes spirocycloalkyl, fused cycloalkyl and bridged cycloalkyl in one embodiment. In one embodiment, the cycloalkyl is optionally substituted cycloalkyl described elsewhere herein or cycloalkyl optionally fused to heterocyclyl, aryl or heteroaryl, and non-limiting examples include indanyl, tetrahydronaphthyl, benzocycloheptyl, etc.

"Hydroxyl" refers to an -OH group.

"Halogen" refers to fluorine, chlorine, bromine, or iodine.

"Amino" refers to -NH₂.

"Cyano" refers to -CN.

"Nitro" refers to -NO₂.

"THF" refers to tetrahydrofuran.

"EtOAc" refers to ethyl acetate.

"DMSO" refers to dimethyl sulfoxide.

"IPA" refers to isopropanol.

"MeOH" refers to methanol.

"EtOH" refers to ethanol.

Different expressions such as "X is selected from A, B, or C", "X is selected from A, B and C", "X is A, B or C", and "X is A, B and C" all express the same meaning, that is, X can be any one or more of A, B, and C.

"Optional" or "optionally" means that the event or circumstance subsequently described may but not necessarily occur, and the description includes the occasion where the event or circumstance occurs or does not occur.

"Substituted" means that one or more hydrogen atoms, preferably at most 5, more preferably 1-3 hydrogen atoms in the group are each independently substituted with a corresponding number of substituents. It goes without saying, the substituents may be only in possible chemical positions thereof, and those skilled in the art can determine the possible or impossible substitutions (by experiment or theory) without paying too much effort. For example, the amino or a hydroxyl having free hydrogen may be unstable when combined the carbon atoms having an unsaturated (e.g., olefinic) bond.

"Stereoisomerism" comprises three types, geometric isomerism (cis-trans isomerism), optical isomerism, and conformational isomerism.

As used herein, the names of compounds are intended to encompass all possible isomeric forms, including stereoisomers (e.g., enantiomers, diastereomers, racemates or racemic mixtures and any mixtures thereof) of the compounds.

The hydrogen atoms according to the present invention can be replaced with the isotope deuterium thereof, and any hydrogen atom in the example compounds involved in the present invention can also be replaced with a deuterium atom.

"Pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

X-ray powder diffraction pattern (XRPD) refers to the experimentally observed diffraction pattern or the parameters derived therefrom. The X-ray powder diffraction pattern is characterized by the peak position (abscissa) and the peak intensity (ordinate). Those skilled in the art will appreciate that the experimental error depends on the conditions of the instrument, the preparation of the sample and the purity of the sample. In particular, it is well known to those skilled in the art that the X-ray diffraction pattern often changes with the conditions of the instrument, and those skilled in the art should understand that the appropriate error tolerance of XRPD can be: 2θ ± 0.5°, 2θ ± 0.4°, 2θ ± 0.3°, or 2θ ± 0.2°. In particular, it should be noted that the relative intensity of the X-ray diffraction pattern may also change with experimental conditions, and thus the order of peak intensity cannot be used as the only or decisive factor. In addition, due to the influence of experimental factors such as sample height, peak angles may shift as a whole, and a certain shift is usually allowed. Therefore, those skilled in the art will appreciate that any crystal form having the characteristic peak identical or similar to those of the pattern of the present invention falls within the scope of the present invention.

"TGA" refers to thermogravimetric analysis (TGA) experiment.

"DSC" refers to differential scanning calorimetry (DSC) experiment.

"HPLC" refers to high performance liquid chromatography (HPLC) experiment.

"PK" refers to pharmacokinetic (PK) experiment.

The present invention will be further described below in conjunction with examples, but these examples are not meant to limit the scope of the present invention.

### Preparation of compounds

The following examples are used to explain the present invention, but these examples should not be construed as limiting the scope of the present invention. If specific conditions of experimental methods are not specifically illustrated in the examples of the present invention, conventional conditions or recommended conditions of raw materials and product manufacturers are generally followed. Reagents without specific source designation are commercially available conventional reagents.

The structure of each compound is identified by nuclear magnetic resonance (NMR) and/or mass spectrometry (MS). NMR chemical shifts (δ) are given in 10⁻⁶ (ppm). NMR is determined by Varian Mercury 300 MHz or Bruker Avance III 400 MHz. The solvents used are deuterated-dimethyl sulfoxide (DMSO-*d₆*), deuterated-chloroform (CDCl₃) and deuterated-methanol (CD₃OD).

High performance liquid chromatography (HPLC) is determined on an Agilent 1200DAD high pressure liquid chromatograph (Sunfire C18 150×4.6 mm chromatographic column) and a Waters 2695-2996 high pressure liquid chromatograph (Gimini C18 150×4.6 mm chromatographic column). Liquid chromatography mass spectrometry (LCMS) is determined on an Agilent 1200 high pressure liquid chromatograph & mass spectrograph (Sunfire C18 4.6*50 mm 3.5 um chromatographic column) and an Agilent 19091S-433 HP-5 high pressure liquid chromatograph & mass spectrograph (XBridge C18 4.6*50 mm 3.5 um chromatographic column).

Chiral high performance liquid chromatography (HPLC) is determined on SFC Thar 80 & 150 & 200 (waters).

The average ATPase inhibition rates and IC₅₀ values are determined by a Victor Nivo multi-mode microplate reader (PerkinElmer, USA).

Yantai Xinnuo silica gel plates are used as the thin-layer silica gel plates in thin-layer chromatography. The dimension of the plates used in TLC is 0.15 mm to 0.2 mm, and the dimension of the plates used in thin-layer chromatography for product purification is 0.4 mm to 0.5 mm.

Column chromatography is typically performed using Qingdao Haiyang silica gel (200-300 mesh) as a carrier.

The known raw materials of the present invention can be prepared by conventional synthesis methods in the prior art, or can be purchased from ABCR GmbH & Co. KG, Acros Organics, Aldrich Chemical Company, Accela ChemBio Inc., Dari chemical Company, etc.

MS denotes mass spectrometry, wherein (+) indicates the positive ionization mode that typically yields an M+1 (or M+H) absorption, and M represents the molecular weight.

### Example 1

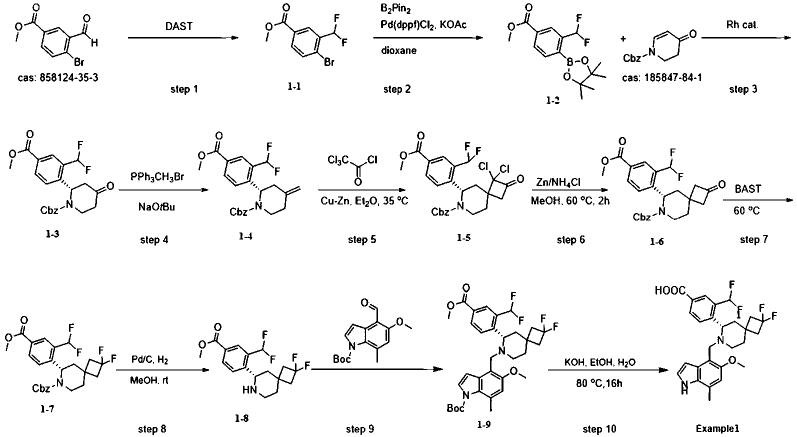

### Step 1: Synthesis of intermediate 1-1

To a solution of methyl 4-bromo-3-formylbenzoate (10.2 g, 42.2 mmol) in dichloroethane (153 ml, 15 V) was added DAST (33.99 g, 210.9 mmol) at -80°C under N₂ atmosphere, and the mixture was heated to room temperature for 4 hours. The reaction mixture was quenched with NH₄Cl solution and concentrated under vacuum to obtain product 1-1 as a yellow oil (7.70 g, yield: 69%). LCMS (m/z): [M+H]⁺ C₉H₈BrF₂O₂ calcd: 265.0; found: 265.0.

### Step 2: Synthesis of intermediate 1-2

At room temperature, under N₂ atmosphere, B₂Pin₂ (10.3 g, 40.7 mmol), Pd(dppf)Cl₂·CH₂Cl₂ (2.38 g, 2.91 mmol) and KOAc (8.28 g, 84.4 mmol) were added to a solution of 1-1 (7.70 g, 29.1 mmol) in dioxane (116 mL, 15 V). The mixture was heated at 90°C for 4 hours and then filtered. The filtrate was concentrated in vacuo, and the residue was purified by flash column chromatography on silica gel (ethyl acetate:petroleum ether = 10/1) to obtain product 1-2 as a yellow oil (5.10 g, yield: 56%). LCMS (m/z): [M+H]⁺ C₁₅H₂₀BF₂O₄ calcd: 313.1; found: 313.0.

### Step 3: Synthesis of intermediate 1-3

In a glovebox, purified water (5 mL, 2 V), Rh(Acac)(C₂H₄)₂ (84 mg, 0.32 mmol) and (*R*, *R*)-Ph-PBE (164 mg, 0.324 mmol) were added to a solution of 43-2 (5.06 g, 16.2 mmol, 1.5 equiv) and benzyl 4-oxo-3,4-dihydropyridine-1(2H)-carboxylate (2.50 g, 10.8 mmol, 1.0 equiv) in tert-amyl alcohol (50 mL, 20 V) at room temperature. The mixture was stirred at 50°C for 12 hours and then filtered. The filtrate was concentrated in vacuo, and the residue was purified by flash column chromatography on silica gel (ethyl acetate : petroleum ether = 4/1) to obtain product 1-3 as a yellow oil (2.08 g, yield: 46%, 100%ee). LCMS (m/z): [M+H]⁺ C₂₂H₂₂F₂NO₅ calcd: 418.15; found: 418.19.
Experimental method: Chromatographic column: CHIRALPAK IA 4.6*250 mm, 5 µm
Mobile phase: 60% hexane, 40% ethanol, 0.1% methanesulphonic acid
Flow rate: 1.0 mL/min
Column temperature: 30°C
Retention time = 7.9 min

### Step 4: Synthesis of intermediate 1-4

To a solution of PPh₃CH₃Br (2.668 g, 7.47 mmol, 1.5 equiv) in toluene (21.6 mL, 20 V) was added NaO*t*Bu (684 mg, 7.12 mmol, 1.43 equiv) at room temperature under N₂ atmosphere using a balloon. The mixture was stirred at room temperature for 2 hours. To a solution of 1-3 (0.080 g 4.94 mmol, 1.0 equiv) in toluene (10.8 mL, 10 V) was added the above mixture at room temperature, and the resultant was stirred for 1 hour. The reaction mixture was quenched with 108 mL (50 V) of saturated NH₄Cl solution, extracted with ethyl acetate (156 mL x 2, 75 V), washed with brine (52 mL, 25 V), dried over Na₂SO₄, and concentrated in vacuo, and the residue was purified by flash column chromatography on silica gel (ethyl acetate:petroleum ether = 1/16 to 1/8) to obtain 1-4 as a colourless oil (1.16 g, yield: 56%). LCMS (m/z): [M+H]⁺ C₂₃H₂₄F₂NO₄ calcd: 416.2; found: 416.3.

### Step 5: Synthesis of intermediate 1-5

To a suspension of Cu-Zn (3.5 g, 3% Cu) and 1-4 (1.0 g, 2.4 mmol, 1.0 equiv) in dioxane (70 mL, 70 V) was added trichloroacetyl chloride (4.4 g, 24 mmol, 10 equiv) over 30 min at 20°C under nitrogen atmosphere. The mixture was heated at 35°C for 3 hours. The reaction mixture was quenched with NH₄Cl solution. The solid was filtered off, and the filtrate was extracted with ethyl acetate (30 mL×3, 30 V), and washed with brine (40 mL, 40 V). The combined filtrates were dried over Na₂SO₄ and concentrated in vacuo to obtain crude product 1-5 as an oil (1.17 g), which was used in the next step without further purification. LCMS (m/z): [M+H]⁺ C₂₅H₂₃Cl₂F₂NO₅ calcd: 526.1; found: 526.3.

### Step 6: Synthesis of intermediate 1-6

To a solution of 1-5 (1.17 g, crude) in MeOH (50 mL) were added NH₄Cl (2.6 g, 48 mmol) and zinc (1.6 g, 24 mmol). The reaction mixture was stirred at 60°C for 2 hours and then filtered. The filtrate was concentrated in vacuo, and the residue was purified by flash column chromatography on silica gel (ethyl acetate:petroleum ether = 4/1) to obtain product 1-6 as an oil (830 mg, two-step yield: 75%). LCMS (m/z): [M+H]⁺ C₂₅H₂₆F₂NO₅ calcd: 458.2; found: 458.3.

### Step 7: Synthesis of intermediate 1-7

1-6 (830 mg, 1.82 mmol) was dissolved in BAST (1.5 mL) at 0°C. The reaction mixture was stirred at 60°C for 12 hours. The reaction was cooled to room temperature, and 15 mL of ethyl acetate was added. The reaction mixture was poured very carefully into ice (10 g). The residue was extracted with ethyl acetate (30 mL x 3), washed with brine (30 mL), and dried over Na₂SO₄. The filtrate was concentrated in vacuo, and the residue was purified by flash column chromatography on silica gel (ethyl acetate : petroleum ether = 4/1) to obtain product 1-7 as an oil (569 mg, yield: 65%). LCMS (m/z): [M+H]⁺ C₂₅H₂₆F₄NO₄ calcd: 480.2; found: 480.1.

### Step 8: Synthesis of intermediate 1-8

To a solution of 1-7 (569 mg, 1.19 mmol) in MeOH (17 mL) was added activated carbon (569 mg). The reaction was heated to reflux for 0.5 hours. The activated carbon was filtered, and Pd/C (57 mg, Pd 10%) was added to the filtrate. The reaction mixture was stirred under a H₂ balloon at room temperature for 2 hours. Pd/C was removed, and the filtrate was concentrated in vacuo to obtain product 1-8 as an oil (328 mg, yield: 80%). LCMS (m/z): [M+H]⁺ C₁₇H₂₀F₄NO₂ calcd: 346.1; found: 346.2.

### Step 9: Synthesis of intermediate 1-9

To a solution of 1-8 (330 mg, 0.95 mmol) in DCE (1.5 mL) were added aldehyde (316 mg, 1.09 mmol) and NaBH(OAc)₃ (632 mg, 2.98 mmol). The mixture was stirred at room temperature for 15 hours. The reaction mixture was quenched with 1.0 mL of water, evaporated to remove the solvent and concentrated to obtain a crude product. The crude product was purified by flash column chromatography on silica gel (ethyl acetate : petroleum ether = 10/1) to obtain product 1-9 as a white solid (270 mg, yield: 46%). LCMS (m/z): [M+H]⁺ C₃₃H₃₉F₄N₂O₅ calcd: 619.3; found: 619.2.

### Step 10: Synthesis of Example 1

To a solution of 1-9 (270 mg, 0.44 mmol) in EtOH (8 mL) was added 30% KOH solution (0.8 mL), and the mixture was stirred at 80°C for 4 hours. The reaction mixture was adjusted to pH = 6 and concentrated in vacuo to obtain 120 mL of a solution. The solution was purified by preparative HPLC. The prepared solution was concentrated to remove CH₃CN, and the residue was extracted with ethyl acetate (20 mL x 5), dried over Na₂SO₄ and filtered. The filtrate was concentrated to dryness to obtain Example 1 as a white solid (32 mg, yield: 15%). LCMS (m/z): [M+H]⁺ C₂₇H₂₉F₄N₂O₃ calcd: 505.21; found: 505.29; Proton nuclear magnetic resonance spectrum (400 MHz, CD₃OD): δ 8.34 (d, *J* = 7.8 Hz, 1H), 8.26 (s, 1H), 7.95 (d, *J* = 7.6 Hz, 1H), 7.33 (d, *J* = 3.0 Hz, 1H), 7.17 (br, 1H), 7.04 (br, 1H), 6.77 (s, 1H), 6.46 (d, *J* = 3.2 Hz, 1H), 4.56 (s, 1H), 4.19 (d, *J* = 12.0 Hz, 1H), 4.05-3.87 (m, 1H), 3.79 (s, 3H), 3.50-3.39 (m, 1H), 3.27-3.05 (m, 1H), 2.80-2.55 (m, 2H), 2.52 (s, 3H), 2.42 (t, *J* =12.4 Hz, 2H), 2.25-1.95 (m, 3H), 1.92-1.80 (m, 1H).

### Example 2

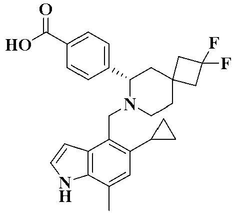

### Example 2

Example 2 was prepared with reference to the method in Example 1;
LCMS: m/z = 465.1 (M+1, ESI+);
¹H NMR (400 MHz, MeOD) δ 8.31 (brs, 1H), 8.07 (d, *J* = 8.2 Hz, 2H), 7.62 (d, *J= 8.0* Hz, 2H), 7.24 (d, *J* = 3.1 Hz, 1H), 6.59 (s, 1H), 6.40 (brs, 1H), 4.40 (d, *J* = 12.1 Hz, 1H), 4.15 - 4.03 (m, 2H), 3.33 - 3.27 (m, 1H), 3.04 - 2.91 (m, 1H), 2.72 - 2.53 (m, 2H), 2.43 - 2.33 (m, 4H), 2.27 - 2.17 (m, 1H), 2.08 - 1.66 (m, 5H), 0.78 - 0.72 (m, 2H), 0.25 (brs, 1H), -0.00 (brs, 1H).

### Example 3

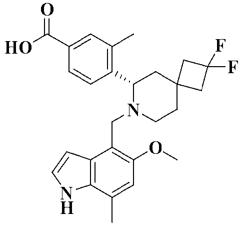

### Example 3

Example 3 was prepared with reference to the method in Example 1;
LCMS: m/z = 469.30 (M+1, ESI+);
¹H NMR (400 MHz, CD₃OD): δ 8.05 (d, *J* = 6.9 Hz, 1H), 7.94 (s, 1H), 7.69 (d, *J* = 8.1 Hz, 1H), 7.33 (d, *J* = 3.1 Hz, 1H), 6.78 (s, 1H), 6.40 (d, *J* = 3.1 Hz, 1H), 4.55 (d, *J* = 11.3 Hz, 1H), 4.30 (d, *J* =12.4 Hz, 1H), 4.15-4.05 (m, 1H), 3.79 (s, 3H), 3.54-3.42 (m, 1H), 3.32-3.24 (m, 1H), 2.80-2.65 (m, 2H), 2.56 (s, 3H), 2.52 (s, 3H), 2.50-2.40 (m, 2H), 2.26-2.12 (m, 1H), 2.11-2.00 (m, 2H), 1.96-1.85 (m, 1H).

### Biological assay and evaluation

The present invention will be further described and explained below in conjunction with test examples, but these examples are not meant to limit the scope of the present invention.

### Biological experiment 1: TR-FRET factor B binding assay

### Materials and related reagents

1. Recombinant human factor B catalytic domain (a.a. 470-764, C-terminal His-tag, produced in-house)
2. 5X kinase buffer A (Thermo Fisher, CAT#PV3189)
3. LANCE Eu-W1024 anti-6xHis antibody (PerkinElmer, CAT#AD0401)
4. Probe (TRFRET_tool 2, described in WO 2015/00916) 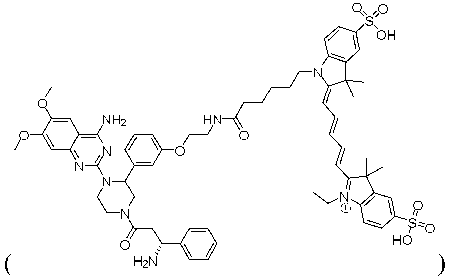
5. Dimethyl sulfoxide (Thermo Fisher Scientific)
6. Compound - 10 mM stock in DMSO
7. Victor Nivo multi-mode microplate reader (PerkinElmer)
8. OptiPlate-384, white opaque 384-well microplate (PerkinElmer, CAT# 6007290)

### Experimental methods:

The binding affinity of each test compound to factor B was determined using the time-resolved fluorescence resonance energy transfer (TR-FRET) technology. 10 nM recombinant his-tagged factor B catalytic domain, different concentrations of inhibitors, 4 nM LANCE Eu-W1024 anti-6xHis antibody and 100 nM TR-FRET_tool2 tracer were incubated in 1X kinase buffer A for 1 hour. Measurements were performed with a reaction volume of 15 µL by adding 5 µL of the test compound, followed by the addition of 5 µL of a factor B/antibody mixture and 5 µL of the tracer to a white opaque 384-well assay plate. TR-FRET signals were read on a plate reader with an excitation wavelength of 340 nm and detection wavelengths of 615 and 665 nm. The binding affinity of each compound was determined by measuring TR-FRET signals at different concentrations of the compound, plotting the relationship between the relative fluorescence emission ratio (665 nm/615 nm) and inhibitor concentrations, and estimating the IC₅₀ based on [compound] and emission ratio using a four-parameter dose-response inhibition curve with a variable slope model in GraphPad Prism.

The binding affinity of the compounds of the present invention to the catalytic domain of recombinant factor B was determined by the above assay. The IC₅₀ values (nM) are shown in Table 1 below.

**Table 1**

| Example | Factor B TR-FRET IC₅₀ (nM) |
|---|---|
| 1 | <2 |
| 2 | <2 |
| 3 | <2 |

### Biological example 2: Determination of target residence time of factor B inhibitors by surface plasmon resonance (SPR)

### Materials and related reagents

1. Recombinant human factor B catalytic domain (a.a. 470-764, C-terminal his-tagged, produced in-house)
2. PBS-P+buffer 10X (Cytiva, CAT. No. 28995084)
3. Series S Sensor Chip NTA (Cytiva, CAT# BR100532)
4. Amine coupling kit (Cytiva, CAT#BR100050)
5. Dimethyl sulfoxide (Millipore Sigma, CAT #34869-1L)
6. Greiner 96-well polypropylene plates (Sigma Aldrich, CAT# M7310-100EA)
7. Microplate foil, 96-well (Cytiva, CAT # 28975816)
8. Biacore 8k (Cytiva)

### Experimental methods:

Before docking the Cytiva NTA chip, the Biacore 8k instrument was pretreated with 1X PBS-P + buffer. The recombinant human factor B catalytic domain was immobilized on the NTA chip using 1X PBS-P + buffer [20 mM phosphate buffer, 2.7 mM KCl, 137 mM NaCl, and 0.05% (v/v) Tween-20] to achieve approximately 5000 resonance units (RU). The protein ligand was further crosslinked to the sensor chip surface via an amine coupling kit. Immobilization and binding experiments were performed at room temperature.

After buffer exchanging to 1X PBS-P + buffer containing 2% (v/v) dimethyl sulfoxide, a pre-run was performed at a flow rate of 30 µl/min for at least 30 minutes to obtain a stable surface. The kinetic constants of the compounds were determined by single-cycle kinetics of six consecutive injections (or multi-cycle kinetics of eight consecutive injections), with compound concentrations ranging from 0.8-200 nM, 12.5-400 nM, 4.1-1000 nM, or 41-10,000 nM depending on compound potency. Single-cycle kinetic experiments were performed with an association time of 60 s and a dissociation time of 300 s per concentration (or multi-cycle kinetic experiments were performed with a dissociation time of 120 s). The flow rate was 30 µl/min. Before the injection of the compound, a blank test was performed under the same conditions.

The SPR sensorgrams were analysed using Biacore Insight Evaluation software with the double-referencing method. The resulting curves were fitted to a 1 : 1 binding model. Compounds that exhibited binding according to the induced fit model were fitted using a two-state reaction model. The kinetic constants (*k*ₒₙ*, k*_{off} and *K*_{D}) obtained from replicate measurements were averaged. The binding half-life (t1/2) for the 1 : 1 binding model and the two-state reaction model was calculated from the dissociation constant koff, using the formula t_{1/2} = ln2/*k*_{off}. The target residence time (t_{1/2}) and residence time (1/*k*_{off}) are shown in Table 2 below.

**Table 2: Kinetic constants and target residence time of compounds of the present invention against human factor B**

| Example | *K*_{D} (nM) | Residence time (s) | t_{1/2} (s) |
|---|---|---|---|
| Iptacopan | 9.93 | 100.1 | 69 |
| 1 | 0.77 | 4628.8 | 3208 |
| 2 | 0.38 | 6308.4 | 4372 |
| 3 | 0.17 | 6171.1 | 4277 |

| | | | |
|---|---|---|---|
| Conclusion: the example compounds of the present invention have remarkable binding affinities. | | | |

### Biological example 3: Pharmacokinetic study in rat eyes

Three-month-old brown Norway rats were orally gavaged with the example compound as a suspension in 2 equivalents of 1 N HCl + 30% PEG300 + 50% (20% Cremophor EL aqueous solution). At 0.25, 0.5, 1, 6, and 24 hours post-dosing, ocular tissues and plasma were collected from both eyes of the rats at respective time points. The ocular tissues collected comprised retina and the posterior eyecup (RPE/choroid and posterior sclera). Tissues were diluted with phosphate-buffered saline containing 10% acetonitrile, followed by homogenization and centrifugation prior to analysis. At each time point, test article concentrations in plasma and eye homogenate supernatants were measured in four individual retinal samples, four individual posterior eyecup samples and two individual plasma samples by using HPLC-MS/MS. Chromatographic separation was performed on a Waters BEH C18 column (2.1 × 50 mm, 1.7 µm) (MAC-MOD Analytical, Chadds-Ford, PA) with a gradient elution method using water and acetonitrile, both containing 0.025% formic acid-1 M NH4OAc. Mass spectrometry measurements in positive electrospray ionization were performed to quantify mass transitions using [M+H]+ as the precursor ion on an API6500 triple quadrupole mass spectrograph (Sciex, Framingham, MA). The relevant pharmacokinetic parameters were estimated using the non-compartmental method in WinNonlin (Enterprise, version 8.2). The rat PK study results are shown in Table 3 below.

**Table 3: Rat PK study results (2 mpk PO)**

| | T1/2 (h) | Retina AUC_{*0*-*last*} (nM·h) | Plasma AUC*₀₋ₗₐₛₜ* (nM·h) |
|---|---|---|---|
| Iptacopan | 5.3 | 141 | 13461 |
| Example 3 | 22.2 | 796.4 | 12,668 |

| | | | |
|---|---|---|---|
| Conclusion: the compounds of the present invention have better exposure in the retina. | | | |

### Biological example 4: In vivo assessment of AP complement functional activity in mice

20 hours before the end of the study, female C57BL/6 mice were orally gavaged with the formulation of compound of Example 3 (20 mg/kg, 0.5% (w/v) methylcellulose, 0.5% (v/v) Tween 80). To activate the complement pathway, lipopolysaccharide (LPS) from Salmonella typhimurium (Sigma) was injected intraperitoneally (2.5 mg/kg) 7.5 hours before the end of the study. Control mice received intraperitoneal injections of normal saline and oral gavage administration of vehicles. At the end of the study, plasma samples were collected from the mice. AP complement activation was assessed by measuring plasma C3 cleavage products, C3b/iC3b/C3c, using an ELISA method with rat anti-mouse C3b/iC3b/C3c monoclonal antibodies (clone 2/11, Hycult Biotech, 0.1 µg/well), and a goat anti-rat IgG (whole molecule)-peroxidase (Sigma) diluted in TBST (TBS/0.05% Tween 20). Plasma C3b/iC3b/C3c are shown in Table 4 below.

**Table 4: Plasma C3b/iC3b/C3c following treatment**

| Treatment | Plasma C3b/iC3b/C3c (1 x 10⁶) |
|---|---|
| None | 168.00 |
| Normal saline | 157.75 |
| LPS | 370.50 **** |
| Example 3 | 210.25 ^{ns} |

| | |
|---|---|
| Conclusion: the results showed that the compound of Example 3 exhibited sustained inhibitory effects in the PD assay in mice over 20 hours (*:p < 0.05; **:p < 0.01; ***:p < 0.001; ns: not significant) | |

### Biological example 5: In vitro assessment of PD inhibition in rat plasma

Male Sprague-Dawley rats (n = 3) were orally administered with vehicles (0.5% (w/v) methylcellulose, 0.5% (v/v) Tween 80), compound Iptacopan, or example compound formulations (in 0.5% (w/v) methylcellulose, 0.5% (v/v) Tween 80) at a dose of 2 mg/kg. Rat serum samples were collected at 0.25, 0.5, 1, 2, 4, 6, 8, and 24 hours post-dosing, and stored at -80°C. 96-well microtitre plates (Black Maxisorp, Invitrogen) were coated with 3 µg/ml LPS from Salmonella enterica strains and incubated overnight at 4°C for alternative complement pathway (AP) ELISA (TLRGRADE, Enzo Life Sciences, in PBS/10 mM MgCl2). The coated plates were washed with GVB buffer (Complement technology) containing 5 mM MgCl2 and 10 mM EGTA (blocking both classical and lectin pathways). The collected serum samples were diluted by adding an equal volume of GVB buffer containing 10 mM MgCl2 and 20 mM EGTA. For negative controls, serum was diluted with GVB buffer containing 40 mM EDTA (blocking all complement pathways). Aliquots (50 ul) of 50% serum samples were added to the LPS-coated wells. The reaction plate was incubated at 37°C for 20 minutes (rat serum). The plate was inverted to empty the wells, and blocking buffer (50 µL, SuperBlock^{™} T20 (TBS) blocking buffer, Thermo #37536) was added. Rat MAC deposition on LPS was detected by using anti-rat C5b-9 neoepitope detection mAb 2Al (HM3033-IA, Hycult Biotech, 0.1 µg/well) and goat anti-mouse IgG (Fc-specific)-peroxidase (Sigma, #A2554). Baseline (EDTA-treated serum) and maximum signal (EGTA-treated serum from vehicle-treated rats) were used to generate a percent inhibition value for each well.

Rats (3 rats/group) were orally administered with compound Iptacopan or Example 3 (2 mg/kg), and then the AP deposition inhibitory activity in 50% compound serum was evaluated 0.25, 0.5, 1, 2, 4, 6, 8, and 24 hours post-dosing. Each data point in FIG. 37 represents the mean AP activity in rat serum. The data results showed that the compound of Example 3 exhibited sustained inhibitory effects in the ex vivo PD assay in rats over 24 hours.

**Table 5: Rat 24-hour PD study results**

| | Serum PD inhibition (%) over 24 hours (2 mpk) |
|---|---|
| Iptacopan | -3 |
| Example 3 | 57.5 |

### Salt and crystal form research

### 1.1 Experimental instruments

### 1.1.1 Parameters of physicochemical testing instruments

| | | |
|---|---|---|
| XRPD | Instrument model | BRUKER D8 ADVANCE |
| | Diffraction line | Cu/K-Alphal (λ= 1.5418Å) |
| | Scan rate | 0.02°/S (2θ value) |
| | Scan range | 4° - 40°(2θ value) |
| DSC | Instrument model | NETZSCH DSC 214 polyma |
| | Purge gas | Nitrogen |
| | Purge rate | 40 mL/min |
| | Heating rate | 10°C/min |
| | Temperature range | 25°C - 300°C |
| | Pan type | Aluminium pan |
| TGA | Instrument model | NETZSCH TG 209 Tarsus |
| | Purge gas | Nitrogen |
| | Purge rate | 40 mL/min |
| | Heating rate | 10°C /min |
| | Temperature range | 35°C -350°C |
| | Pan type | Al₂O₃ |

### 1.2 Instrument and liquid phase analysis conditions

### 1.2.1 Instruments and equipment

| Instrument name | Model | |
|---|---|---|
| Analytical balance | Sartorius BSA224S-CW | |
| Water purifier | Milli-Q Plus, Millipore | |
| High performance liquid chromatograph | Thermo | U3000 |
| | Pump | LPG-3400SDN |
| | Sample injector | WPS-3000TSL |
| | Column oven | TCC-3000SD |
| | Detector | DAD-3000 |

### 1.2.2 Chromatographic conditions

Chromatographic column: Aglient Bonus, 3.5 µm, 4.6*150 mm
Flow rate: 1 mL/min
Column temperature 40°C
Detection wavelength: 254 nm
Injection volume: 5.0 µL
Run time: 15 min
Diluent: methanol-water (50 : 50, v : v)
Mobile phase: A: water (0.1% phosphoric acid); B: acetonitrile

| T (min) | A (%) | B (%) |
|---|---|---|
| 0.00 | 90 | 10 |
| 5.00 | 50 | 50 |
| 10.00 | 50 | 50 |
| 15.00 | 90 | 10 |

### 2. Screening of salt form and crystal form of compound

### 2.1 Experimental objective

Different counterions were selected, and salts of the compounds were formed via appropriate crystallisation methods.

### 2.2 Experimental steps

### 2.2.1 Instruments and equipment

| Name | Model | Source |
|---|---|---|
| Analytical balance | BSA224S-CW | Sartorius |
| Ultrasonic cleaner | SK5200LHC | Shanghai Kudos Ultrasonic Instrument |
| Pipette | Eppendorf (100 µL, 1000 µL) | Eppendorf |

### 2.2.2 Operation procedures and results

### 1) Natural evaporation method using methanol, tetrahydrofuran, acetone, ethyl acetate or toluene

Salt form screening experiments were conducted via the solvent evaporation method. About 10 mg of the free base of Example 3 was weighed into a 2 mL glass vial and dissolved in 100 µL of an organic solvent. A solution of the counterion acid in methanol was added dropwise at a molar ratio of 1:1. The resulting clear solution was allowed to evaporate at room temperature. The obtained solid was first characterised by PLM. If no birefringence was observed, the solids were slurried by adding a solvent. If birefringence was observed, the solids were further characterised by XRPD. Compounds with high crystallinity were selected for characterization by DSC and TGA. The phenomena observed during evaporation and the characterization results are shown in the table below:

| **Solvent** | **Counterion solution (µL)** | **Phenomenon** | **PLM/XRPD or slurry solvent** | **Post-slurry PLM/XRPD** |
|---|---|---|---|---|
| Methanol | Hydrobromic acid (22) | Clear → off-white solid | √, high crystallinity | // |
| Methanol | Hydrochloric acid (22) | Clear → off-white solid | ×, 2-methyltetrahydrofuran | √, crystal form B of hydrochloride |
| Acetone | Hydrobromic acid (22) | Clear → off-white solid | ×, ethyl acetate | √, crystal form A of hydrobromide |
| Acetone | Hydrochloric acid (22) | Clear → off-white solid | ×, ethyl acetate | √, high crystallinity, crystal form A of hydrochloride |
| Acetone | Methanesulphon ic acid (22) | Clear → off-white solid | ×, ethyl acetate | √, crystal form A of methanesulfonat e |
| Acetone | p-Toluenesulphoni c acid (22) | Clear → off-white solid | ×, ethyl acetate | √, crystal form A of p-toluenesulfonate |
| Methanol | Adipic acid (22) | Clear → off-white solid | √, crystal form A of adipate | // |
| Methanol | Fumaric acid (88) | Clear → off-white solid | √, crystal form A of fumarate | // |
| Methanol | Hippuric acid (55) | Clear → off-white solid | √, crystal form A of hippurate | // |
| Methanol | Succinic acid (22) | Clear → off-white solid | √, crystal form A of succinate | // |
| Methanol | Malonic acid (22) | Clear → off-white solid | √, crystal form A of malonate | // |
| Methanol | Oxalic acid (22) | Clear → off-white solid | √, crystal form A of oxalate | // |
| Ethyl acetate | Hydrobromic acid (22) | Clear → Suspension (slurried) | √, crystal form A of hydrobromide | // |
| Toluene | Hydrobromic acid (22) | Clear → Suspension (slurried) | √, crystal form A of hydrobromide | // |

| | | | | |
|---|---|---|---|---|
| Notes: "√" indicates the presence of birefringence phenomenon; "×" indicates the absence of birefringence phenomenon; and "//" indicates no further characterization. | | | | |

### 2) Salt formation by reactive crystallisation method

Approximately 10 mg of the free base compound of Example 3 was weighed into a 2 mL glass vial and dissolved in 100 µL of an organic solvent. 22 µL of a solution of the acid in ethyl acetate was slowly added dropwise at a molar ratio of 1 : 1 under stirring. The resulting clear solution was stirred at room temperature for approximately 24 hours. The resulting solid was dried in vacuo at 40°C and characterised by XRPD, DSC and TGA. The phenomena observed during the process and the characterization results are shown in the table below.

| **Solvent** | **Acid** | **Phenomenon** | **Characterization result** |
|---|---|---|---|
| Ethyl acetate | Hydrochloric acid | Clear → Suspension | Crystal form A of hydrochloride |
| Ethyl acetate | Methanesulphon ic acid | Clear → Suspension | Crystal form A of methanesulfonate |
| Ethyl acetate | Hydrobromic acid | Clear → Suspension | Crystal form A of hydrobromide |

Nine crystalline salts, namely hydrobromide, hydrochloride, methanesulfonate, p-toluenesulfonate, fumarate, adipate, oxalate, hippurate and malonate, were obtained through salt form screening via the evaporation method and reactive crystallisation method.

### 3) Preparation of crystal form A of phosphate

Approximately 10 mg of the free base compound of Example 3 was weighed, and 200 µL of ethyl acetate was added. A solution of phosphoric acid in methanol was slowly added dropwise at 1 : 1, with the gradual precipitation of an off-white solid. The mixture was stirred at room temperature for 1 day, and then centrifugated to collect a product. The product was dried in vacuo at 40°C to obtain the crystal form A of the phosphate.

### 4) Screening of stable crystal form of hydrochloride

Approximately 20 mg of the hydrochloride (crystal form A) of the free base compound of Example 3 was weighed and slurried by adding 100 µL of the solvent at room temperature for 2 weeks. The sample was then centrifuged, and the resulting solid was dried in vacuo overnight at 40°C. The dried solid was characterized by XRPD. If a polymorphic change was observed, further characterization by DSC and TGA was performed. The phenomena and characterization results are as follows:

| **Solvent** | **Phenomenon** | **Characterization result** |
|---|---|---|
| Acetone-water (88 : 12, v:v) | Off-white solid | Crystal form A of hydrochloride |
| Acetone | Off-white solid | Crystal form C of hydrochloride |
| Ethanol | Off-white solid | Crystal form D of hydrochloride |
| Isopropanol | Off-white solid | Crystal form E of hydrochloride |
| Dichloromethane | Off-white solid | Crystal form F of hydrochloride |
| Isopropyl acetate | Off-white solid | Crystal form A of hydrochloride |
| Toluene | Off-white solid | Crystal form A of hydrochloride |
| Methyl tert-butyl ether | Off-white solid | Crystal form A of hydrochloride |
| acetonitrile | Off-white solid | Crystal form A of hydrochloride |
| Ethyl acetate | Off-white solid | Crystal form A of hydrochloride |
| 1,4-Dioxane | Light yellow solid | Crystal form A of hydrochloride |
| 2-Methyltetrahydrofuran | Off-white solid | Crystal form A of hydrochloride |
| Tetrahydrofuran | Light yellow solid | Crystal form G of hydrochloride |
| 2-Butanone | Off-white solid | Crystal form G of hydrochloride |
| Water | Off-white solid | Crystal form A of hydrochloride |

In the experiments for screening stable crystal forms of hydrochlorides, crystal form C of hydrochloride, crystal form D of hydrochloride, crystal form E of hydrochloride, crystal form F of hydrochloride and crystal form G of hydrochloride were prepared.

### 5) Preparation of crystal form H of hydrochloride

52.06 mg of Example 3 was weighed into a 4 mL glass vial, and 400 µL of 2-Me-THF was added. The mixture was magnetically stirred until complete dissolution was achieved. A H₂O-HCl system (6 M) was slowly added dropwise at a salt formation ratio of 1 : 1.3 to precipitate an off-white solid, and the crystal form H of hydrochloride was thus obtained.

### 6) Preparation of crystal form I of hydrochloride

Approximately 20 mg of Example 3 was weighed and dissolved in 137 µL of ethyl acetate. 40 µL of acetone-water (88 : 12, v : v) was added to obtain a clear solution. The solution was magnetically stirred at room temperature, and 1 M EA-HCl solution was slowly added dropwise at a salt formation ratio of 1 : 1, with the gradual precipitation of an off-white solid. After stirring for 3 h, the solid was collected by centrifugation and dried in vacuo at 40°C. The resulting solid was characterized by XRPD, and the crystal form I of hydrochloride was thus obtained.

### 7) Preparation of crystal form J of hydrochloride

Approximately 20 mg of Example 3 was weighed and dissolved in 137 µL of ethyl acetate. 40 µL of water was added to obtain a clear solution. The solution was magnetically stirred at room temperature, and 1 M EA-HCl solution was slowly added dropwise at a salt formation ratio of 1 : 1, with the gradual precipitation of an off-white solid. After stirring for 3 h, the solid was collected by centrifugation and dried in vacuo at 40°C. The resulting solid was characterized by XRPD, and the crystal form J of hydrochloride was thus obtained.

### 3. Hygroscopicity experiment

### 3.1 Experimental objective

The hygroscopicity of different salts of the compound was investigated under various relative humidity conditions to provide a basis for screening and storage of the salts of the compound.

### 3.2 Experimental scheme

The salts of the compound were placed in saturated water vapor at different relative humidities to reach a dynamic equilibrium between the compound and the water vapor. The percentage weight gain of the compound due to moisture absorption was calculated after the equilibrium.

### 3.3 Experimental result

### 3.3.1 Hygroscopicity of crystal form of hydrochloride of compound of Example 3

Hygroscopicity of crystal form A of hydrochloride: Under dynamic hygroscopic conditions, the crystal form A of hydrochloride of the compound of example 3 exhibited slight hygroscopicity. No change was observed in the crystal form after the hygroscopicity determination.

### 4. Solid stability experiment (screening)

### 4.1 Grinding experiment

The crystal form I of hydrochloride was ground in a mortar for 1 minute and 3 minutes. No change was observed in the crystal form, indicating that the crystal form I had good stability under the grinding conditions. The XRPD characterization of crystal form I after grinding is shown in FIG. 40.

### 5. Stability experiment

The sample of crystal form I of hydrochloride was placed under high temperature of 60°C and 40°C, high humidity of 92.5% and 75%, and light conditions for 30 days. No change was observed in the crystal form, and no increase in related substances was detected, indicating that the crystal form I had good stability and was suitable for pharmaceutical development.

| Condition | Time point | HPLC | XRD |
|---|---|---|---|
| High temperature 60°C | 0 days | 99.80% | Crystal form I of hydrochloride |
| | 30 days | 99.79% | Crystal form I of hydrochloride |
| High humidity 92.5% | 0 days | 99.80% | Crystal form I of hydrochloride |
| | 30 days | 99.84% | Crystal form I of hydrochloride |
| Illumination (5000 LUX) | 0 days | 99.80% | Crystal form I of hydrochloride |
| | 30 days | 99.57% | Crystal form I of hydrochloride |

## Claims

1. An acid salt of a compound as shown in general formula (I), wherein the compound has a structure as shown below: 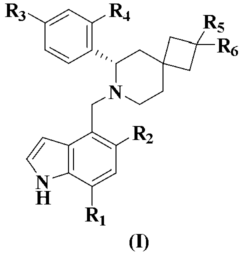 **characterised in that**
R₁ is selected from C₁-C₃ alkyl or C₃-C₆ cycloalkyl;
R₂ is selected from C₁-C₃ alkyl, C₁-C₃ alkoxy or C₃-C₆ cycloalkyl;
R₃ is selected from -COOH, -C(O)NHSO₂CH₃ or -S(O)NHCH₃;
R₄ is selected from hydrogen, halogen, C₁-C₃ alkyl or C₁-C₃ haloalkyl;
R₅ and R₆ are each independently selected from halogen;
provided that the compound as shown in general formula (I) is not 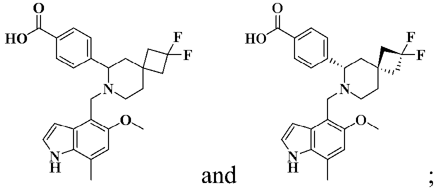
the acid is an inorganic acid or an organic acid; the inorganic acid is selected from hydrochloric acid, sulphuric acid, nitric acid, hydrobromic acid, hydrofluoric acid, hydroiodic acid or phosphoric acid; the organic acid is selected from 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, embonic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, or L-malic acid.

2. The acid salt of the compound according to claim 1, **characterised in that**
R₁ is C₁-C₃ alkyl;
R₂ is selected from C₁-C₃ alkoxy or cyclopropyl;
R₃ is -COOH;
R₄ is selected from hydrogen, C₁-C₃ alkyl or C₁-C₃ haloalkyl;
R₅ and R₆ are each independently selected from hydrogen or halogen.

3. The acid salt of the compound according to claim 1, **characterised in that** the general formula (I) is selected from the following compounds: 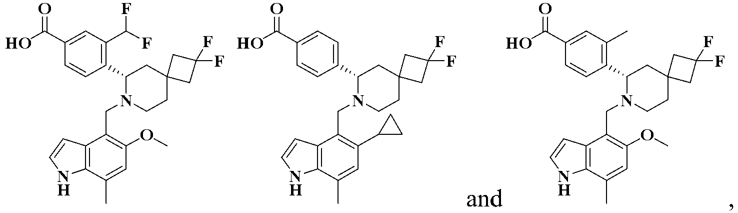 wherein the inorganic acid is selected from hydrochloric acid, sulphuric acid, phosphoric acid or hydrobromic acid, and the organic acid is selected from 1,5-naphthalenedisulphonic acid, methanesulphonic acid, ethanesulphonic acid, p-toluenesulphonic acid, hydroxyethylsulphonic acid, acetic acid, maleic acid, fumaric acid, oxalic acid, malonic acid, tartaric acid, malic acid, adipic acid, hippuric acid, succinic acid or camphoric acid; preferably, the acid is selected from p-toluenesulphonic acid, methanesulphonic acid or hydrochloric acid; more preferably, the acid is hydrochloric acid.

4. The acid salt of the compound according to claim 2, **characterised in that** the compound is (S)-4-(2,2-difluoro-7-((5-methoxy-7-methyl-1H-indol-4-yl)methyl)-7-azaspiro[3.5]nonan-6-yl)-3-methylbenzoic acid, wherein the inorganic acid is selected from hydrochloric acid, sulphuric acid, phosphoric acid or hydrobromic acid, and the organic acid is selected from 1,5-naphthalenedisulphonic acid, methanesulphonic acid, ethanesulphonic acid, p-toluenesulphonic acid, hydroxyethylsulphonic acid, acetic acid, maleic acid, fumaric acid, oxalic acid, malonic acid, tartaric acid, malic acid, adipic acid, hippuric acid, succinic acid or camphoric acid; preferably, the acid is selected from p-toluenesulphonic acid, methanesulphonic acid or hydrochloric acid; more preferably, the acid is hydrochloric acid.

5. The acid salt of the compound according to any one of claims 1 to 4, **characterised in that** the number of acid molecules is 0.2-3, preferably 0.2, 0.5, 1, 1.5, 1.2, 2, 2.5 or 3, more preferably 0.5, 1, 2 or 3, and further preferably 1.

6. The acid salt of the compound according to any one of claims 1 to 5, **characterised in that** the acid salt is a hydrate or an anhydrate; and when the acid salt is a hydrate, the number of water molecules is 0.2 to 3, preferably 0.2, 0.5, 1, 1.5, 2, 2.5 or 3, and more preferably 0.5, 1, 2 or 3.

7. The acid salt of the compound according to claim 4, **characterised in that** the acid salt of (S)-4-(2,2-difluoro-7-((5-methoxy-7-methyl-1H-indol-4-yl)methyl)-7-azaspiro[3.5]nonan-6-yl)-3-methylbenzoic acid is in a crystal form.

8. The acid salt of the compound according to claim 7, **characterised in that** the crystal form of the acid salt is selected from crystal form A of hydrobromide, crystal forms A to J of hydrochloride, crystal form A of a methanesulfonate, crystal form A of a p-toluenesulfonate, crystal form A of a hippurate, crystal form A of a malonate, crystal form A of an oxalate, crystal form A of an adipate, crystal form A of a succinate, crystal form A of a phosphate, or crystal form A of a fumarate;
the crystal form A of the hydrobromide has an X-ray powder diffraction pattern comprising a diffraction peak at one or more of 2θ (±0.2°) of 8.8, 17.5, 15.3, 22.8, 26.4, 22.0, 25.4, 31.0 and 35.5, preferably comprising a characteristic peak at any 2, 4, 6 and 8 of the above-mentioned 2θ;
the crystal form A of the hydrochloride has an X-ray powder diffraction pattern comprising a diffraction peak at one or more of 2θ (±0.2°) of 9.2, 15.7, 11.4, 16.8, 20.0, 13.7, 19.6, 20.3 and 21.7, preferably comprising a characteristic peak at any 2, 4, 6 and 8 of the above-mentioned 2θ;
the crystal form B of the hydrochloride has an X-ray powder diffraction pattern comprising a diffraction peak at one or more of 2θ (±0.2°) of 7.2, 15.9, 7.8, 14.0, 20.0, 10.5, 17.7 and 21.5, preferably comprising a characteristic peak at any 2, 4, 6 and 8 of the above-mentioned 2θ;
the crystal form C of the hydrochloride has an X-ray powder diffraction pattern comprising a diffraction peak at one or more of 2θ (±0.2°) of 9.2, 16.0, 14.8, 15.2, 8.8 and 18.4, preferably comprising a characteristic peak at any 2, 4 and 6 of the above-mentioned 2θ;
the crystal form D of the hydrochloride has an X-ray powder diffraction pattern comprising a diffraction peak at one or more of 2θ (±0.2°) of 23.3, 16.1, 13.1, 8.2, 15.9, 24.7 and 27.1, preferably comprising a characteristic peak at any 2, 4, 6 and 8 of the above-mentioned 2θ;
the crystal form E of the hydrochloride has an X-ray powder diffraction pattern comprising a diffraction peak at one or more of 2θ (±0.2°) of 23.0, 15.3, 9.8, 11.2, 12.7, 17.4, 20.6 and 25.0, preferably comprising a characteristic peak at any 2, 4, 6 and 8 of the above-mentioned 2θ;
the crystal form F of the hydrochloride has an X-ray powder diffraction pattern comprising a diffraction peak at one or more of 2θ (±0.2°) of 9.3, 19.4, 8.4, 10.3, 13.2, 17.5 and 18.9, preferably comprising a characteristic peak at any 2, 4 and 6 of the above-mentioned 2θ;
the crystal form G of the hydrochloride has an X-ray powder diffraction pattern comprising a diffraction peak at one or more of 2θ (±0.2°) of 8.0, 9.9, 13.0, 13.9, 16.0, 16.6 and 22.9, preferably comprising a characteristic peak at any 2 and 4 of the above-mentioned 2θ;
the crystal form H of the hydrochloride has an X-ray powder diffraction pattern comprising a diffraction peak at one or more of 2θ (±0.2°) of 6.8, 10.0, 11.4, 13.6, 20.0, 21.5, 26.0 and 31.9, preferably comprising a characteristic peak at any 2, 4 and 6 of the above-mentioned 2θ;
the crystal form I of the hydrochloride has an X-ray powder diffraction pattern comprising a diffraction peak at one or more of 2θ (±0.2°) of 9.5, 19.6, 13.8, 23.0, 24.2, 16.8, 18.0 and 21.2, preferably comprising a characteristic peak at any 2, 4, 6 and 8 of the above-mentioned 2θ;
the crystal form J of the hydrochloride has an X-ray powder diffraction pattern comprising a diffraction peak at one or more of 2θ (±0.2°) of 7.5, 8.8, 9.5, 13.8, 19.6, 21.2, 23.0 and 26.5, preferably comprising a characteristic peak at any 2, 4, 6 and 8 of the above-mentioned 2θ;
the crystal form A of the methanesulfonate has an X-ray powder diffraction pattern comprising a diffraction peak at one or more of 2θ (±0.2°) of 8.4, 20.0, 13.7, 14.5, 23.6, 20.5 and 23.8, preferably comprising a characteristic peak at any 2, 4 and 6 of the above-mentioned 2θ;
the crystal form A of the p-toluenesulfonate has an X-ray powder diffraction pattern comprising a diffraction peak at one or more of 2θ (±0.2°) of 7.2, 12.8, 8.6, 10.9, 16.4, 17.7, 10.6, 13.4, 14.0, 18.2, 19.5 and 23.3, preferably comprising a characteristic peak at any 2, 4, 6 and 8 of the above-mentioned 2θ;
the crystal form A of the hippurate has an X-ray powder diffraction pattern comprising a diffraction peak at one or more of 2θ (±0.2°) of 7.4, 10.0, 8.1, 14.2, 21.6, 10.5, 20.5 and 25.8, preferably comprising a characteristic peak at any 2, 4, 6 and 8 of the above-mentioned 2θ;
the crystal form A of the malonate has an X-ray powder diffraction pattern comprising a diffraction peak at one or more of 2θ (±0.2°) of 4.8, 19.1, 7.4, 9.5, 12.3, 23.1, 11.7, 14.3, 18.1 and 20.4, preferably comprising a characteristic peak at any 2, 4, 6 and 8 of the 2θ;
the crystal form A of the oxalate has an X-ray powder diffraction pattern comprising a diffraction peak at one or more of 2θ (±0.2°) of 4.8, 9.6, 10.1, 14.4, 19.2, 18.6, 22.1, 22.8 and 24.1, preferably comprising a characteristic peak at any 2, 4, 6 and 8 of the above-mentioned 2θ;
the crystal form A of the adipate has an X-ray powder diffraction pattern comprising a diffraction peak at one or more of 2θ (±0.2°) of 7.4, 10.0, 7.7, 12.3, 19.5, 21.7, 25.8, 1.8, 14.2, 14.9, 20.5, 21.2, 21.5 and 23.2, preferably comprising a characteristic peak at any 2, 4, 6 and 8 of the above-mentioned 2θ;
the crystal form A of the succinate has an X-ray powder diffraction pattern comprising a diffraction peak at one or more of 2θ (±0.2°) of 7.4, 10.0, 7.8, 9.5, 19.1, 20.5, 8.1, 11.8, 18.2 and 25.9, preferably comprising a characteristic peak at any 2, 4, 6 and 8 of the above-mentioned 2θ;
the crystal form A of the phosphate has an X-ray powder diffraction pattern comprising a diffraction peak at one or more of 2θ (±0.2°) of 5.5, 8.6, 7.5, 12.9, 18.3, 10.6, 12.4, 17.3 and 25.3, preferably comprising a characteristic peak at any 2, 4, 6 and 8 of the above-mentioned 2θ;
the crystal form A of the fumarate has an X-ray powder diffraction pattern comprising a diffraction peak at one or more of 2θ (±0.2°) of 7.5, 10.7, 7.9, 14.4, 15.0, 8.3, 10.0 and 20.5, preferably comprising a characteristic peak at any 2, 4, 6 and 8 of the above-mentioned 2θ.

9. The acid salt of the compound according to claim 8, **characterised in that**
the crystal form A of the hydrobromide has an X-ray powder diffraction pattern comprising a characteristic peak at one or more of 2θ (±0.2°) of 8.8, 15.3, 17.5 and 22.8, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 22.0 and 26.4; further preferably, further comprising a characteristic peak at one or more of 2θ (+0.2°) of 25.4 and 31.0; more preferably, further comprising a characteristic peak at 2θ (±0.2°) of 35.5; most preferably, the crystal form has an X-ray powder diffraction pattern substantially as shown in FIG. 1; the crystal form has a DSC pattern substantially as shown in FIG. 2; even more preferably, the crystal form A of the hydrobromide has an X-ray powder diffraction pattern comprising a diffraction peak at 2θ (±0.2°) of
8.8, 17.5, 15.3 and 22.8;
alternatively, 8.8, 17.5, 15.3, 22.8, 26.4, 22.0 and 25.4;
alternatively, 8.8, 17.5, 15.3, 22.8, 26.4, 22.0, 25.4, 31.0 and 35.5;
the crystal form A of the hydrochloride has an X-ray powder diffraction pattern comprising a characteristic peak at one or more of 2θ (±0.2°) of 9.2, 11.4, 15.7 and 20.0, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 13.7 and 16.8; further preferably, further comprising a characteristic peak at one or more of 2θ
(±0.2°) of 19.6 and 20.3; more preferably, further comprising a characteristic peak at 2θ (±0.2°) of 16.5 and 21.7; most preferably, the crystal form has an X-ray powder diffraction pattern substantially as shown in FIG. 3; the crystal form has a DSC pattern substantially as shown in FIG. 4; the crystal form has a TGA pattern substantially as shown in FIG. 5; even more preferably, the crystal form A of the hydrochloride has an X-ray powder diffraction pattern comprising a diffraction peak at 2θ (±0.2°) of
9.2, 15.7, 11.4, 16.8, 20.0, 13.7, 16.5 and 20.3;
alternatively, 9.2, 15.7, 11.4, 16.8, 21.7, 13.7, 19.6 and 20.3;
alternatively, 21.7, 15.7, 11.4, 16.5, 20.0, 13.7, 19.6 and 20.3;
alternatively, 9.2, 15.7, 21.7, 16.8, 20.0, 13.7, 19.6 and 20.3;
alternatively, 9.2, 15.7, 11.4, 16.8, 20.0, 13.7, 19.6, 20.3 and 21.7;
the crystal form B of the hydrochloride has an X-ray powder diffraction pattern comprising a characteristic peak at one or more of 2θ (±0.2°) of 7.2, 7.8, 14.0 and 15.9, preferably 2-4 of the above-mentioned 2θ, more preferably 3-4 of the 2θ, and most preferably 4 of the above-mentioned 2θ; preferably, further comprising a characteristic peak at one or more of 2θ (±0.2°) of 10.5 and 20.0; further preferably, further comprising a characteristic peak at one or more of 2θ (±0.2°) of 17.7 and 21.5; most preferably, the crystal form has an X-ray powder diffraction pattern substantially as shown in FIG. 6; the crystal form has a DSC pattern substantially as shown in FIG. 7; even more preferably, the crystal form B of the hydrochloride has an X-ray powder diffraction pattern comprising a diffraction peak at 2θ (±0.2°) of 7.2, 15.9, 7.8 and 14.0;
alternatively, 7.2, 15.9, 7.8, 14.0, 20.0 and 10.5;
alternatively, 7.2, 15.9, 7.8, 14.0, 20.0, 10.5, 17.7 and 21.5;
the crystal form C of the hydrochloride has an X-ray powder diffraction pattern comprising a characteristic peak at one or more of 2θ (±0.2°) of 9.2, 14.8, 15.2 and 16.0, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 8.8 and 18.4; most preferably, the crystal form has an X-ray powder diffraction pattern substantially as shown in FIG. 8; the crystal form has a DSC pattern substantially as shown in FIG. 9; even more preferably, the crystal form C of the hydrochloride has an X-ray powder diffraction pattern comprising a diffraction peak at 2θ (±0.2°) of 9.2, 16.0, 14.8 and 15.2;
alternatively, 9.2, 16.0, 14.8, 15.2, 8.8 and 18.4;
the crystal form D of the hydrochloride has an X-ray powder diffraction pattern comprising a characteristic peak at one or more of 2θ (±0.2°) of 8.2, 13.1, 23.3 and 16.1, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 15.9 and 24.7; further preferably, further comprising a characteristic peak at 2θ (±0.2°) of 27.1; most preferably, the crystal form has an X-ray powder diffraction pattern substantially as shown in FIG. 10; the crystal form has a DSC pattern substantially as shown in FIG. 11; even more preferably, the crystal form D of the hydrochloride has an X-ray powder diffraction pattern comprising a diffraction peak at 2θ (±0.2°) of
8.2, 13.1, 15.9, 16.1, 23.3 and 24.7;
alternatively, 23.3, 16.1, 13.1, 8.2, 15.9, 24.7 and 27.1;
the crystal form E of the hydrochloride has an X-ray powder diffraction pattern comprising a characteristic peak at one or more of 2θ (±0.2°) of 9.8, 11.2, 23.0 and 15.3, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 12.7 and 17.4; further preferably, further comprising a characteristic peak at one or more of 2θ (±0.2°) of 20.6 and 25.0; most preferably, the crystal form has an X-ray powder diffraction pattern substantially as shown in FIG. 12; the crystal form has a DSC pattern substantially as shown in FIG. 13; even more preferably, the crystal form E of the hydrochloride has an X-ray powder diffraction pattern comprising a diffraction peak at 2θ (±0.2°) of
23.0, 15.3, 9.8, 11.2, 12.7 and 17.4;
alternatively, 23.0, 15.3, 9.8, 11.2, 12.7, 17.4, 20.6 and 25.0;
the crystal form F of the hydrochloride has an X-ray powder diffraction pattern comprising a characteristic peak at one or more of 2θ (±0.2°) of 8.4, 9.3, 10.3 and 19.4, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 13.2 and 17.5; further preferably, further comprising a characteristic peak at one or more of 2θ
(±0.2°) of 18.9; most preferably, the crystal form has an X-ray powder diffraction pattern substantially as shown in FIG. 14; the crystal form has a DSC pattern substantially as shown in FIG. 15; even more preferably, the crystal form F of the hydrochloride has an X-ray powder diffraction pattern comprising a diffraction peak at 2θ (±0.2°) of
9.3, 19.4, 8.4, 10.3, 13.2 and 17.5;
alternatively, 9.3, 19.4, 8.4, 10.3, 13.2, 17.5 and 18.9;
the crystal form G of the hydrochloride has an X-ray powder diffraction pattern comprising a characteristic peak at one or more of 2θ (±0.2°) of 8.0 and 9.9, preferably, further comprising a characteristic peak at one or more of 2θ (±0.2°) of 13.0 and 13.9; further preferably, further comprising a characteristic peak at one or more of 2θ (±0.2°) of 16.0 and 16.6; more preferably, further comprising a characteristic peak at 2θ (±0.2°) of 22.9; most preferably, the crystal form has an X-ray powder diffraction pattern substantially as shown in FIG. 16; even more preferably, the crystal form G of the hydrochloride has an X-ray powder diffraction pattern comprising a diffraction peak at 2θ (±0.2°) of
8.0, 9.9, 13.0, 13.9, 16.0 and 16.6;
alternatively, 8.0, 9.9, 13.0, 13.9, 16.0, 16.6 and 22.9;
the crystal form H of the hydrochloride has an X-ray powder diffraction pattern comprising a characteristic peak at one or more of 2θ (±0.2°) of 6.8, 10.0, 11.4 and 13.6, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 20.0 and 21.5; further preferably, further comprising a characteristic peak at one or more of 2θ (±0.2°) of 26.0 and 31.9; most preferably, the crystal form has an X-ray powder diffraction pattern substantially as shown in FIG. 17; the crystal form has a DSC pattern substantially as shown in FIG. 18; even more preferably, the crystal form G of the hydrochloride has an X-ray powder diffraction pattern comprising a diffraction peak at 2θ (±0.2°) of
6.8, 10.0, 11.4, 13.6, 20.0 and 21.5;
alternatively, 6.8, 10.0, 11.4, 13.6, 20.0, 21.5, 26.0 and 31.9;
the crystal form I of the hydrochloride has an X-ray powder diffraction pattern comprising a characteristic peak at one or more of 2θ (±0.2°) of 9.5, 13.8, 19.6 and 23.0, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 10.4 and 24.2; further preferably, further comprising a characteristic peak at one or more of 2θ (±0.2°) of 16.8 and 18.0; more preferably, further comprising a characteristic peak at 2θ (±0.2°) of 8.8 and 21.2; even more preferably, the crystal form I of the hydrochloride has an X-ray powder diffraction pattern comprising a diffraction peak at 2θ (±0.2°) of
9.5, 8.8, 13.8, 21.2 , 10.4, 16.8 and 18.0;
alternatively, 9.5, 19.6, 13.8, 23.0, 24.2 , 10.4, 16.8 and 18.0;
alternatively, 8.8, 19.6, 13.8, 23.0, 24.2 , 10.4, 16.8 and 21.2;
alternatively, 9.5, 8.8, 21.2, 24.2, 10.4, 16.8 and 18.0;
most preferably, the crystal form has an X-ray powder diffraction pattern substantially as shown in FIG. 19; the crystal form has a DSC pattern substantially as shown in FIG. 20; the crystal form has a TGA pattern substantially as shown in FIG. 38;
the crystal form J of the hydrochloride has an X-ray powder diffraction pattern comprising a characteristic peak at one or more of 2θ (±0.2°) of 9.5, 13.8, 19.6 and 23.0, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 21.2 and 21.7; further preferably, further comprising a characteristic peak at one or more of 2θ (±0.2') of 7.5 and 26.5; more preferably, further comprising a characteristic peak at one or more of 2θ (±0.2°) of 8.8, 9.5, 18.0 and 18.1; most preferably, the crystal form has an X-ray powder diffraction pattern substantially as shown in FIG. 21; the crystal form has a DSC pattern substantially as shown in FIG. 22; even more preferably, the crystal form J of the hydrochloride has an X-ray powder diffraction pattern comprising a diffraction peak at 2θ (±0.2°) of
9.5, 19.6, 13.8, 23.0, 21.2, 21.7 and 26.5;
alternatively, 9.5, 19.6, 13.8, 23.0, 21.2, 21.7, 26.5, 7.5, 8.8, 9.5 and 18.1;
the crystal form A of the methanesulfonate has an X-ray powder diffraction pattern comprising a characteristic peak at one or more of 2θ (±0.2°) of 8.4 and 20.0, preferably, further comprising a characteristic peak at one or more of 2θ (±0.2°) of 13.7 and 14.5; further preferably, further comprising a characteristic peak at one or more of 2θ (±0.2°) of 20.5 and 23.6; more preferably, further comprising a characteristic peak at 2θ (±0.2°) of 23.8; most preferably, the crystal form has an X-ray powder diffraction pattern substantially as shown in FIG. 23; the crystal form has a DSC pattern substantially as shown in FIG. 24; even more preferably, the crystal form A of the methanesulfonate has an X-ray powder diffraction pattern comprising a diffraction peak at 2θ (±0.2°) of
8.4, 20.0, 13.7, 14.5 and 23.6;
alternatively, 8.4, 20.0, 13.7, 14.5, 23.6, 20.5 and 23.8;
the crystal form A of the p-toluenesulfonate has an X-ray powder diffraction pattern comprising a characteristic peak at one or more of 2θ (±0.2°) of 7.2, 8.6, 10.9 and 12.8, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 16.4 and 17.7; further preferably, further comprising a characteristic peak at one or more of 2θ (±0.2°) of 10.6 and 13.4; more preferably, further comprising a characteristic peak at one or more of 2θ (±0.2°) of 14.0, 18.2, 19.5 and 23.3; most preferably, the crystal form has an X-ray powder diffraction pattern substantially as shown in FIG. 25; the crystal form has a DSC pattern substantially as shown in FIG. 26; even more preferably, the crystal form A of the p-toluenesulfonate has an X-ray powder diffraction pattern comprising a diffraction peak at 2θ (±0.2°) of
7.2, 12.8, 8.6, 10.9, 16.4 and 17.7;
alternatively, 7.2, 12.8, 8.6, 10.9, 16.4, 17.7, 10.6, 13.4, 14.0 and 18.2;
alternatively, 7.2, 12.8, 8.6, 10.9, 16.4, 17.7, 10.6, 13.4, 14.0, 18.2, 19.5 and 23.3;
the crystal form A of the hippurate has an X-ray powder diffraction pattern comprising a characteristic peak at one or more of 2θ (±0.2°) of 7.4, 8.1, 10.0 and 14.2, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 10.5 and 21.6; further preferably, further comprising a characteristic peak at one or more of 2θ (±0.2°) of 20.5 and 25.8; most preferably, the crystal form has an X-ray powder diffraction pattern substantially as shown in FIG. 27; even more preferably, the crystal form A of the hippurate has an X-ray powder diffraction pattern comprising a diffraction peak at 2θ (±0.2°) of
7.4, 10.0, 8.1, 14.2, 21.6, 10.5 and 20.5;
alternatively, 7.4, 10.0, 8.1, 14.2, 21.6, 10.5, 20.5 and 25.8;
the crystal form A of the malonate has an X-ray powder diffraction pattern comprising a characteristic peak at one or more of 2θ (±0.2°) of 4.8, 7.4, 9.5 and 19.1, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 12.3 and 23.1; further preferably, further comprising a characteristic peak at one or more of 2θ (±0.2°) of 11.7 and 14.3; more preferably, further comprising a characteristic peak at one or more of 2θ (±0.2°) of 18.1 and 20.4; most preferably, the crystal form has an X-ray powder diffraction pattern substantially as shown in FIG. 28; even more preferably, the crystal form A of the malonate has an X-ray powder diffraction pattern comprising a diffraction peak at 2θ (±0.2°) of
4.8, 19.1, 7.4, 9.5, 12.3, 23.1, 11.7 and 14.3;
alternatively, 4.8, 19.1, 7.4, 9.5, 12.3, 23.1, 11.7, 14.3, 18.1 and 20.4;
the crystal form A of the oxalate has an X-ray powder diffraction pattern comprising a characteristic peak at one or more of 2θ (±0.2°) of 4.8, 9.6, 10.1 and 14.4, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 18.6 and 19.2; further preferably, further comprising a characteristic peak at one or more of 2θ (±0.2°) of 22.1 and 22.8; more preferably, further comprising a characteristic peak at 2θ (±0.2°) of 24.1; most preferably, the crystal form has an X-ray powder diffraction pattern substantially as shown in FIG. 29; the crystal form has a DSC pattern substantially as shown in FIG. 30; even more preferably, the crystal form A of the oxalate has an X-ray powder diffraction pattern comprising a diffraction peak at 2θ (±0.2°) of
4.8, 9.6, 10.1, 14.4, 19.2 and 18.6;
alternatively, 4.8, 9.6, 10.1, 14.4, 19.2, 18.6, 22.1, 22.8 and 24.1;
the crystal form A of the adipate has an X-ray powder diffraction pattern comprising a characteristic peak at one or more of 2θ (±0.2°) of 7.4, 7.7, 10.0 and 12.3, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 19.5 and 21.7; further preferably, further comprising a characteristic peak at one or more of 2θ
(±0.2') of 1.8 and 25.8; more preferably, further comprising a characteristic peak at one or more of 2θ (±0.2°) of 14.2, 14.9, 20.5, 21.2, 21.5 and 23.2; most preferably, the crystal form has an X-ray powder diffraction pattern substantially as shown in FIG. 31; the crystal form has a DSC pattern substantially as shown in FIG. 32; even more preferably, the crystal form A of the adipate has an X-ray powder diffraction pattern comprising a diffraction peak at 2θ (±0.2°) of
7.4, 10.0, 7.7, 12.3, 19.5 and 21.7;
alternatively, 7.4, 10.0, 7.7, 12.3, 19.5, 21.7, 25.8 and 1.8;
alternatively, 7.4, 10.0, 7.7, 12.3, 19.5, 21.7, 25.8, 1.8, 14.2, 14.9, 20.5, 21.2, 21.5 and 23.2;
the crystal form A of the succinate has an X-ray powder diffraction pattern comprising a characteristic peak at one or more of 2θ (±0.2°) of 7.4, 7.8, 9.5 and 10.0, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 19.1 and 20.5; further preferably, further comprising a characteristic peak at one or more of 2θ (+0.2') of 8.1 and 11.8; more preferably, further comprising a characteristic peak at 2θ (±0.2°) of 18.2 and 25.9; most preferably, the crystal form has an X-ray powder diffraction pattern substantially as shown in FIG. 33; even more preferably, the crystal form A of the succinate has an X-ray powder diffraction pattern comprising a diffraction peak at 2θ (±0.2°) of
7.4, 10.0, 7.8, 9.5, 19.1, 20.5, 8.1 and 11.8;
alternatively, 7.4, 10.0, 7.8, 9.5, 19.1, 20.5, 8.1, 11.8, 18.2 and 25.9;
the crystal form A of the phosphate has an X-ray powder diffraction pattern comprising a characteristic peak at one or more of 2θ (±0.2°) of 5.5, 8.6, 7.5 and 12.9, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 10.6 and 18.3; further preferably, further comprising a characteristic peak at one or more of 2θ (±0.2°) of 12.4 and 17.3; more preferably, further comprising a characteristic peak at 2θ (±0.2°) of 25.3; most preferably, the crystal form has an X-ray powder diffraction pattern substantially as shown in FIG. 34; the crystal form has a DSC pattern substantially as shown in FIG. 35; even more preferably, the crystal form A of the phosphate has an X-ray powder diffraction pattern comprising a diffraction peak at 2θ (±0.2°) of
5.5, 8.6, 7.5, 12.9, 18.3 and 10.6;
alternatively, 5.5, 8.6, 7.5, 12.9, 18.3, 10.6, 12.4, 17.3 and 25.3;
the crystal form A of the fumarate has an X-ray powder diffraction pattern comprising a characteristic peak at one or more of 2θ (±0.2°) of 7.5, 10.7, 7.9 and 14.4, preferably 2-4 of the above-mentioned 2θ, more preferably 3-4 of the above-mentioned 2θ, and most preferably 4 of the above-mentioned 2θ; preferably, further comprising a characteristic peak at one or more of 2θ (±0.2°) of 8.3 and 15.0; further preferably, further comprising a characteristic peak at one or more of 2θ (±0.2°) of 10.0 and 20.5; most preferably, the crystal form has an X-ray powder diffraction pattern substantially as shown in FIG. 36; even more preferably, the crystal form A of the fumarate has an X-ray powder diffraction pattern comprising a diffraction peak at 2θ (±0.2°) of
7.5, 10.7, 7.9, 14.4, 15.0 and 8.3;
alternatively, 7.5, 10.7, 7.9, 14.4, 15.0, 8.3, 10.0 and 20.5.

10. A method for preparing the acid salt of the compound according to any one of claims 1-9, **characterised in that** the method specifically comprises the following steps:
1) weighing an appropriate amount of a free base and dissolving the free base in a benign solvent;
2) weighing an appropriate amount of a counterion acid and dissolving the counterion acid in an organic solvent, wherein the amount of the counterion acid is preferably 1 equivalent;
3) combining the above-described two solutions, and stirring to allow precipitation, or dropwise adding a poor solvent followed by stirring to allow precipitation; and
4) rapidly centrifuging the resultant or leaving the resultant to stand for blow drying to obtain a target product,
wherein
the benign solvent is selected from methanol, dichloromethane, 1,4-dioxane, acetone, tetrahydrofuran, N,N-dimethylformamide, ethyl acetate, N,N-dimethylacetamide or N-methylpyrrolidone, preferably methanol or ethyl acetate;
the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide, preferably methanol, acetone or ethyl acetate; the above-described benign solvent and organic solution must be miscible when used;
the poor solvent is selected from heptane, water and cyclohexane, preferably water and n-heptane;
alternatively,
1) weighing an appropriate amount of a free base, and suspending the free base in a poor solvent;
2) weighing an appropriate amount of a counterion acid and dissolving the counterion acid in an organic solvent, wherein the amount of the counterion acid is preferably 1 equivalent;
3) combining the above-described two solutions with stirring until dissolved, and further stirring to allow precipitation, or dropwise adding a poor solvent followed by further stirring to allow precipitation; and
4) rapidly centrifuging the resultant or leaving the resultant to stand for blow drying to obtain a target product,
wherein the organic solvent is selected from methanol, ethanol, ethyl acetate, dichloromethane, acetone, n-hexane, petroleum ether, benzene, toluene, chloroform, acetonitrile, carbon tetrachloride, dichloroethane, tetrahydrofuran, 2-butanone, 3-pentanone, heptane, methyl tert-butyl ether, isopropyl ether, 1,4-dioxane, tert-butyl alcohol, or N,N-dimethylformamide; preferably, the organic solvent is methanol;
the poor solvent is selected from heptane, acetonitrile, ethanol, methyl tert-butyl ether, 2-methyltetrahydrofuran, ethyl acetate, isopropyl acetate or toluene, preferably methyl tert-butyl ether; the above-described poor solvent and organic solution must be miscible when used;
the counterion acid is selected from hydrochloric acid, sulphuric acid, nitric acid, hydrobromic acid, hydrofluoric acid, hydroiodic acid, phosphoric acid, 2,5-dihydroxybenzoic acid, 1-hydroxy-2-naphthoic acid, acetic acid, dichloroacetic acid, trichloroacetic acid, acetohydroxamic acid, adipic acid, benzenesulphonic acid, 4-chlorobenzenesulphonic acid, benzoic acid, 4-acetylaminobenzoic acid, 4-aminobenzoic acid, decanoic acid, hexanoic acid, octanoic acid, cinnamic acid, citric acid, cyclohexaneaminosulphonic acid, camphorsulphonic acid, asparaginic acid, camphoric acid, gluconic acid, glucuronic acid, glutamic acid, isoascorbic acid, lactic acid, malic acid, mandelic acid, pyroglutamic acid, tartaric acid, dodecylsulphuric acid, dibenzoyltartaric acid, ethane-1,2-disulphonic acid, ethanesulphonic acid, formic acid, fumaric acid, galactonic acid, gentisuric acid, glutaric acid, 2-ketoglutaric acid, glycolic acid, hippuric acid, hydroxyethylsulphonic acid, lactobionic acid, ascorbic acid, aspartic acid, lauric acid, camphoric acid, maleic acid, malonic acid, methanesulphonic acid, 1,5-naphthalenedisulphonic acid, naphthalene-2-sulphonic acid, nicotinic acid, oleic acid, orotic acid, oxalic acid, palmitic acid, embonic acid, propanoic acid, salicylic acid, 4-aminosalicylic acid, sebacic acid, stearic acid, succinic acid, thiocyanic acid, undecylenoic acid, trifluoroacetic acid, benzenesulphonic acid, p-methylbenzenesulphonic acid, or L-malic acid; preferably, the counterion acid is selected from methanesulphonic acid, p-toluenesulphonic acid or hydrochloric acid; more preferably, the counterion acid is hydrochloric acid.

11. A pharmaceutical composition, **characterised by** comprising a therapeutically effective amount of the acid salt of the compound according to any one of claims 1 to 9, and one or more pharmaceutically acceptable carriers, diluents or excipients.

12. Use of the acid salt of the compound according to any one of claims 1 to 9, or the pharmaceutical composition according to claim 11 in the preparation of a medicament for treating a disease or condition associated with activation of complement alternative pathway.

13. The use according to claim 12, **characterised in that** the disease or condition is a disease or condition that is associated with activation of a complement alternative pathway and treated by modulating complement factor B.

14. The use according to either claim 12 or 13, **characterised in that** the disease or condition is selected from age-related macular degeneration, geographic atrophy, diabetic retinopathy, uveitis, retinitis pigmentosa, macular oedema, Behçet's uveitis, multifocal choroiditis, Vogt-Koyangi-Harada syndrome, intermediate uveitis, birdshot retinochoroidopathy, sympathetic ophthalmia, ocular pemphigoid, ocular pemphigus, retinal vein occlusion, neurological diseases, multiple sclerosis, stroke, Guillain-Barré syndrome, traumatic brain injury, Parkinson's disease, disorders of inappropriate or undesirable complement activation, haemodialysis complications, hyperacute allograft rejection, xenograft rejection, interleukin-2-induced toxicity during IL-2 therapy, inflammatory diseases, inflammation in autoimmune diseases, Crohn's disease, adult respiratory distress syndrome, myocarditis, postischemic reperfusion conditions, myocardial infarction, balloon angioplasty, post-pump syndrome associated with cardiopulmonary bypass or renal bypass, atherosclerosis, haemodialysis, renal ischemia, mesenteric artery reperfusion after aortic reconstruction, infectious diseases or sepsis, immune complex diseases and autoimmune diseases, rheumatoid arthritis, systemic lupus erythematosus, SLE nephritis, proliferative nephritis, liver fibrosis, haemolytic anaemia, myasthenia gravis, tissue regeneration, nerve regeneration, dyspnoea, haemoptysis, ARDS, asthma, chronic obstructive pulmonary disease, emphysema, pulmonary embolism and infarction, pneumonia, fibrogenic dust disease, pulmonary fibrosis, asthma, allergy, bronchoconstriction, hypersensitivity pneumonitis, parasitic diseases, Goodpasture syndrome, pulmonary vasculitis, pauci-immune vasculitis, immune complex-related inflammation, antiphospholipid syndrome, glomerulonephritis, and obesity.
